# EUROPEAN PATENT SPECIFICATION

(11) **EP 3 820 461 B1**
(45) Date of publication and mention of the grant of the patent: **07.01.2026**
(21) Application number: 19833416.1
(22) Date of filing: 12.07.2019
(51) Int. Cl.: A61K 38/50, A61K 31/167, A61K 31/415, A61K 31/4015, A61K 31/404, A61K 31/437, A61P 35/00, A61K 31/506, A61K 31/5517, A61K 31/7105, A61K 31/15, A61K 31/5377

(54) **USE OF ASPARAGINASE IN COMBINATION WITH GSK3A INHIBITION FOR TREATING CANCER**
VERWENDUNG VON ASPARAGINASE ZUSAMMEN MIT HEMMUNG VON GSK3A ZUR BEHANDLUNG VON KREBS
UTILISATION DE L'ASPARAGINASE COMBINÉE AVEC L'INHIBITION DE GSK3A POUR LE TRAITEMENT DU CANCER

(30) Priority: 12.07.2018 US 201862697053 P; 26.10.2018 US 201862751129 P; 29.04.2019 US 201962839912 P
(43) Date of publication of application: 19.05.2021
(73) Proprietor: The Children's Medical Center Corporation, Boston, MA 02115 (US)
(72) Inventor: GUTIERREZ, Alejandro, Boston, MA 02115 (US); HINZE, Laura, Boston, MA 02115 (US)
(74) Representative: Brand Murray Fuller LLP
(86) International application number: PCT/US2019/041555
(87) International publication number: WO 2020/014581

(56) References cited:
- WO-A1-2006/018633
- WO-A1-2013/130364
- WO-A1-2014/135244
- US-A1- 2009 004 173
- US-A1- 2016 312 207
- US-A1- 2016 375 006
- US-A1- 2017 246 174
- RACHEL A EGLER ET AL: "L-asparaginase in the treatment of patients with acute lymphoblastic leukemia", JOURNAL OF PHARMACOLOGY AND PHARMACOTHERAPEUTICS, vol. 7, 1 January 2016 (2016-01-01), pages 62 - 71, XP055462282
- WAGNER FLORENCE F. ET AL: "Exploiting an Asp-Glu "switch" in glycogen synthase kinase 3 to design paralog-selective inhibitors for use in acute myeloid leukemia", vol. 10, no. 431, 7 March 2018 (2018-03-07), XP055893087, ISSN: 1946-6234, Retrieved from the Internet <URL:https://www.ncbi.nlm.nih.gov/pmc/articles/PMC6553635/pdf/nihms-1029225.pdf> DOI: 10.1126/scitranslmed.aam8460
- HINZE ET AL.: "Synthetic Lethality of Wnt Pathway Activation and Asparaginase in Drug-Resistant Acute Leukemias", BIORXIV, 12 September 2018 (2018-09-12), pages 1 - 33, XP055675078
- HU ET AL.: "A novel glycogen synthase kinase-3 inhibitor optimized for acute myeloid leukemia differentiation activity", MOLECULAR CANCER THERAPEUTICS, vol. 15, no. 7, 1 July 2017 (2017-07-01), pages 1485 - 1494, XP055675043
- VIMAL ET AL.: "L-Asparaginase: a feasible therapeutic molecule for multiple diseases", 3 BIOTECH, vol. 8, no. 278, 28 May 2018 (2018-05-28), pages 1 - 3, XP055675042

## Description

### CROSS-REFERENCE TO RELATED APPLICATION

This Application claims benefit under 35 U.S.C. § 119(e) of the U.S. Provisional Application No 62/697,053 filed July 12, 2018; U.S. Provisional Application No 62/751,129 filed October 26, 2018; and U.S. Provisional Application No 62/839,912 filed April 29, 2019.

### FIELD OF THE INVENTION

The field of the invention relates to asparaginase for use in the treatment of cancer.

### GOVERNMENT SUPPORT

This invention was made with Government support under Grant No. R01 CA193651 awarded by the National Institutes of Health. The Government has certain rights in the invention.

### BACKGROUND

Leukemia is the most common of pediatric cancers accounting for about 30% of diagnoses. There are two main subtypes; acute lymphoblastic leukemia (ALL) and acute myeloid leukemia (AML). AML is less common, accounting for approximately 18% of childhood leukemia diagnoses. These leukemia types also occur in adults, and AML becomes more common in older individuals. The etiology of the two subtypes is likely quite different based on both cell lineage and epidemiological studies of incidence and risk factors. Aggressive chemotherapies are required to improve the prognosis of patients diagnoses with leukemias such as ALL or AML.

Asparaginase, a bacterial enzyme that depletes the nonessential amino acid asparagine, is an integral component of acute leukemia therapy^{1,2}. The antineoplastic effects of asparaginase are likely caused by its depletion of extracellular asparagine and glutamine creating a state of amino acid deficiency and subsequent inhibition of protein synthesis. However, other mechanisms remain to be identified. Despite its efficacy in ALL, asparaginase has been used only occasionally in the treatment of other leukemias and solid tumors. Previous *in vitro* studies have observed varied response to asparaginase in acute myeloid leukemia (AML) across the French-American-British subtypes. Asparaginase resistance remains a common clinical problem for leukemia patients where the biologic basis of the resistance is poorly understood.

### SUMMARY OF THE INVENTION

The invention described herein is related, in part, to the discovery that inhibition of GSK3α sensitized cancer cells, e.g., leukemia cells, to asparaginase. Accordingly, in a first aspect the present invention provides an asparaginase for use in the treatment of cancer in a subject, wherein: the asparaginase is used in combination with an agent that inhibits GSK3α, or the cancer comprises a mutation that results in inhibition of GSK3α. Alternative embodiments are set out in the dependent claims herein.

In one embodiment, the cancer is a carcinoma, a melanoma, a sarcoma, a myeloma, a leukemia, and a lymphoma.

In one embodiment, the cancer is a solid tumor.

In one embodiment, the caner is a leukemia selected from acute myeloid leukemia (AML), Chronic myeloid leukemia (CML), Acute lymphocytic leukemia (ALL), and Chronic lymphocytic leukemia (CLL).

In one embodiment, the cancer is resistant to an asparaginase.

In one embodiment, the cancer is not resistant to an asparaginase.

In one embodiment, the asparaginase is selected from the group consisting of: L-asparaginase (Elspar), pegaspargase (PEG-asparaginase; Oncaspar), SC-PEG asparaginase (Calaspargase pegol), and Erwinia asparaginase (Erwinaze).

In one embodiment, the agent that inhibits GSK3α is selected from the group consisting of a small molecule, an antibody, a peptide, a genome editing system, an antisense oligonucleotide, and an RNAi.

In one embodiment, the small molecule is selected from the group consisting of: BRD0705, BRD4963, BRD1652, BRD3731, CHIR-98014, LY2090314, AZD1080, CHIR-99021 (CT99021) HCl, CHIR-99021 (CT99021), BIO-acetoxime, SB216763, SB415286, Abemaciclib (LY2835210), AT-9283, RGB-286638, PHA-793887, AT-7519, AZD-5438, OTS-167, 9-ING-41, Tideglusib (NP031112), and AR-A014418. In one embodiment the small molecule is BRD0705.

In one embodiment, the RNAi is a microRNA, an siRNA, or a shRNA.

In one embodiment, inhibiting GSK3α is inhibiting the expression level and/or activity of GSK3α. In one embodiment the expression level and/or activity of GSK3α is inhibited by at least 50%, at least 60%, at least 70%, at least 80%, at least 90%, or more as compared to an appropriate control.

In one embodiment, the subject has previously been administered an anti-cancer therapy.

In one embodiment, the subject has not previously been administered an anti-cancer therapy.

In one embodiment the subject has, prior to treatment, been diagnosed as having cancer.

In one embodiment the subject has, prior to treatment, received a result from an assay that diagnoses a subject as having cancer.

In one embodiment, the mutation results in the activation of the WNT signaling pathway in the cancer cell.

In one embodiment, the mutation is in a gene selected from the group consisting of: R-spondin1 (RSPO1), R-spondin 2 (RSPO2), R-spondin 3 (RSPO3), R-spondin4 (RSPO4), ring finger protein 43 (RNF43), and glycogen synthase kinase 3 alpha (GSK3A, more commonly referred to as GSK3α).

In one embodiment, the mutation alters the expression of a gene selected from the group consisting of: R-spondin1 (RSPO1), R-spondin 2 (RSPO2), R-spondin 3 (RSPO3), R-spondin4 (RSPO4), ring finger protein 43 (RNF43), and glycogen synthase kinase 3 alpha (GSK3A, more commonly referred to as GSK3α).

In one embodiment, the cancer is colon or pancreatic cancer.

In one embodiment, the cancer is metastatic.

In one embodiment, the subject has, prior to treatment, been identified as having cancer comprising a mutation that results in inhibition of GSK3α.

In one embodiment, the mutation is identified in a biological sample obtained from the subject. In one embodiment, the biological sample is a tissue sample or a blood sample. In one embodiment, the subject has been identified as having a cancer having a mutation that results in inhibition of GSK3α by an assay of a biological sample, optionally a tissue sample or a blood sample, from said subject.

In one embodiment, the cancer is resistant to a cancer therapy. In one embodiment, the cancer relapsed following a cancer therapy. In one embodiment the cancer therapy is chemotherapy, radiation therapy, immunotherapy, surgery, hormone therapy, stem cell therapy, targeted therapy, gene therapy, or precision therapy.

Disclosed herein is a method of treating cancer, the method comprising: (a) obtaining a biological sample from a subject having cancer; (b) assaying the sample and identifying the cancer as having a mutation that results in inhibition of GSK3α; and (c) administering an asparaginase to a subject who has been identified as having cancer having a mutation that results in inhibition of GSK3α.

Disclosed herein is a method of treating cancer, the method comprising: (a) receiving the results of an assay that identifies a subject as having a cancer having a mutation that results in inhibition of GSK3α; and (b) administering an asparaginase to a subject who has been identified as having cancer having a mutation that results in inhibition of GSK3α.

### Definitions

For convenience, the meaning of some terms and phrases used in the specification, examples, and appended claims, are provided below. Unless stated otherwise, or implicit from context, the following terms and phrases include the meanings provided below. The definitions are provided to aid in describing particular embodiments, and are not intended to limit the claimed technology, because the scope of the technology is limited only by the claims. Unless otherwise defined, all technical and scientific terms used herein have the same meaning as commonly understood by one of ordinary skill in the art to which this technology belongs. If there is an apparent discrepancy between the usage of a term in the art and its definition provided herein, the definition provided within the specification shall prevail.

As used herein, the terms "treat," "treatment," "treating," or "amelioration" refer to therapeutic treatments, wherein the object is to reverse, alleviate, ameliorate, inhibit, slow down or stop the progression or severity of a condition associated with cancer, e.g., leukemia, colon cancer, or pancreatic cancer. The term "treating" includes reducing or alleviating at least one adverse effect or symptom of cancer. Treatment is generally "effective" if one or more symptoms or clinical markers are reduced. Alternatively, treatment is "effective" if the progression of a disease is reduced or halted. That is, "treatment" includes not just the improvement of symptoms or markers, but also a cessation of, or at least slowing of, progress or worsening of symptoms compared to what would be expected in the absence of treatment. Beneficial or desired clinical results include, but are not limited to, alleviation of one or more symptom(s), diminishment of extent of disease, stabilized (*i.e.,* not worsening) state of disease, delay or slowing of disease progression, amelioration or palliation of the disease state, remission (whether partial or total), and/or decreased mortality, whether detectable or undetectable. The term "treatment" of a disease also includes providing relief from the symptoms or side-effects of the disease (including palliative treatment).

As used herein, the term "administering," refers to the placement of a therapeutic (e.g., an agent that inhibits GSK3α and/or asparaginase) or pharmaceutical composition as disclosed herein into a subject by a method or route which results in at least partial delivery of the agent to the subject. Pharmaceutical compositions comprising agents as disclosed herein can be administered by any appropriate route which results in an effective treatment in the subject.

As used herein, a "subject" means a human or animal. Usually the animal is a vertebrate such as a primate, rodent, domestic animal or game animal. Primates include, for example, chimpanzees, cynomolgus monkeys, spider monkeys, and macaques, e.g., Rhesus. Rodents include, for example, mice, rats, woodchucks, ferrets, rabbits and hamsters. Domestic and game animals include, for example, cows, horses, pigs, deer, bison, buffalo, feline species, e.g., domestic cat, canine species, e.g., dog, fox, wolf, avian species, e.g., chicken, emu, ostrich, and fish, e.g., trout, catfish and salmon. In some embodiments, the subject is a mammal, e.g., a primate, e.g., a human. The terms, "individual," "patient" and "subject" are used interchangeably herein.

Preferably, the subject is a mammal. The mammal can be a human, non-human primate, mouse, rat, dog, cat, horse, or cow, but is not limited to these examples. Mammals other than humans can be advantageously used as subjects that represent animal models of disease e.g., cancer. A subject can be male or female.

A subject can be one who has been previously diagnosed with or identified as suffering from or having a disease or disorder in need of treatment (e.g., cancer) or one or more complications related to such a disease or disorder, and optionally, have already undergone treatment (e.g., one or more cancer therapies) for the disease or disorder or the one or more complications related to the disease or disorder. Alternatively, a subject can also be one who has not been previously diagnosed as having such disease or disorder or related complications. For example, a subject can be one who exhibits one or more risk factors for the disease or disorder or one or more complications related to the disease or disorder or a subject who does not exhibit risk factors.

As used herein, an "agent" refers to e.g., a molecule, protein, peptide, antibody, or nucleic acid, that inhibits expression of a polypeptide or polynucleotide, or binds to, partially or totally blocks stimulation, decreases, prevents, delays activation, inactivates, desensitizes, or down regulates the activity of the polypeptide or the polynucleotide. Agents that inhibit GSK3α, *e.g.,* inhibit expression, *e.g.,* translation, post-translational processing, stability, degradation, or nuclear or cytoplasmic localization of a polypeptide, or transcription, post transcriptional processing, stability or degradation of a polynucleotide or bind to, partially or totally block stimulation, DNA binding, transcription factor activity or enzymatic activity, decrease, prevent, delay activation, inactivate, desensitize, or down regulate the activity of a polypeptide or polynucleotide. An agent can act directly or indirectly.

The term "agent" as used herein means any compound or substance such as, but not limited to, a small molecule, nucleic acid, polypeptide, peptide, drug, ion, etc. An "agent" can be any chemical, entity or moiety, including without limitation synthetic and naturally-occurring proteinaceous and non-proteinaceous entities. In some embodiments, an agent is nucleic acid, nucleic acid analogues, proteins, antibodies, peptides, aptamers, oligomer of nucleic acids, amino acids, or carbohydrates including without limitation proteins, oligonucleotides, ribozymes, DNAzymes, glycoproteins, siRNAs, lipoproteins, aptamers, and modifications and combinations thereof etc. In certain embodiments, agents are small molecule having a chemical moiety. For example, chemical moieties included unsubstituted or substituted alkyl, aromatic, or heterocyclyl moieties including macrolides, leptomycins and related natural products or analogues thereof. Compounds can be known to have a desired activity and/or property, or can be selected from a library of diverse compounds.

The agent can be a molecule from one or more chemical classes, e.g., organic molecules, which may include organometallic molecules, inorganic molecules, genetic sequences, etc. Agents may also be fusion proteins from one or more proteins, chimeric proteins (for example domain switching or homologous recombination of functionally significant regions of related or different molecules), synthetic proteins or other protein variations including substitutions, deletions, insertion and other variants.

As used herein, the term "small molecule" refers to a chemical agent which can include, but is not limited to, a peptide, a peptidomimetic, an amino acid, an amino acid analog, a polynucleotide, a polynucleotide analog, an aptamer, a nucleotide, a nucleotide analog, an organic or inorganic compound (e.g., including heterorganic and organometallic compounds) having a molecular weight less than about 10,000 grams per mole, organic or inorganic compounds having a molecular weight less than about 5,000 grams per mole, organic or inorganic compounds having a molecular weight less than about 1,000 grams per mole, organic or inorganic compounds having a molecular weight less than about 500 grams per mole, and salts, esters, and other pharmaceutically acceptable forms of such compounds.

The term "RNAi" as used herein refers to interfering RNA or RNA interference. RNAi refers to a means of selective post-transcriptional gene silencing by destruction of specific mRNA by molecules that bind and inhibit the processing of mRNA, for example inhibit mRNA translation or result in mRNA degradation. As used herein, the term "RNAi" refers to any type of interfering RNA, including but are not limited to, siRNA, shRNA, endogenous microRNA and artificial microRNA. For instance, it includes sequences previously identified as siRNA, regardless of the mechanism of down-stream processing of the RNA (i.e. although siRNAs are believed to have a specific method of in vivo processing resulting in the cleavage of mRNA, such sequences can be incorporated into the vectors in the context of the flanking sequences described herein).

As used herein, the term "cancer therapy" or "cancer treatment" refers to a therapy useful in treating a cancer. Examples of anti-cancer therapeutic agents include, but are not limited to, e.g., surgery, chemotherapeutic agents, immunotherapy, growth inhibitory agents, cytotoxic agents, agents used in radiation therapy, anti-angiogenesis agents, apoptotic agents, anti-tubulin agents, and other agents to treat cancer, such as anti-HER-2 antibodies (e.g., HERCEPTIN^{®}), anti-CD20 antibodies, an epidermal growth factor receptor (EGFR) antagonist (e.g., a tyrosine kinase inhibitor), HER1/EGFR inhibitor (e.g., erlotinib (TARCEVA^{®})), platelet derived growth factor inhibitors (e.g., GLEEVEC^{™} (Imatinib Mesylate)), a COX-2 inhibitor (e.g., celecoxib), interferons, cytokines, antagonists (e.g., neutralizing antibodies) that bind to one or more of the following targets ErbB2, ErbB3, ErbB4, PDGFR-beta, BlyS, APRIL, BCMA or VEGF receptor(s), TRAIL/Apo2, and other bioactive and organic chemical agents, etc. Combinations thereof are also contemplated for use with the methods described herein.

Methods and compositions described herein require that the levels and/or activity of GSK3α are inhibited. As used herein, "Glycogen synthase kinase-3 alpha (GSK3α)" refers to a multifunctional Ser/Thr protein kinase that is implicated in the control of several regulatory proteins including glycogen synthase, and transcription factors, such as JUN. It also plays a role in the WNT and PI3K signaling pathways, as well as regulates the production of beta-amyloid peptides associated with Alzheimer's disease. GSK3α sequences are known for a number of species, e.g., human GSK3α (NCBI Gene ID: 2931) polypeptide (e.g., NCBI Ref Seq NP_063937.2) and mRNA (e.g., NCBI Ref Seq NM_019884.2). GSK3α can refer to human GSK3α, including naturally occurring variants, molecules, and alleles thereof. GSK3α refers to the mammalian GSK3α of, e.g., mouse, rat, rabbit, dog, cat, cow, horse, pig, and the like. The nucleic sequence of SEQ ID NO: 1 comprises the nucleic sequence which encodes GSK3α.

SEQ ID NO: 1 is a nucleic acid sequence that encodes GSK3α. ctcggcgcca

ctcggcgcca
tgagcggcgg cgggccttcg ggaggcggcc ctgggggctc gggcagggcg cggactagct
cgttcgcgga gcccggcggc ggaggcggag gaggcggcgg cggccccgga ggctcggcct
ccggcccagg cggcaccggc ggcggaaagg catctgtcgg ggccatgggt gggggcgtcg
gggcctcgag ctccgggggt ggacccggcg gcagcggcgg aggaggcagc ggaggccccg
gcgcaggcac tagcttcccg ccgcccgggg tgaagctggg ccgtgacagc gggaaggtga
ccacagtcgt agccactcta ggccaaggcc cagagcgctc ccaagaagtg gcttacacgg
acatcaaagt gattggcaat ggctcatttg gggtcgtgta ccaggcacgg ctggcagaga
ccagggaact agtcgccatc aagaaggttc tccaggacaa gaggttcaag aaccgagagc
tgcagatcat gcgtaagctg gaccactgca atattgtgag gctgagatac tttttctact
ccagtggcga gaagaaagac gagctttacc taaatctggt gctggaatat gtgcccgaga
cagtgtaccg ggtggcccgc cacttcacca aggccaagtt gaccatccct atcctctatg
tcaaggtgta catgtaccag ctcttccgca gcttggccta catccactcc cagggcgtgt
gtcaccgcga catcaagccc cagaacctgc tggtggaccc tgacactgct gtcctcaagc
tctgcgattt tggcagtgca aagcagttgg tccgagggga gcccaatgtc tcctacatct
gttctcgcta ctaccgggcc ccagagctca tctttggagc cactgattac acctcatcca
tcgatgtttg gtcagctggc tgtgtactgg cagagctcct cttgggccag cccatcttcc
ctggggacag tggggtggac cagctggtgg agatcatcaa ggtgctggga acaccaaccc
gggaacaaat ccgagagatg aaccccaact acacggagtt caagttccct cagattaaag
ctcacccctg gacaaaggtg ttcaaatctc gaacgccgcc agaggccatc gcgctctgct

ctagcctgct ggagtacacc ccatcctcaa ggctctcccc actagaggcc tgtgcgcaca
gcttctttga tgaactgcga tgtctgggaa cccagctgcc taacaaccgc ccacttcccc
ctctcttcaa cttcagtgct ggtgaactct ccatccaacc gtctctcaac gccattctca
tccctcctca cttgaggtcc ccagcgggca ctaccaccct caccccgtcc tcacaagctt
taactgagac tccgaccagc tcagactggc agtcgaccga tgccacacct accctcacta
actcctcctga (SEQ ID NO: 1)

The term "decrease", "reduced", "reduction", or "inhibit" are all used herein to mean a decrease by a statistically significant amount. In some embodiments, "decrease", "reduced", "reduction", or "inhibit" typically means a decrease by at least 10% as compared to an appropriate control (e.g. the absence of a given treatment) and can include, for example, a decrease by at least about 10%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 98%, at least about 99% , or more. As used herein, "reduction" or "inhibition" does not encompass a complete inhibition or reduction as compared to a reference level. "Complete inhibition" is a 100% inhibition as compared to an appropriate control.

As used herein, a "reference level" refers to a normal, otherwise unaffected cell population or tissue (e.g., a biological sample obtained from a healthy subject, or a biological sample obtained from the subject at a prior time point, e.g., a biological sample obtained from a patient prior to being diagnosed with cancer, or a biological sample that has not been contacted with an agent disclosed herein).

As used herein, an "appropriate control" refers to an untreated, otherwise identical cell or population (e.g., a subject who was not administered an agent described herein, or was administered by only a subset of agents described herein, as compared to a non-control cell).

The term "statistically significant" or "significantly" refers to statistical significance and generally means a two standard deviation (2SD) or greater difference.

As used herein the term "comprising" or "comprises" is used in reference to compositions, methods, and respective component(s) thereof, that are essential to the method or composition, yet open to the inclusion of unspecified elements, whether essential or not.

As used herein the term "consisting essentially of" refers to those elements required for a given embodiment. The term permits the presence of elements that do not materially affect the basic and novel or functional characteristic(s) of that embodiment. The term "consisting of" refers to compositions, methods, and respective components thereof as described herein, which are exclusive of any element not recited in that description of the embodiment.

The singular terms "a," "an," and "the" include plural referents unless context clearly indicates otherwise. Similarly, the word "or" is intended to include "and" unless the context clearly indicates otherwise. Although methods and materials similar or equivalent to those described herein can be used in the practice or testing of this disclosure, suitable methods and materials are described below. The abbreviation, "e.g." is derived from the Latin exempli gratia, and is used herein to indicate a non-limiting example. Thus, the abbreviation "e.g." is synonymous with the term "for example."

### BRIEF DESCRIPTION OF THE DRAWINGS

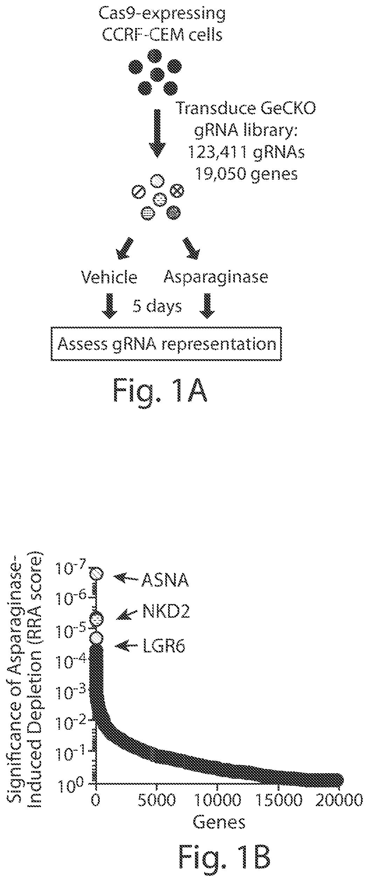

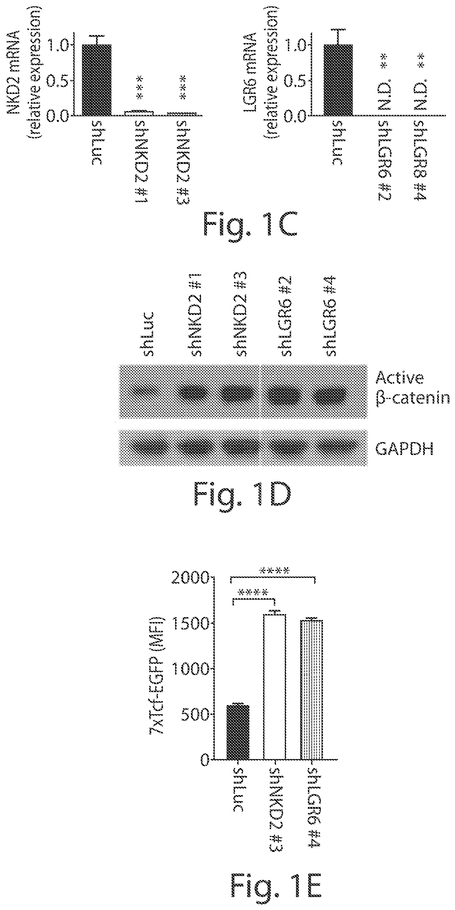

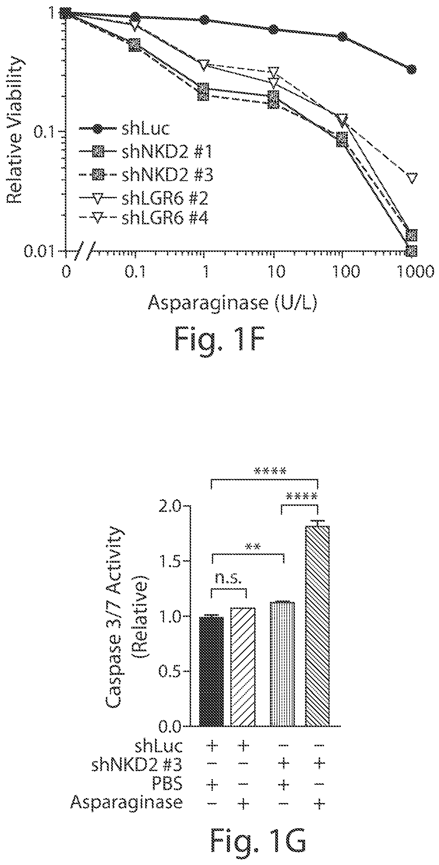

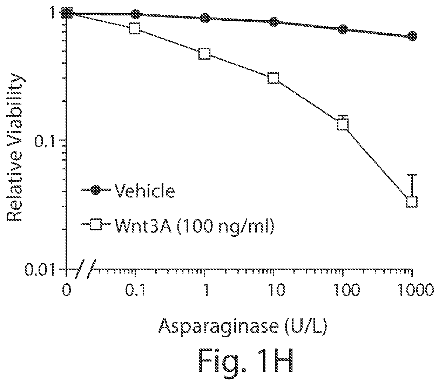

**Figs 1A-1H** show Wnt pathway activation sensitizes leukemia cells to asparaginase. **Fig. 1A** shows that Cas9-expressing CCRF-CEM cells were transduced with the GeCKO genome-wide guide RNA library in biologic duplicates. After puromycin selection, each group was split into treatment with vehicle or asparaginase (10 U/L), and guide RNA representation was assessed after 5 days of treatment. **Fig. 1B** shows that genes covered by the GeCKO library are shown ranked by significance of depletion in asparaginase-treated conditions, as assessed by robust ranking aggregation (RRA) score calculated using MAGeCK analysis. Note that microRNA genes are not shown. **Fig. 1C** shows that CCRF-CEM cells were transduced with the indicated shRNAs, and knockdown efficiency was assessed by RT-PCR analysis for expression of the indicated gene, normalized to β-actin. Note that CT values greater than 36 were defined as not detected (N.D.). Significance was calculated using one-way ANOVA with Dunnett's adjustment for multiple comparisons. **Fig. 1D** shows that CCRF-CEM cells were transduced with the indicated shRNAs, and subjected to Western blot analysis for active (nonphosphorylated) β-catenin (Ser33/37/Thr41) or GAPDH. **Fig. 1E** shows that CCRF-CEM cells were first transduced with a lentiviral 7xTcf-EGFP (TopFLASH) reporter, then transduced with the indicated shRNAs, and reporter-driven EGFP fluorescence was assessed by flow cytometry. Significance was assessed by one-way ANOVA with Dunnett's adjustment for multiple comparisons. **Fig. 1F** shows that CCRF-CEM cells were transduced with the indicated shRNAs, and treated with the indicated doses of asparaginase for 8 days. Relative viability was assessed by counting viable cells based on trypan blue vital dye staining, with cell counts normalized to those in shLuc-transduced, no-asparaginase controls. **Fig. 1G** shows that CCRF-CEM cells were transduced with the indicated shRNAs, treated with asparaginase (10 U/L) for 48 hours, and caspase 3/7 activity assay was assessed. Significance was assessed by two-way ANOVA with Tukey adjustment for multiple comparisons. **Fig. 1H** shows that CCRF-CEM cells were treated with 100 ng/ml Wnt3A ligand or vehicle control, and the indicated doses of asparaginase. The number of viable cells after 8 days of treatment was counted by trypan blue vital dye staining, with results normalized to those in no-Wnt3a, no-asparaginase controls. All bar charts and relative viability curves represent the mean of 3 biologic replicates, with error bars representing standard error of the mean (s.e.m.). * P ≤ 0.05; ** P ≤ 0.01; *** P ≤ 0.001; **** P ≤ 0.0001. n.s., P > 0.05.

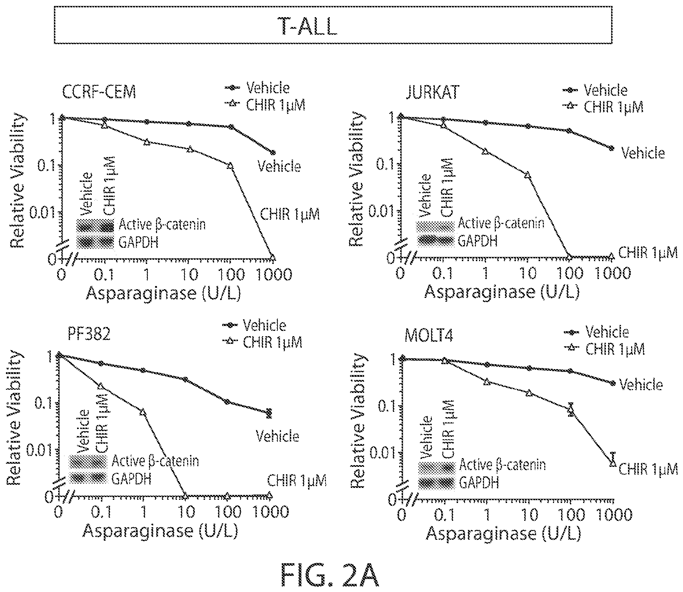

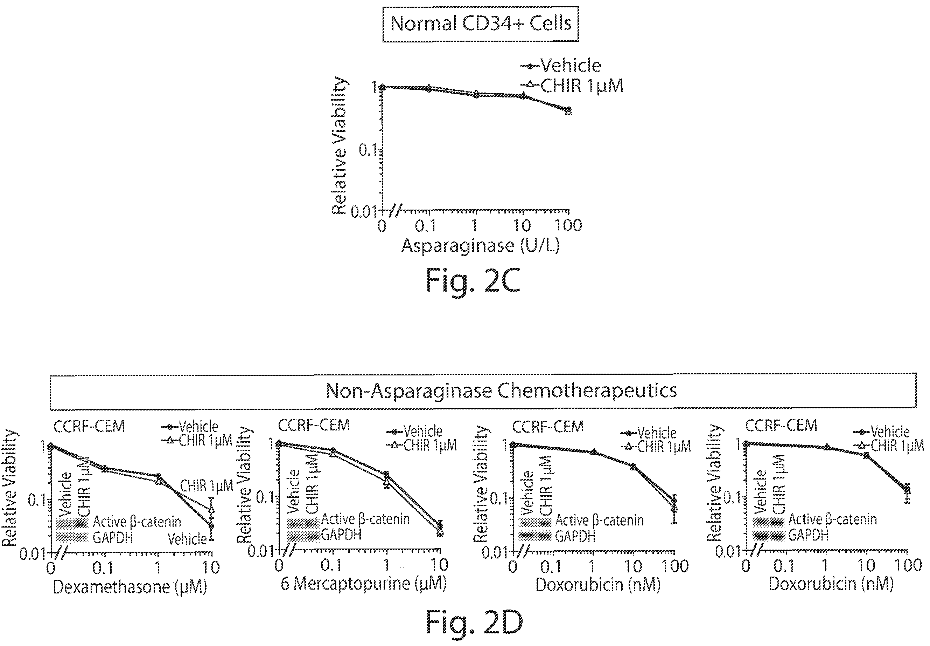

**Figs 2A-2D** show GSK3 inhibition sensitizes distinct acute leukemia subtypes, but not normal hematopoietic progenitors, to asparaginase-induced cytotoxicity. **Fig. 2A-2B****,** The indicated cells were treated with the GSK3 inhibitor CHIR99021 (1 µM) or vehicle control, together with the indicated doses of asparaginase for 8 days. The number of viable cells was counted by trypan blue vital staining, and normalized to counts in no-CHIR, no-asparaginase controls. **Fig. 2C** shows normal CD34+ human hematopoietic progenitor cells were treated with CHIR99021 (1 µM) or vehicle, together with the indicated doses of asparaginase. The number of viable cells was counted by trypan blue vital staining was counted on day 4 of treatment, and normalized to counts in no-CHIR, no-asparaginase controls. Note that these normal hematopoietic progenitors could not be maintained more than 4 days in culture. **Fig. 2D** shows CCRF-CEM cells were treated with CHIR99021 (1 µM) or vehicle, and the indicated chemotherapeutic drugs for 8 days. The number of viable cells was counted by trypan blue dye exclusion, and normalized to counts in no-CHIR, no-chemotherapy controls. All Western Blots shown indicate levels of active (nonphosphorylated) β-catenin (Ser33/37/Thr41) or GAPDH after treatment with CHIR 99021 (1 µM) or vehicle. Results shown are the mean of at least n = 3 biologic replicates, with error bars representing s.e.m.

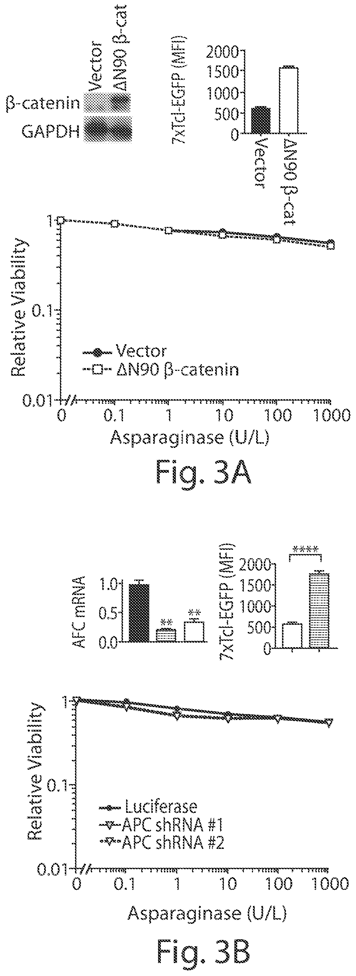

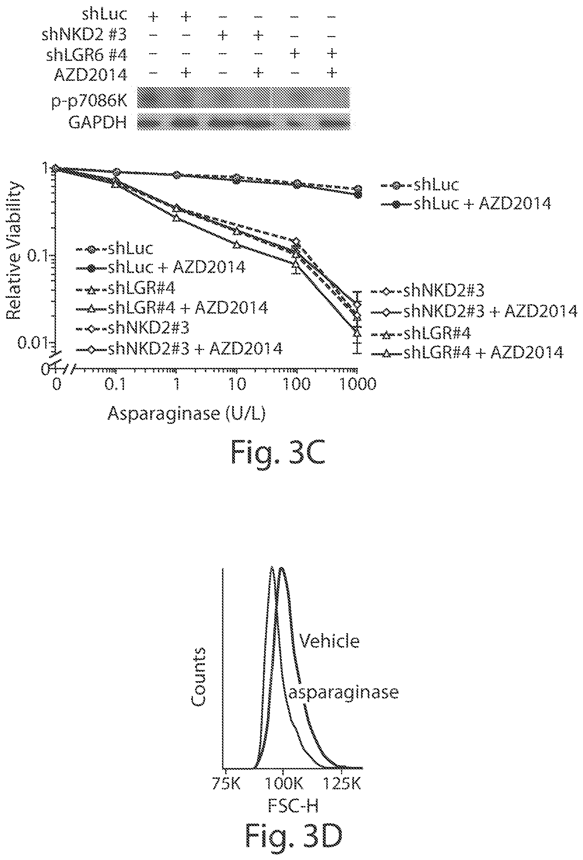

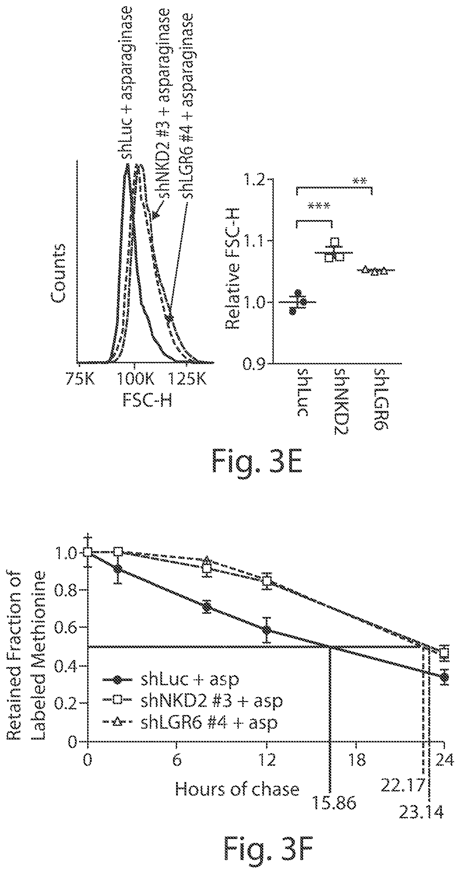

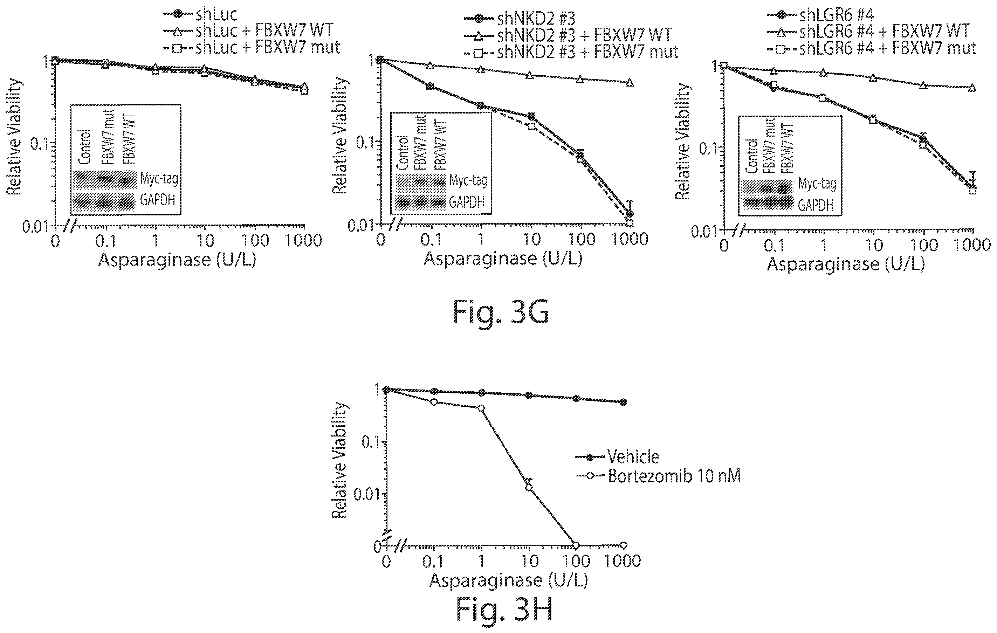

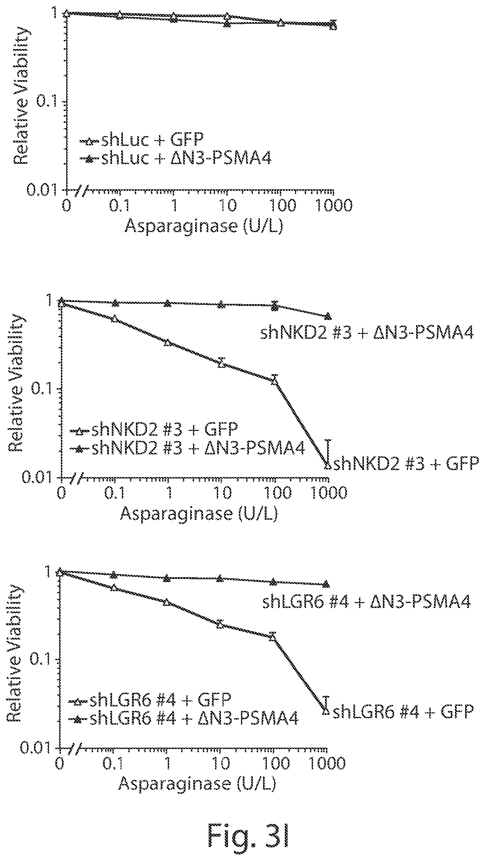

**Figs 3A-3I** show Wnt-dependent stabilization of proteins mediates sensitization to asparaginase. **Fig. 3A** shows that CCRF-CEM cells were transduced with an expression construct encoding a constitutively active ΔN90 β-catenin allele, or vector control. Efficacy of transduction was assessed by Western Blot analysis for the indicated proteins, and effects on canonical Wnt/β-catenin signaling was assessed by 7xTcf-EGFP (TopFLASH) reporter expression (top). Statistical significance assessed by a Welch t-test. Cells transduced with the indicated constructs were then treated with the indicated doses of asparaginase for 8 days, and the number of viable cells was counted by trypan blue vital dye staining (bottom). Viable cell counts were normalized to vector-transduced, no-asparaginase controls. **Fig. 3B** shows that CCRF-CEM cells were transduced with the indicated shRNAs, and effects on APC mRNA and canonical Wnt/β-catenin signaling were assessed by RT-PCR analysis and 7xTcf-EGFP (TopFLASH) reporter activity, respectively (top). Statistical significance was calculated using one-way ANOVA with Dunnett's adjustment for multiple comparisons for three groups and a Welch t-test for two groups. Cells transduced with the indicated shRNAs were treated with the indicated doses of asparaginase for 8 days, and the number of viable cells was counted as in **(****Fig. 3A****).** **Fig. 3C** shows that CCRF-CEM cells were transduced with the indicated shRNAs, and then treated with the dual mTORC1/mTORC2 inhibitor AZD2014 (10 nM) or vehicle control, together with the indicated doses of asparaginase for 8 days. The number of viable cells was counted by trypan blue vital dye staining, and normalized to viable cell counts in no-AZD2014, no-asparaginase controls. Where indicated, transduced cells were treated with AZD2014 and subjected to Western blot analysis for phospho-p70S6 Kinase (Thr389) or GAPDH. **Fig. 3D** shows that CCRF-CEM cells were treated with vehicle or asparaginase (10 U/L) for 48 hours, and cell size was assessed by flow cytometry analysis of forward scatter height (FSC-H). Fig. **3E** shows that CCRF-CEM cells were transduced with the indicated shRNAs, and treated with asparaginase (10 U/L) for 48 hours. Cell size was assessed by flow cytometry analysis of forward scatter height (FSC-H). Significance was assessed by one-way ANOVA with Dunnett's adjustment for multiple comparisons. **Fig. 3F** shows that CCRF-CEM cells were transduced with the indicated shRNAs, incubated with an 18-hour pulse of the cell-permeable methionine analog azidohomoalanine, and then released from the label and treated with asparaginase (10 U/L). Azidohomoalanine was fluorescently labeled by click chemistry, and the degree of label retention was measured by flow cytometry at the indicated time points. **Fig. 3G** shows that CCRF-CEM cells were transduced with the indicated shRNAs. Where indicated, cells were also transduced with expression constructs encoding wild-type FBXW7, or a R465C FBXW7 point mutant with impaired substrate binding ability. Transduction efficiency was assessed by Western blot analysis for the indicated proteins. Cells were then treated with the indicated doses of asparaginase for 8 days, and the number of viable cells was counted by trypan blue vital staining. Cell counts were normalized to those in shLuc-transduced, no-asparaginase controls. **Fig. 3H** shows that CCRF-CEM cells were treated with vehicle or the proteasome inhibitor bortezomib, and the indicated doses of asparaginase for 8 days. The number of viable cells was counted by trypan blue vital staining, and normalized to cell counts in no-bortezomib, no-asparaginase controls. **Fig. 3I** shows that CCRF-CEM cells were transduced with the indicated shRNAs, and expression constructs encoding GFP control, or a hyperactive mutant of the proteasomal subunit PSMA4 (ΔN3-PSMA4) or vehicle control. Cells were then treated with the indicated doses of asparaginase for 8 days, and the number of viable cells was counted by trypan blue vital staining. Cell counts were normalized to those in GFP-transduced, no-asparaginase controls. Results shown are the mean of at least n = 3 biologic replicates, with error bars representing s.e.m. * P ≤ 0.05; ** P ≤ 0.01; *** P ≤ 0.001; **** P ≤ 0.0001. n.s., P > 0.05.

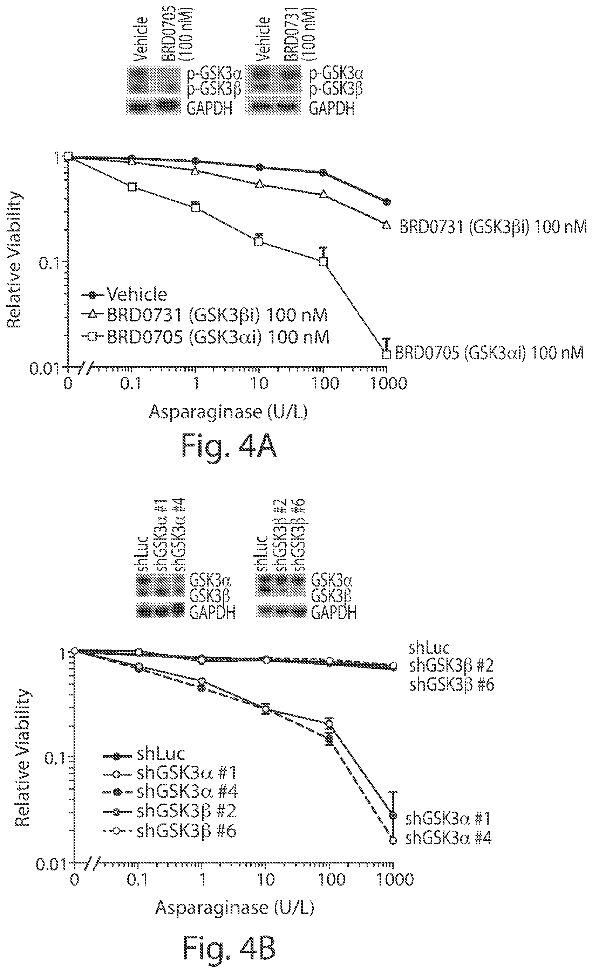

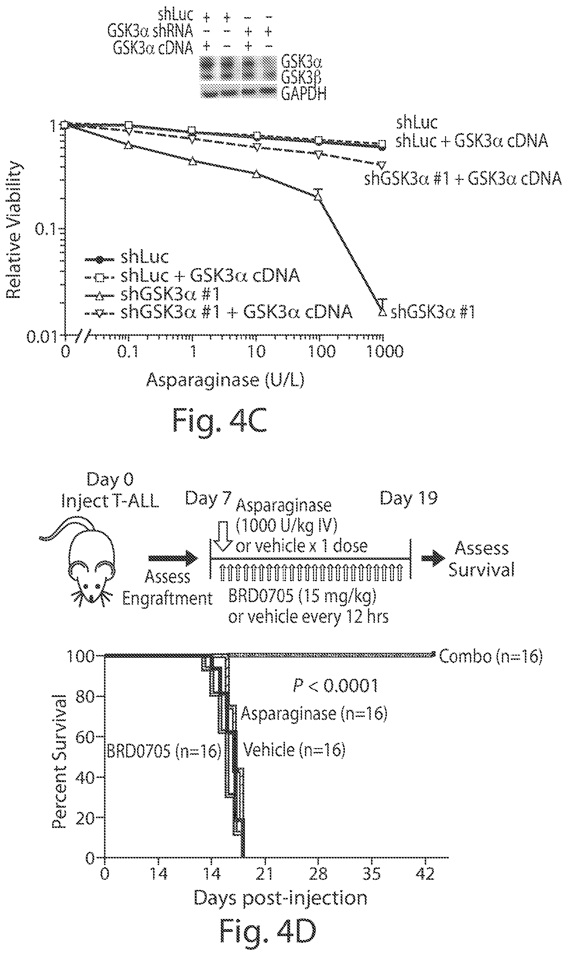

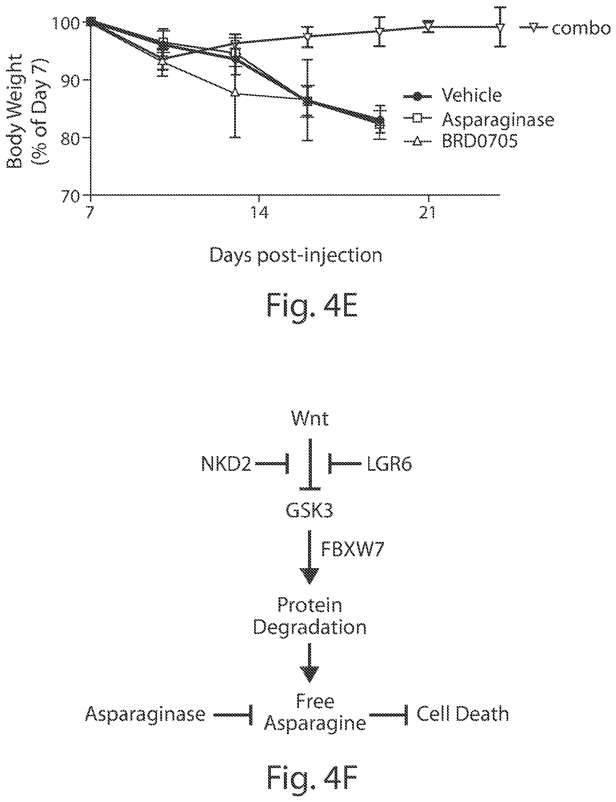

**Figs 4A-4F** show synthetic lethality of GSK3α inhibition and asparaginase in human leukemia. **Fig.** 4A shows that CCRF-CEM cells were treated with vehicle, the GSK3α-selective inhibitor BRD0705, or the GSK3β-selective inhibitor BRD0731, together with asparaginase at the indicated doses for 8 days. The number of viable cells was counted by trypan blue vital staining, and normalized to counts in vehicle-treated controls. Protein levels of the indicated proteins were assessed by Western blot analysis (inset). The phospho-specific GSK3 antibody (p-GSK3) recognizes autophosphorylation at Tyr279 of GSK3α and Tyr216 of GSK3β²⁷. Note that the GSK3 paralogs are distinguishable by molecular weight. **Fig. 4B** shows that CCRF-CEM cells were transduced with the indicated shRNAs, and knockdown efficacy was assessed by Western blot analysis (inset) for total GSK3 or GAPDH. Cells were then treated with the indicated doses of asparaginase for 8 days, and viable cell counts based on trypan blue vital staining were performed. Cell counts were normalized to shLuc, no-asparaginase controls. **Fig. 4C** shows that CCRF-CEM cells were transduced with the indicated shRNAs, without or with a GSK3α expression construct that escapes shRNA targeting. Expression of the indicated proteins was assessed by Western blot analysis (inset) for total GSK3 or GAPDH. Cells were then treated with the indicated doses of asparaginase for 8 days, and the number of viable cells was counted by trypan blue vital staining. Cell counts were normalized to those in shLuc, no-asparaginase controls. **Fig. 4D** shows that T-ALL cells from a patient-derived xenograft were injected into NRG immunodeficient mice. After engraftment of leukemia (≥5% leukemic cells in the peripheral blood), mice were treated with asparaginase or vehicle intravenously x 1 dose, and BRD0705 or vehicle by oral gavage every 12 hours for 12 days. Survival was calculated by Kaplan-Meier analysis. P value calculated by log-rank test comparing vehicle-treated mice to those treated with the combination of asparaginase and BRD0705. Differences in survival of mice treated with vehicle, asparaginase alone, or BRD0705 alone were not significant. Differences between each of these groups and mice treated with the combination of asparaginase and BRD0705 (combo) were all P < 0.0001. **Fig. 4E** shows the weights of mice in the experiment shown in **(****Fig. 4D****).** **Fig. 4F** shows the proposed model.

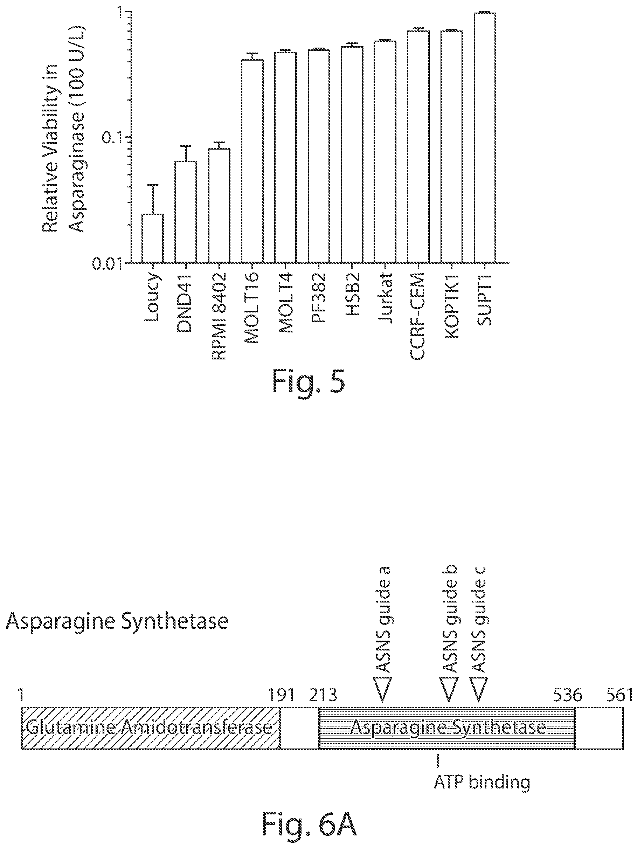

**Fig. 5** shows that asparaginase sensitivity of human T-ALL cell lines. The indicated human T-ALL cell lines were treated with 100 U/L asparaginase or vehicle control for 48 hours, and viability was assessed by counting viable cells based on trypan blue vital dye staining. For each cell line, results were normalized to viability in vehicle-treated cells. Bar charts represent the mean of duplicate experiments. For the genetic screen, CCRF-CEM cells were selected because efficient CRISPR/Cas9 genome editing was unable to be achieved in SUPT1 or KOPTK1 cells.

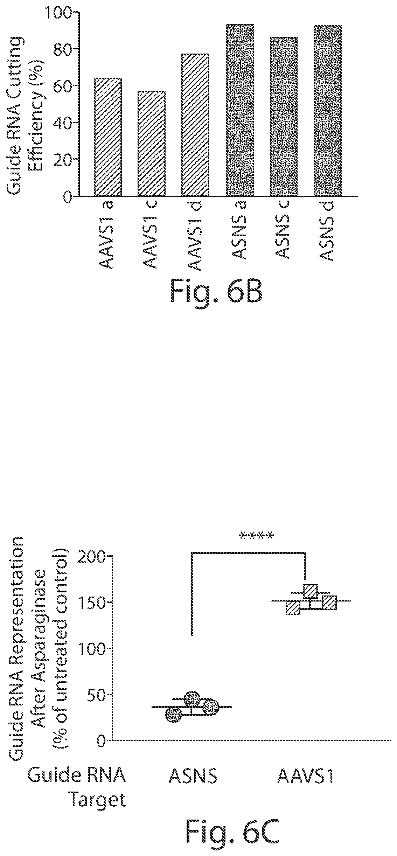

**Figs 6A-6C** show asparagine synthetase (ASNS) is a positive control for depletion in asparaginase-treated CCRF-CEM cells. **Fig. 6A** shows the predicted location of the mutations induced by positive control guide RNAs targeting the asparagine synthetase catalytic domain are shown on the ASNS protein (https://www.uniprot.org/uniprot/P08243). **Fig. 6B** shows that Cas9-expressing CCRF-CEM cells were transduced with positive control guide RNAs targeting the catalytic domain of asparagine synthetase (ASNS), or negative controls targeting the safe-harbor genomic locus AAVS1, and cutting efficiency was assessed by PCR amplification and next-generation sequencing of the target locus. **Fig. 6C** shows that Cas9-expressing CCRF-CEM cells were transduced with a pool of guide RNAs targeting ASNS or AAVS1 (n = 3 guide RNAs targeting each locus), and subsequently treated with vehicle (PBS) or 10 U/L of asparaginase. After 5 days of treatment, guide RNA representation was assessed by next-generation sequencing. Abundance of each guide RNA was normalized to its abundance in vehicle-treated cells. Bars indicate the mean +/- standard error of the mean and statistical significance was calculated using a Welch t-test. ****, P < 0.0001. n.s., P ≥ 0.05

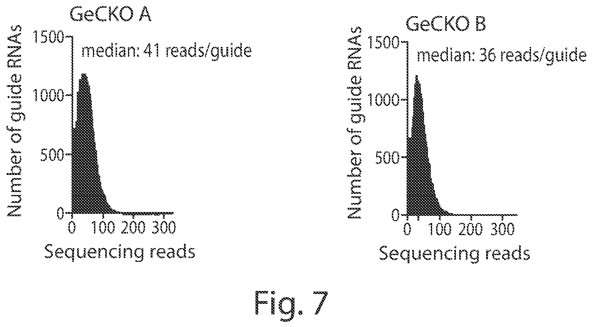

**Fig. 7** shows the guide RNA representation within the genome-wide guide RNA libraries utilized. The GeCKO genome-wide guide RNA library, which is maintained in two distinct half-library pools (A and B), were amplified, and guide RNA representation with each half-library was assessed using next-generation sequencing.

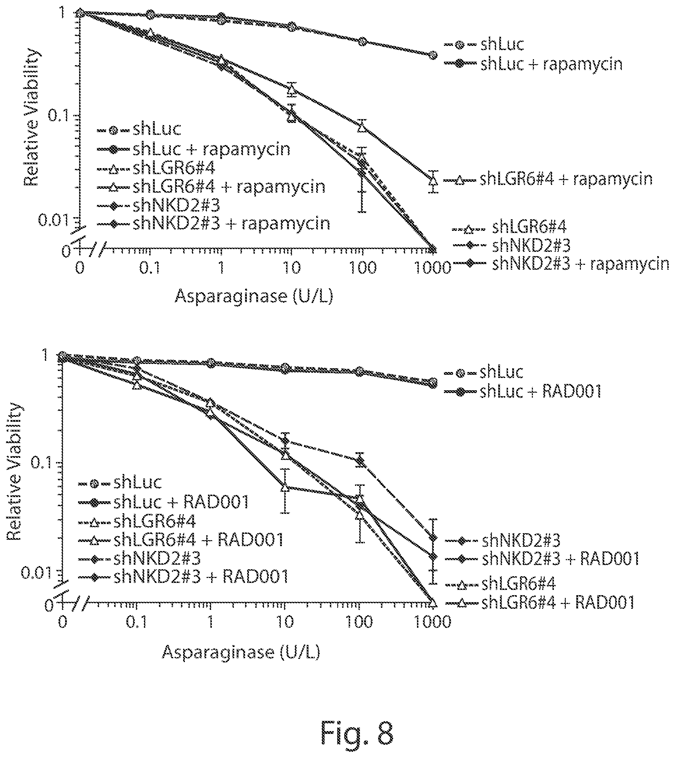

**Fig. 8** shows that mTORC1 inhibition does not rescue sensitization to asparaginase. CCRF-CEM cells were infected with indicated shRNAs and treated with indicated doses of asparaginase and rapamycin (1 nM, left) or RAD001 (10 nM, right) for 8 days. Relative viability was assessed by counting viable cells using trypan blue vital dye staining, with cell counts normalized to those in shLuc-transduced controls (no-asparaginase, no mTORC1 inhibitor). Relative viability curves represent the mean of 3 biologic replicates, with error bars representing s.e.m.

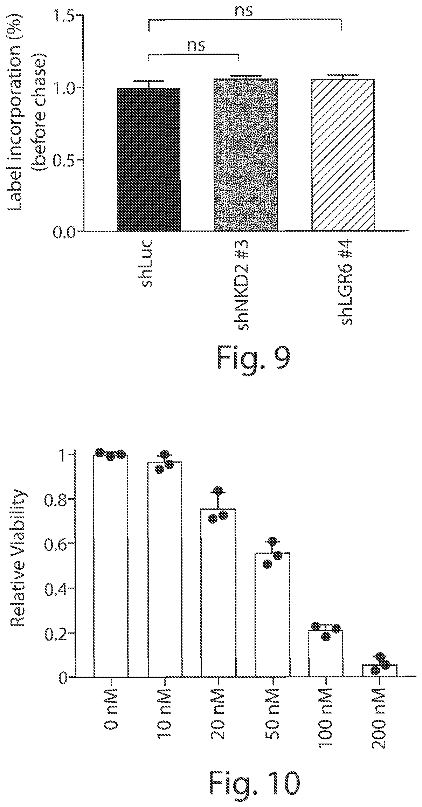

**Fig. 9** shows that wnt pathway activation does not affect degree of label incorporation during the pulse. CCRF-CEM cells transduced with indicated shRNAs were incubated with azidohomoalanine (AHA) for 18 hours. After the pulse, cells were subsequently fixed and fluorescence intensity of tagged AHA assessed by flow cytometry. Results shown are the mean of n = 3 biologic replicates, with error bars representing s.e.m. Statistical significance was assessed by a one-way ANOVA with Dunnett's adjustment for multiple comparisons. * P ≤ 0.05; ** P ≤ 0.01; *** P ≤ 0.001; **** P ≤ 0.0001. n.s., P > 0.05.
**Fig. 10** shows that bortezomib is highly toxic at intermediate doses. CCRF-CEM cells were treated with indicated doses of bortezomib for 48 hours. Cell viability was assessed by counting viable cells based on trypan blue vital dye staining with cell counts normalized to no-bortezomib controls. Bar charts represent the mean of triplicate experiments, with dots representing results of each individual experiment.

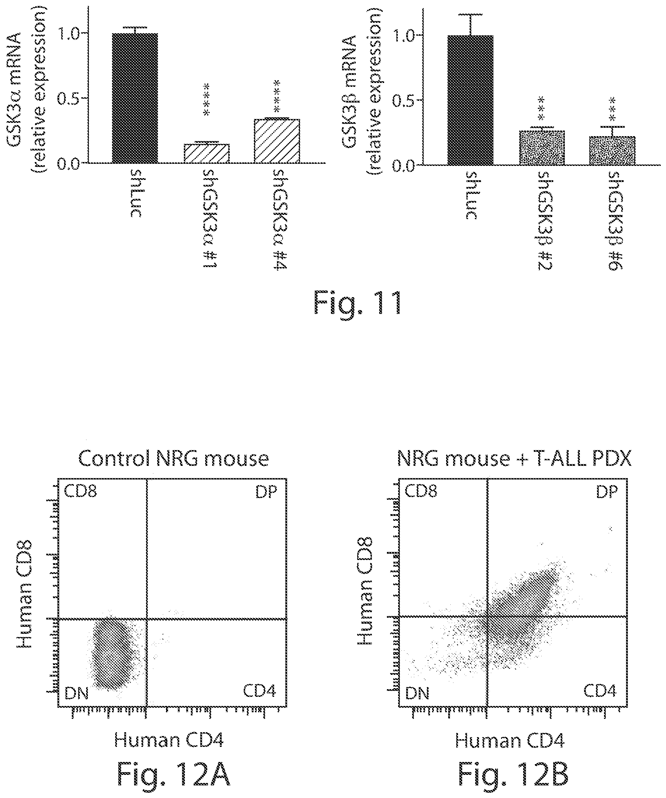

**Fig. 11** shows that shRNA knockdown leads to significant decrease in mRNA expression of GSK3α (left) and GSK3β (right). Quantitative reverse transcriptase PCR (qRT-PCR) for the indicated genes was performed to test knockdown efficiency. Results were normalized to shLuc cells. Significance was assessed by one-way ANOVA with Dunnett's adjustment for multiple comparisons. Error bars represent s.e.m. of 3 biologic replicates. * P ≤ 0.05 ** P ≤ 0.01 *** P ≤ 0.001 **** P ≤ 0.0001. n.s., P > 0.05.
**Figs 12A and 12B** show confirmation of leukemic burden. **Fig. 12A** shows bone marrow cells were harvested from control NRG mice. **Fig. 12B** shows NRG mice injected with the human T-ALL PDX and treated with vehicle (from **Fig. 4D****).** Cells were stained with the indicated antibodies and positivity for human cells was assessed by flow cytometry.

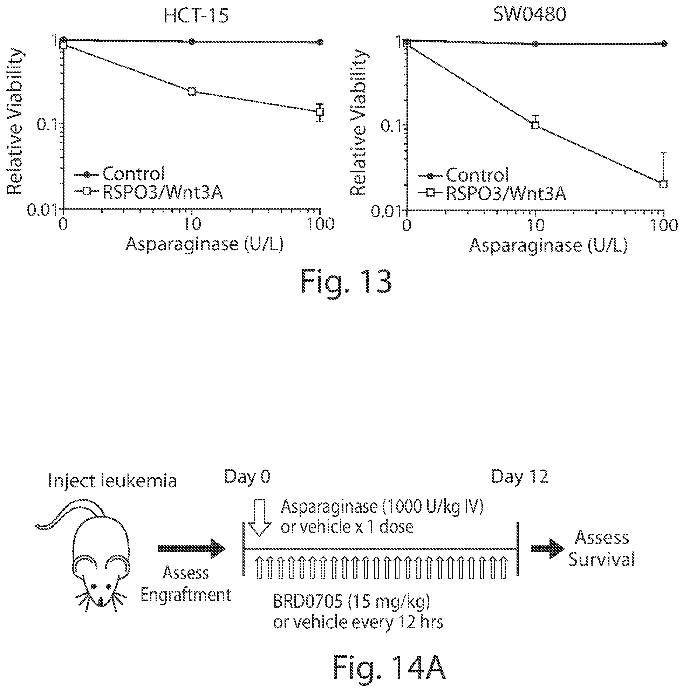

**Fig. 13** shows activation of Wnt/STOP signaling sensitizes colon cancer cells to asparaginase. The indicated human colon cancer cell lines utilized harbor mutations of APC that activate canonical Wnt/β-catenin signaling downstream of GSK3, and thus do not have active Wnt/STOP signaling at baseline. Cells were treated with vehicle or human recombinant RSPO3 (75 ng/ml) and Wnt3A (100 ng/ml) to induce ligand-dependent activation of Wnt receptor signaling, which activates Wnt/STOP signaling, and asparaginase at the indicated doses for 8 days. Note that activating Wnt/STOP signaling by treatment with RSPO3 and Wnt3A induces asparaginase sensitization.

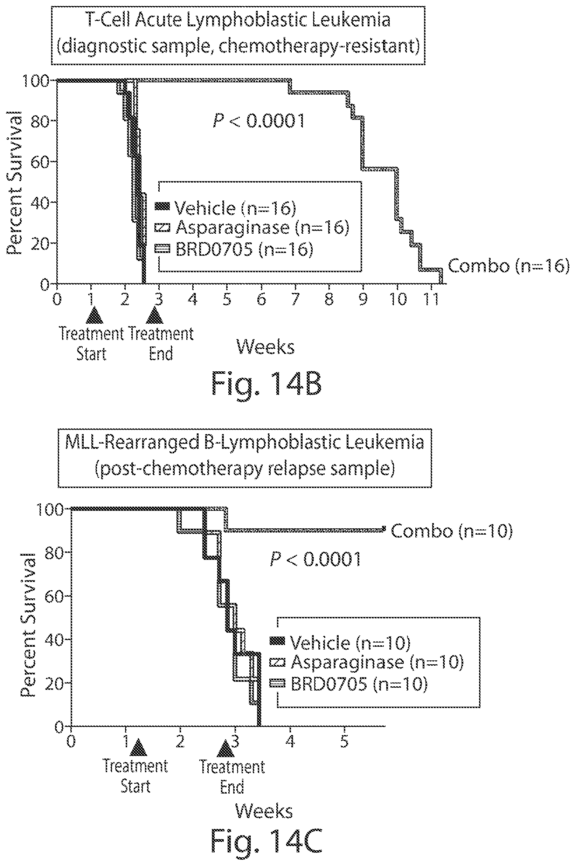

**Figs 14A-14C** show profound therapeutic activity of GSKα-selective inhibitor in combination with an asparaginase. **Fig. 14A** shows a schematic of dosing for the GSKα-selective inhibitor and asparaginase. The asparaginase is administered to the mouse in a single dose at 1000U/kg via I.V. administration. The GSKα-selective inhibitor (BRD0705) or the vehicle are administered to the mouse every 12 hours at 15mg/kg. **Fig. 14B** shows percent survival of immunodeficient mice engrafted with human T-Cell Acute Lymphoblastic Leukemia (chemotherapy-resistant) mice. **Fig. 14C** shows percent survival of immunodeficient mice engrafted with human MLL-Rearranged B-Lymphoblastic Leukemia (sample collected from a patient at relapse post-chemotherapy). Survival is greatly increase in both leukemia models when the GSKα-selective inhibitor and asparaginase is administered in combination, as compared to each as a monotherapy.

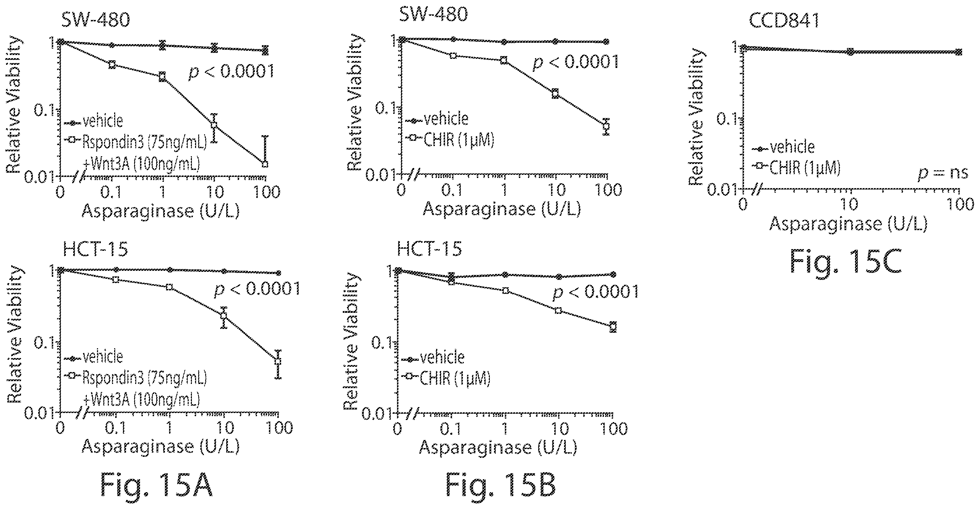

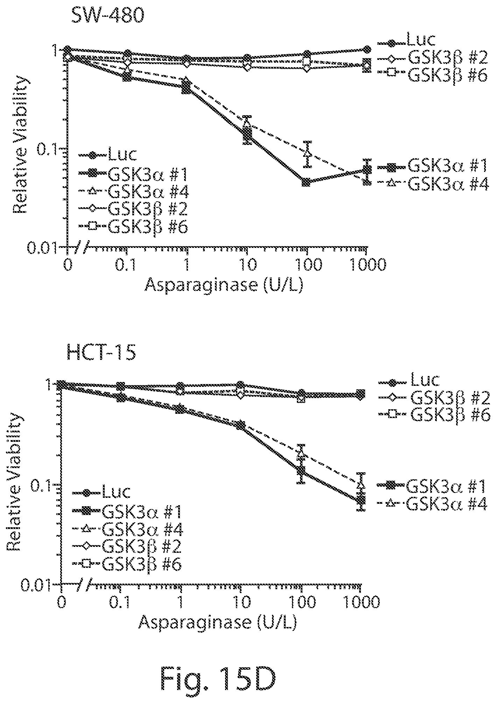

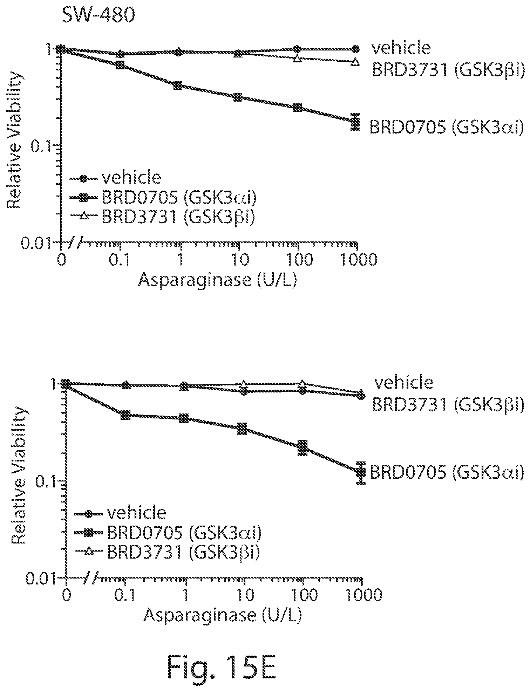

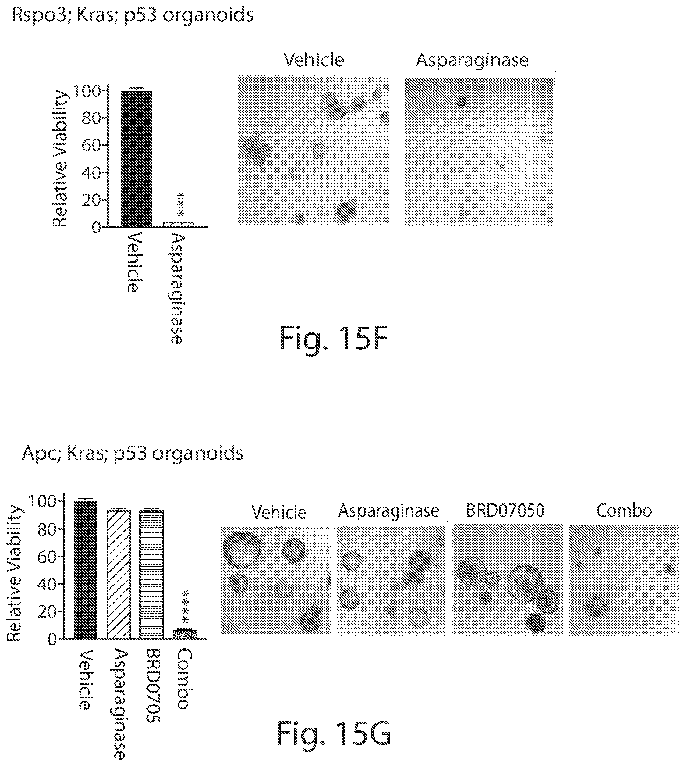

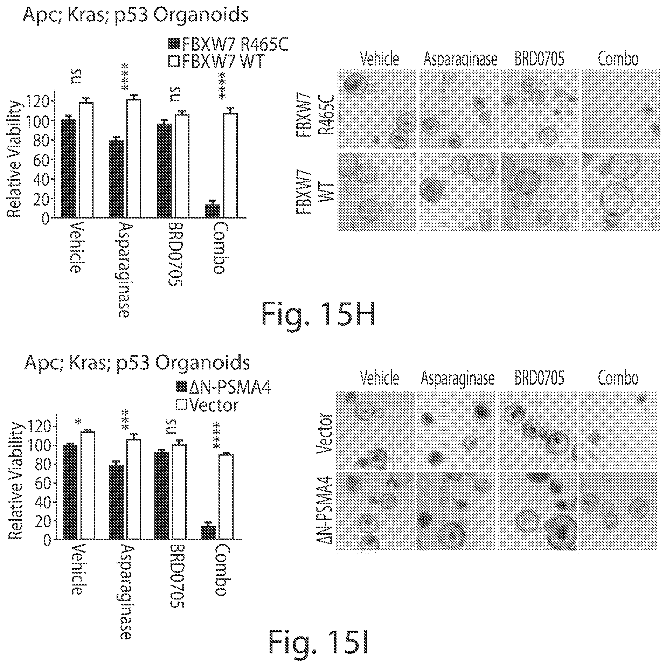

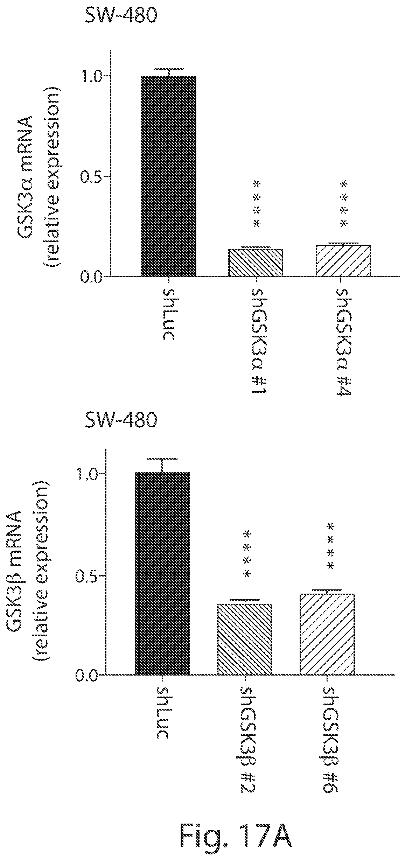

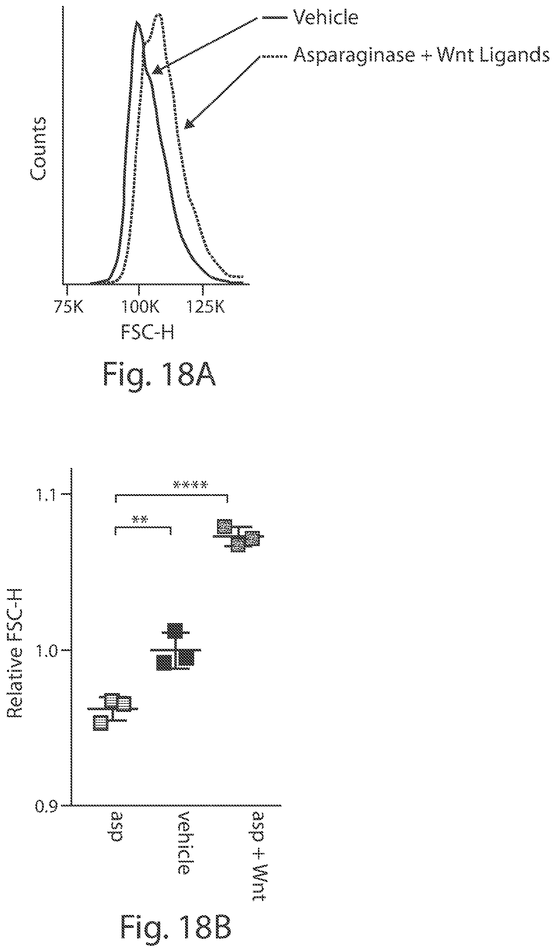

**Figs 15A-15I** shows mutations that activate Wnt signaling upstream of GSK3 induce asparaginase hypersensitivity. **Fig. 15A** shows SW-480 and HCT-15 cells were treated with the indicated doses of asparaginase for 10 days in the presence of recombinant Rspondin3 (75 ng/ml) and Wnt3a (100 ng/ml) or vehicle. The number of viable cells was counted and all cell counts were normalized to those in vehicle, no-asparaginase cells. Two-way ANOVA was performed for each cell line and included interaction terms between asparaginase doses and Wnt ligands. The p value for the main effect of Wnt ligands vs. vehicle is presented in each plot and the interaction terms were overall significant (p < 0.0001). **Fig. 15B** shows SW-480 and HCT-15 cells were treated with the GSK3 inhibitor CHIR99021 (CHIR, 1µM) or vehicle, together with the indicated doses of asparaginase for 10 days. Viability was assessed as in (Fig. 15A). Statistical significance was calculated using a two-way ANOVA and included interaction terms between asparaginase doses and GSK3 inhibitor. The p value for the main effect of Wnt ligands vs. vehicle is presented in each plot and all interaction terms were significant (p < 0.0001). **Fig. 15C** shows CCD-841 cells derived from normal human colonic epithelium were treated with CHIR99021 (1 µM) or vehicle, together with the indicated doses of asparaginase for 10 days, and viable cell counts were assessed as described in (Fig. 15A). Two-way ANOVA was performed for each cell line and included interaction terms between asparaginase dose and GSK3 inhibitor. The p value for the main effect of CHIR99021 vs. vehicle is presented in the plot and all interaction terms were not significant (p = n.s.). **Fig. 15D** shows SW-480 and HCT-15 cells were transduced with the indicated shRNAs and then treated with the indicated doses of asparaginase. Viability was assessed after 10 days of treatment by counting viable cells. All cell counts were normalized to those in shLuc-transduced, vehicle-treated controls. **Fig. 15E** shows the indicated cell lines were treated with vehicle, the GSK3α-selective inhibitor BRD0705 (1 µM), or the GSK3β-selective inhibitor BRD3731 (1 µM), in the presence of the indicated doses of asparaginase for 10 days. Viability was assessed as in (Fig. 15A). **Fig. 15F** shows mouse intestinal organoids with mutations of both *p53* and *Kras,* together with *Rspo3* translocation were cultured in basal organoid medium (without Wnt3A and Rspondin1 proteins) and treated with vehicle or asparaginase (100 U/L) for 10 days. Viability was assessed by counting viable organoids using an Axio Imager A1 microscope. Images were taken from a representative of three experiments and analyzed with ImageJ software. Statistical significance was calculated using a two-sided Welch t-test. **Fig. 15G** shows *Apc* deficient organoids with mutations of *p53* and *Kras* were cultured in basal organoid medium (not containing Wnt3A and Rspondin 1 proteins) and treated with vehicle, asparaginase (100 U/L), BRD0705 (1 µM) or combo (100 U/L asparaginase + 1 µM BRD0705) for 10 days. Viability was assessed as in (Fig. 15F). Images were taken from a representative of three experiments and analyzed with ImageJ software. Differences between groups were analyzed using a one-way ANOVA with Dunnett's adjustment for multiple comparisons, using the vehicle group as the reference group. **** p ≤ 0.0001. n.s., p > 0.05. **Fig. 15H** shows indicated organoids were cultured in basal organoid medium, transduced with the indicated constructs and treated with vehicle, asparaginase (100 U/L), BRD0705 (1 µM) or combo (100 U/L asparaginase + 1 µM BRD0705) for 10 days. Viability was assessed as in (Fig. 15F). Images were taken from a representative of three experiments and analyzed with ImageJ software. Differences between groups were analyzed using a one-way ANOVA with Tukey adjustment for multiple comparisons. **** p ≤ 0.0001. n.s., p > 0.05. **Fig. 15I** shows indicated organoids were cultured in basal organoid medium, transduced with the indicated constructs and treated with vehicle, asparaginase (100 U/L), BRD0705 (1 µM) or combo (100 U/L asparaginase + 1 µM BRD0705) for 10 days. Viability was assessed as in (Fig. 15F). Images were taken from a representative of three experiments and analyzed with ImageJ software. Differences between groups were analyzed using a one-way ANOVA with Tukey adjustment for multiple comparisons. * p ≤ 0.05; *** p ≤ 0.001; **** p ≤ 0.0001. n.s., p > 0.05. All error bars represent SEM. See also Fig. 17A-17D and Fig. 18A-18B.

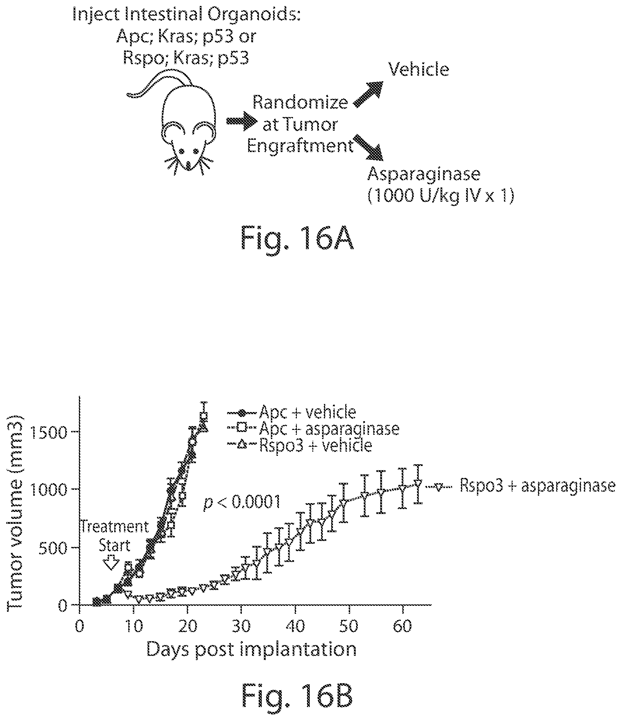

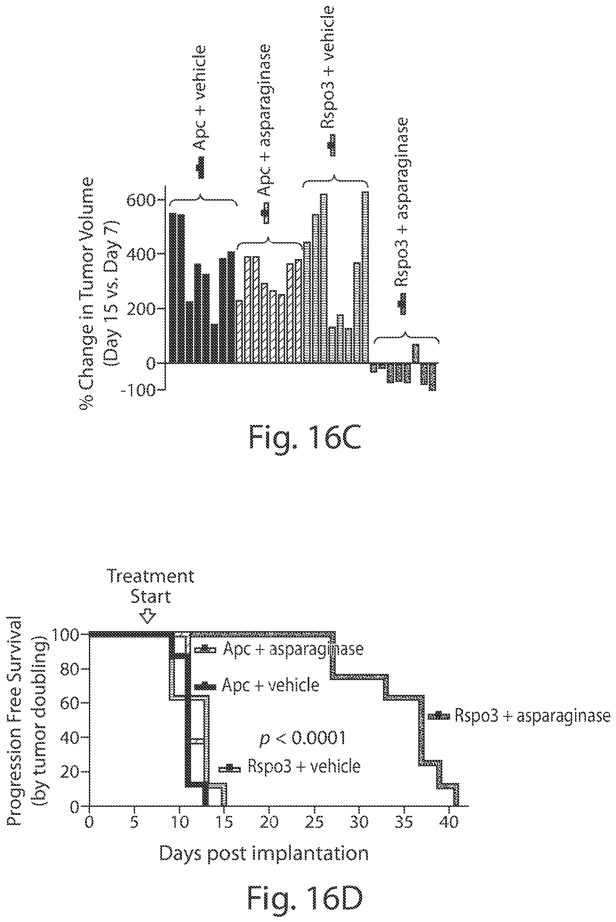

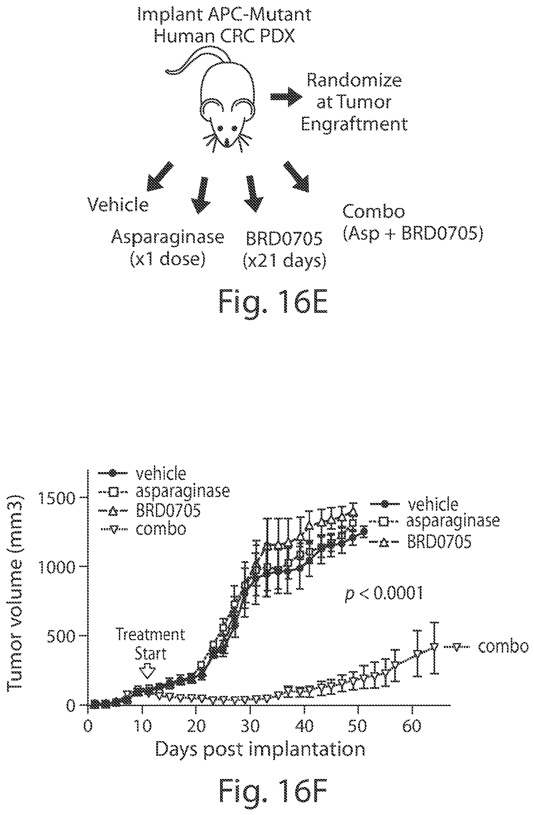

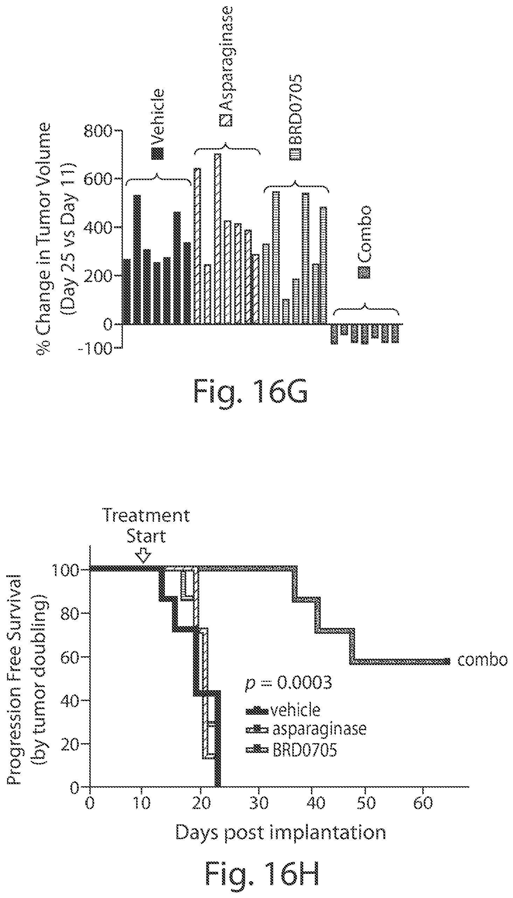

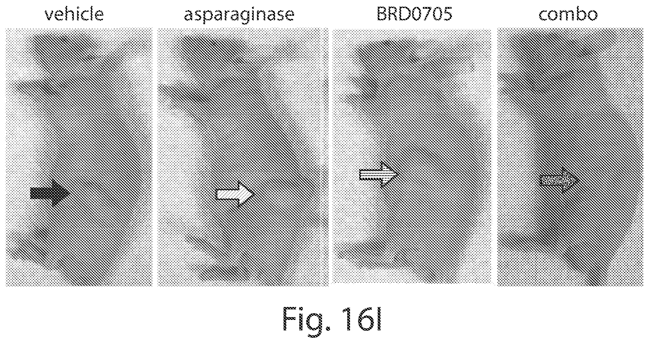

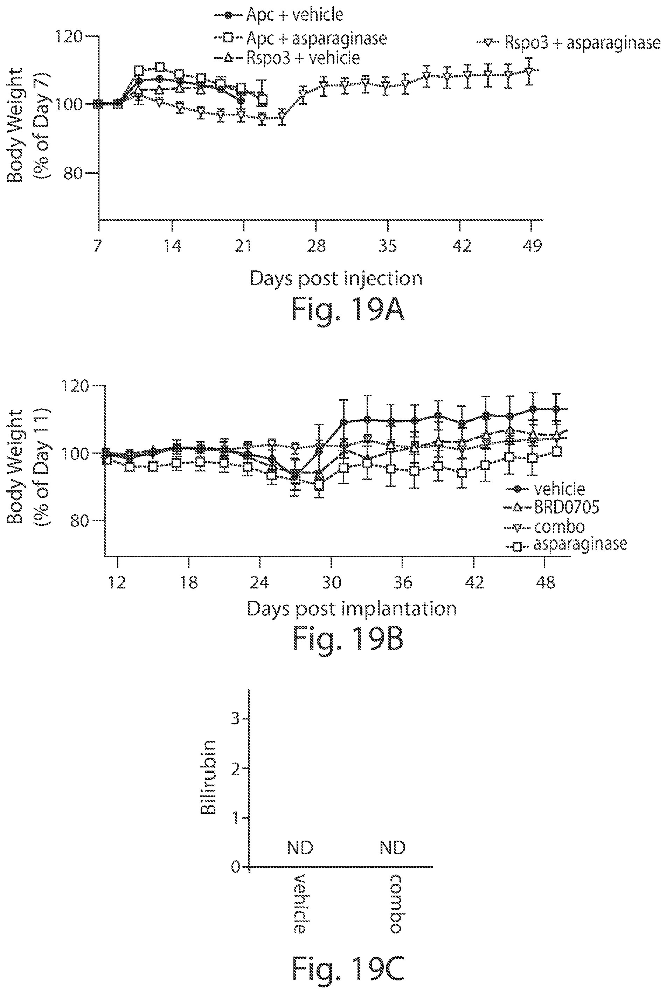

**Figs 16A-16I** shows methods of leveraging synthetic lethality of Wnt pathway activation and asparaginase in colorectal cancer. **Fig. 16A** shows experimental schema. Mouse intestinal organoids with mutations of both *p53* and *Kras,* together with an *Rspo3* fusion or *Apc* deficiency were injected subcutaneously into male nude mice (n=9 per group). Once tumor engraftment was confirmed (>100 mm³ tumor volume by caliper measurements), mice were randomly assigned to treatment groups, and treated as indicated. **Fig. 16B** shows tumor volumes of mice injected with intestinal organoids in the experiment shown in (Fig. 16A). Treatment start is denoted by the arrowhead on the graph and tumor volumes were assessed every other day by caliper measurements. Two-way ANOVA was performed for mice implanted with Rspo3-fusion organoids and included interaction terms between vehicle and asparaginase. The p value for the main effect of asparaginase vs. vehicle after start of treatment is presented in the plot. All error bars represent SEM. **Fig. 16C** shows waterfall plots of response to asparaginase treatment across mice injected with indicated organoids; each bar represents an individual mouse. **Fig. 16D** shows Kaplan-Meier progression-free survival curve of mice injected with indicated organoids and treated with vehicle or asparaginase. The log rank test was used to test for progression-free survival differences in mice injected with Rspo3-fusion organoids and treated with asparaginase vs. vehicle. **Fig. 16E** shows experimental schema. A human patient-derived APC-mutant CRC xenograft was implanted subcutaneously into male nude mice (n=8 per group). Mice were treated at the time of tumor engraftment as described in (Fig. 16A). **Fig. 16F** shows tumor volumes of mice implanted with the human CRC PDX in the experiment shown in (Fig. 16E). Arrowheads denote treatment start and end points. Tumor volumes were assessed every other day by caliper measurements. Two-way ANOVA was performed and included interaction terms between vehicle and combo. The p value for the main effect of combo vs. vehicle after start of treatment is presented in the plot and all interaction terms were overall significant (p < 0.0001). All error bars represent SEM. **Fig. 16G** shows waterfall plot of response to asparaginase, BRD0705 or combo treatment in mice implanted with the human CRC PDX; each bar represents an individual mouse. **Fig. 16H** shows Kaplan-Meier progression-free survival curve of mice treated with asparaginase, BRD0705 or combo. The log rank test was used to test for progression-free survival differences in combo vs. vehicle treated mice. **Fig. 16I** shows representative images of mice implanted with the APC-mutant CRC PDX from the experiment shown in Figure 16E. Images were taken 30 days post treatment from mice anesthetized with isoflurane. See also Fig. 19A-19C.

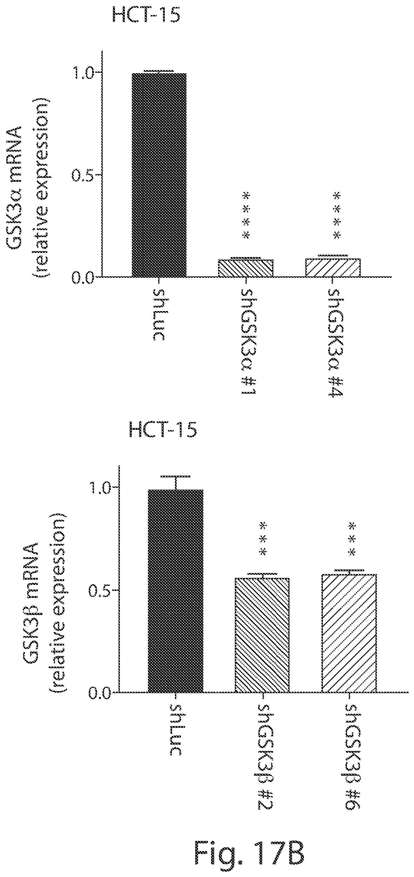

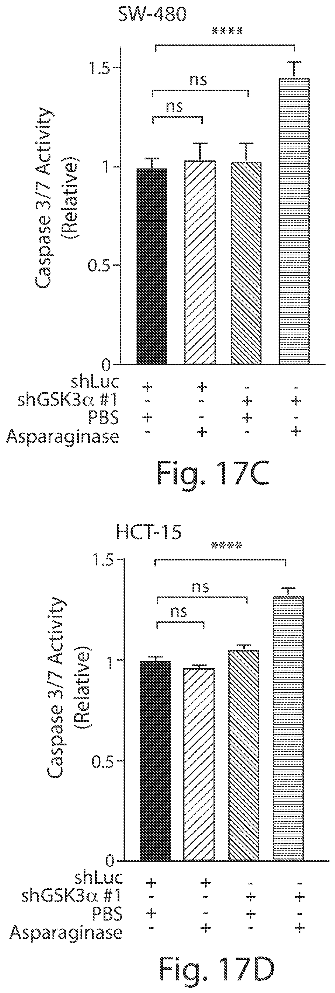

**Figs 17A-17D** (related to Fig. 15A-15I) shows GSK3α inhibition and Asparaginase induce mitochondrial apoptosis in colorectal cancer cells. **Fig. 17A** shows SW-480 cells were transduced with the indicated constructs and knockdown efficiency was assessed by RT-PCR analysis. Statistical analysis was calculated using a one-way ANOVA with Dunnett's adjustment for multiple comparisons. **Fig. 17B** shows HCT-15 cells were infected with the indicated constructs and knockdown efficiency was assessed as described in (Fig. 17A). **Fig.** 17C shows SW-480 cells were infected with shLuciferase or shGSK3α, and subsequently treated with vehicle (PBS) or asparaginase (100 U/L). Caspase 3/7 activity was assessed using the Caspase Glo 3/7 Assay and normalized to shLuciferase, vehicle control. Statistical Significance was calculated by a one-way ANOVA with Dunnett's adjustment for multiple comparisons. **Fig. 17D** shows HCT-15 cells were infected with the indicated constructs and treated with vehicle (PBS) or asparaginase (100 U/L). Caspase activity was assessed as described in (Fig. 17A). **** p ≤ 0.0001. n.s., p > 0.05.
**Fig. 18A-18B** (related to Fig. 15A-15I) shows treatment with Wnt ligands reverses asparaginase-Induced decrease in cell size. **Fig. 18A** shows HCT-15 cells were treated with vehicle or asparaginase (100 U/L) together with human RSpondin3 (75 ng/ml) and Wnt3A protein (100 ng/ml), and cell size was assessed by flow cytometry forward scatter hight (FSC-H) on a Beckton-Dickinson LSR-II instrument. **Fig. 18B** shows scatter plot depicts results of individual biologic replicates, with horizontal bars indicating mean, and error bars indicating SEM. Differences between groups were analyzed using a one-way ANOVA with Dunnett's adjustment for multiple comparisons. ** p ≤ 0.01; **** p ≤ 0.0001.
**Fig. 19A-19C** (related to Fig. 16A-16I) shows treatment is well-tolerated and does not induce weight loss in nude mice. **Fig. 19A** shows body weights of mice injected with organoids and treated as indicated. Values were normalized to the body weight before start of treatment. Error bars represent SEM. **Fig. 19B** shows body weights of mice implanted with a human CRC PDX. Values were normalized to the body weight before start of treatment. Error bars represent SEM. **Fig. 19C** shows bilirubin levels of mice inplanted with the human CRC PDX and treated with vehicle or combination treatment (asparaginase + BRD0705).

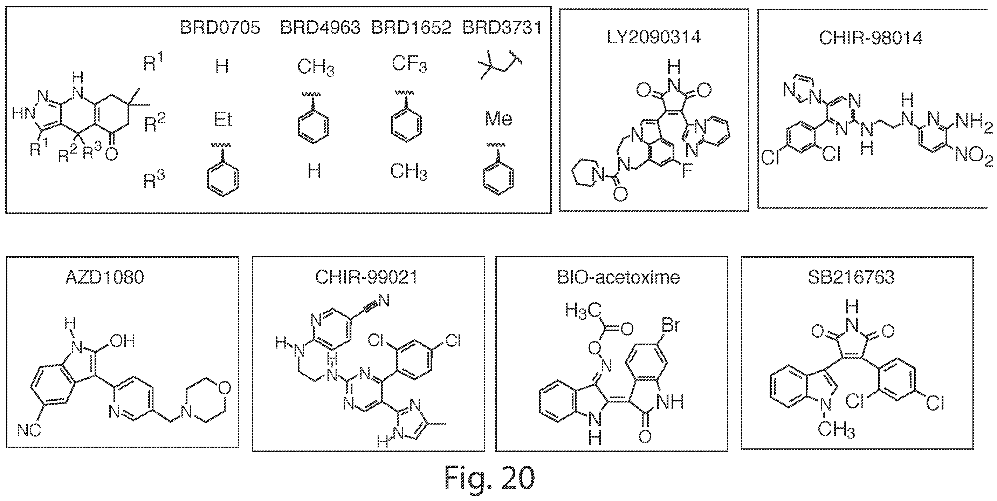

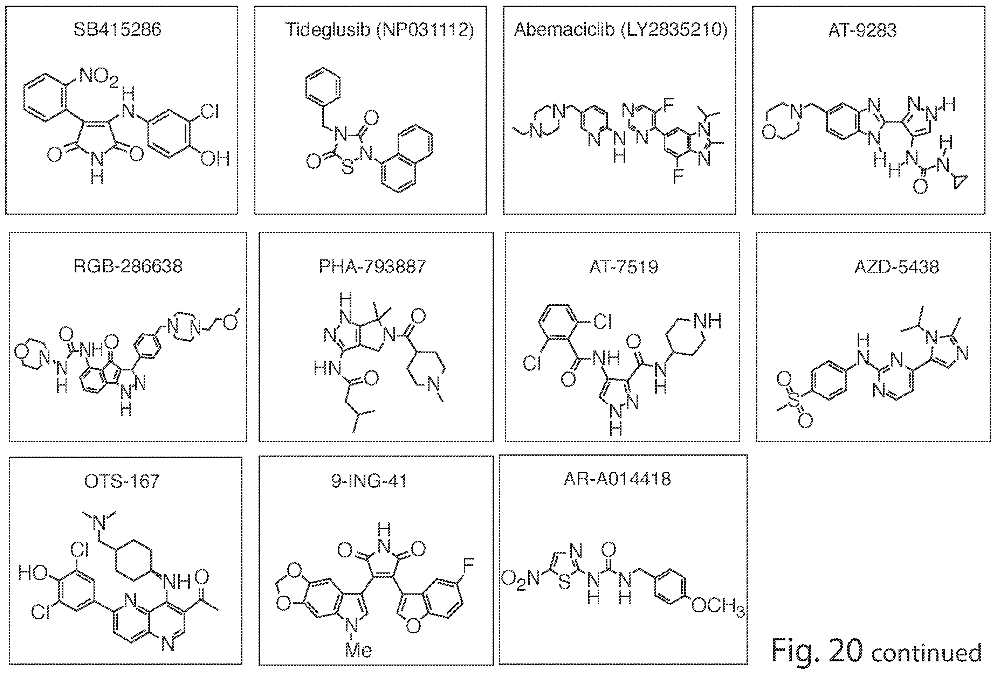

**FIG. 20** show chemical structures of exemplary small molecular inhibitors of GSK3α.

### DETAILED DESCRIPTION

### Methods of treating cancer

The invention described herein is related, in part, to the discovery that inhibition of GSK3α via the administration of a GSK3α inhibitor sensitized cancer cells, e.g., leukemia cells, to treatment with an asparaginase. Specifically, treatment with a GSK3α inhibitor sensitized asparaginase-resistant cells such that they were now susceptible to treatment with an asparaginase. Accordingly, the first aspect of the invention provides an asparaginase for use in the treatment of cancer in a subject, wherein: the asparaginase is used in combination with an agent that inhibits GSK3α, or the cancer comprises a mutation that results in inhibition of GSK3α..

Further embodiments of this invention are based in part on the finding that asparaginase had little efficacy against APC-mutant human colorectal cancers (CRCs) or mouse intestinal organoids, but was profoundly cytotoxic in the setting of R-spondin translocations, which stimulate Wnt ligand-induced inhibition of GSK3. In APC-mutant CRC, pharmacologic inhibition of GSK3α was sufficient for asparaginase sensitization. Thus, Wnt pathway activation provides an exploitable therapeutic vulnerability in CRC.

Most CRCs have mutations that activate Wnt/β-catenin signaling, but outcomes remain poor for patients with unresectable disease. It was specifically contemplated that the synthetic lethal interaction of Wnt-dependent stabilization of proteins and asparaginase could be exploited for CRC therapy. Data provided herein in Example 2 show that in human CRC cells and mouse intestinal organoids treatment with asparaginase was profoundly toxic to tumors with Wnt ligand-induced inhibition of GSK3, but not to CRCs with APC mutations predicted to activate β-catenin without inhibiting GSK3. APC-mutant CRCs were sensitized to asparaginase by treatment with recombinant Wnt/R-spondin ligands, or by selective inhibition of GSK3α.

In one embodiment, GSK3α is inhibited by at least 50% as compared to a substantially identical cell not having the mutation that results in the inhibition of GSK3α (e.g., a cancer cell not having the mutation, or a non-cancer, wild-type cell). In one embodiment, GSK3α is inhibited by at least 10%, at least 20%, at least 30%, at least 40%, at least 50%, at least 60%, at least 70%, at least 80%, at least 90%, at least 99% or more as compared to an identical cell not having the mutation that results in the inhibition of GSK3α. One skilled in the art can determine if a cancer cell has a mutation that inhibits GSK3α, e.g., via PCR-based assays or western-blotting to measure the mRNA and protein levels of GSK3α, respectively. Functional assays that assess GSK3α activity can be further used to determine if GSK3α is inhibited. For example, one skilled in the art can determine if the nuclear translocation of β-Catenin fails to occur, indicating that GSK3α is inhibited.

In various embodiments, the subject is diagnosed with having cancer or has received the results of an assay that diagnoses a subject of having cancer prior to administering the treatment (e.g., an asparaginase and optionally an agent that inhibits GSK3α). A skilled clinician can diagnose a subject as having cancer using various assays known in the art. Exemplary assays include: (1) a physical exam, in which a skilled clinician feels areas of the subject's body for lumps that may indicate a tumor, or for abnormalities, such as changes in skin color or enlargement of an organ, that may indicate the presence of cancer; (2) laboratory tests, such as urine and blood tests, to identify abnormalities caused by cancer. For example, in people with leukemia, a common blood test called complete blood count may reveal an unusual number or type of white blood cells; (3) Noninvasive imaging tests to examine your bones and internal organs for signs of cancer or tumors. Imaging tests used in diagnosing cancer may include a computerized tomography (CT) scan, bone scan, magnetic resonance imaging (MRI), positron emission tomography (PET) scan, ultrasound and X-ray, among others; and (4) biopsy, in which a skilled clinician collects a sample of cells from an area of the body that is suspected of having cancer for testing. Biopsies can be obtained using any method known in the art. Biopsy samples are examined to identify the presence of cancer cells using standards known in the art.

An assay for diagnosing cancer need not be performed by a clinician performing the methods of treating as described herein and not forming part of the invention. For example, a first clinician can perform the assay to diagnose and provide the results of the assay to a second clinician, who will perform the methods of treatment described herein.

In one embodiment, the subject has, prior to treatment, been identified as having cancer comprising a mutation that results in inhibition of GSK3α.In one embodiment, the mutation that inhibits GSK3α is a mutation in a gene selected from: R-spondin1 (RSPO1), R-spondin 2 (RSPO2), R-spondin 3 (RSPO3), R-spondin4 (RSPO4), ring finger protein 43 (RNF43). In one embodiment, the mutation is in the GSK3α gene. A mutation can be identified in a biological sample obtained from the subject, for example, via genomic sequencing of the biological sample and comparing the sequence to a wild-type sequence of the gene. Exemplary biological samples include a tissue sample or a blood sample. A biological sample can be obtained from a subject using standard techniques known in the art, e.g., a biological sample can be obtained from a biopsy, or a standard blood draw.

Table 1 shows the Gene ID number and aliases for the genes listed above. It is to be understood a gene listed in Table 1 (e.g., RSPO1) can refer to human form of that gene (e.g., human RSPO1), including naturally occurring variants, molecules, and alleles thereof. A gene listed in Table 1 can refers to the mammalian form of that gene, e.g., mouse, rat, rabbit, dog, cat, cow, horse, pig, and the like. The nucleic sequence of the genes listed in Table 1 can be readily identified by searching the NCBI Gene ID on the world wide web at www.ncbi.nlm.nih.gov/gene/.

| Table 1. Information for exemplary genes. | | |
|---|---|---|
| **Gene Name** | **NCBI Gene ID** | **Alternative Gene names** |
| R-spondin1 (RSPO1) | 284654 | RSPO; CRISTIN3 |
| R-spondin 2 (RSPO2) | 340419 | HHRRD; TETAMS2; CRISTIN2 |
| R-spondin 3 (RSPO3) | 84870 | CRISTIN1, PWTSR, THSD2 |
| R-spondin4 (RSPO4) | 343637 | C20orf182, CRISTIN4 |
| ring finger protein 43 (RNF43) | 54894 | RNF124, SSPCS, URCC |

In another embodiment, the mutation that inhibits GSK3a is a mutation that alters the expression of a gene selected from: R-spondin1 (RSPO1), R-spondin 2 (RSPO2), R-spondin 3 (RSPO3), R-spondin4 (RSPO4), ring finger protein 43 (RNF43), and glycogen synthase kinase 3 alpha (GSK3A, more commonly referred to as GSK3α). As used herein, "alter expression" refers to a change in the level (e.g., increased or decreased) or function (e.g., production of a gene product) of the gene that differs significantly from that expression of the gene in comparable tissue (e.g., cell type) from a sample obtained from a healthy individual. One skilled in the art can determine if gene expression is altered for a given gene using western blotting or PCR-based assays to assess gene product or mRNA levels for a given gene, respectively, or via functional assays to determine if, e.g., downstream functions of the gene occur normally, for example.

In one embodiment, the mutation results in the activation of the WNT pathway, e.g., the canonical WNT pathway. The canonical Wnt pathway causes an accumulation of β-catenin in the cytoplasm and its eventual translocation into the nucleus to act as a transcriptional coactivator of transcription factors that belong to the TCF/LEF family. Without Wnt, β-catenin would not accumulate in the cytoplasm since a destruction complex would normally degrade it. This destruction complex includes the following proteins: Axin, adenomatosis polyposis coli (APC), protein phosphatase 2A (PP2A), glycogen synthase kinase 3 (GSK3) and casein kinase 1α (CK1α). It degrades β-catenin by targeting it for ubiquitination, which subsequently sends it to the proteasome to be digested. However, as soon as Wnt binds Fz and LRP5/6, the destruction complex function becomes disrupted. This is due to Wnt causing the translocation of the negative Wnt regulator, Axin, and the destruction complex to the plasma membrane. Phosphorylation by other proteins in the destruction complex subsequently binds Axin to the cytoplasmic tail of LRP5/6. Axin becomes de-phosphorylated and its stability and levels decrease. Dsh then becomes activated via phosphorylation and its DIX and PDZ domains inhibit the GSK3 activity of the destruction complex. This allows β-catenin to accumulate and localize to the nucleus and subsequently induce a cellular response via gene transduction alongside the TCF/LEF (T-cell factor/lymphoid enhancing factor) transcription factors. It is to be understood that a mutation that results in activation of the Wnt pathway can be a mutation within any gene, and is not limited to a genes within the Wnt pathway. A mutation that activates the Wnt pathway can be, e.g., in a gene that regulates a Wnt pathway component, e.g., Axin, upstream of the Wnt pathway.

In one embodiment, WNT signaling is activated by at least 10%, at least 20%, at least 30%, at least 40%, at least 50%, at least 60%, at least 70%, at least 80%, at least 90%, at least 99% or more as compared to an appropriate control. As used herein, an "appropriate control" refers to the level and/or activity of WNT signaling in an otherwise identical population of cells that do not harbor any mutations known to activate WNT signaling. One skilled in the art can assess whether WNT signaling is activated by, e.g., identifying an increase in nuclear β-catenin in a cell via immunofluorescence, or western blotting of a nuclear fraction of the cancer cell to probe for nuclear levels of β-catenin. Nuclear β-catenin levels in cancer cells should be compared to levels of nuclear β-catenin in wild-type cells not having a WNT-activating mutation.

Disclosed herein is a method of treating cancer, the method comprising: (a) obtaining a biological sample from a subject having cancer; (b) assaying the sample and identifying the cancer as having a mutation that results in inhibition of GSK3α; and (c) administering an asparaginase to a subject who has been identified as having cancer having a mutation that results in inhibition of GSK3α.

Disclosed herein is a method of treating cancer, the method comprising: (a) receiving the results of an assay that identifies a subject as having a cancer having a mutation that results in inhibition of GSK3α; and (b) administering an asparaginase to a subject who has been identified as having cancer having a mutation that results in inhibition of GSK3α.

### Cancer

As used herein, "cancer" refers to a hyperproliferation of cells that have lost normal cellular control, resulting in unregulated growth, lack of differentiation, local tissue invasion, and metastasis. Cancers are classified based on the histological type (*e.g.,* the tissue in which they originate) and their primary site (*e.g.,* the location of the body the cancer first develops), and can be a carcinoma, a melanoma, a sarcoma, a myeloma, a leukemia, or a lymphoma. "Cancer" can also refer to a solid tumor. As used herein, the term "tumor" refers to an abnormal growth of cells or tissues, *e.g.,* of malignant type or benign type. "Cancer" can be metastatic, meaning the cancer cells have disseminated from its primary site of origin and migrated to a secondary site.

In various embodiments, the cancer treated herein is a carcinoma, a melanoma, a sarcoma, a myeloma, a leukemia, a lymphoma, or a solid tumor.

A carcinoma is a cancer that originates in an epithelial tissue. Carcinomas account for approximately 80-90% of all cancers. Carcinomas can affect organs or glands capable of secretion (e.g., breasts, lung, prostate, colon, or bladder). There are two subtypes of carcinomas: adenocarcinoma, which develops in an organ or gland, and squamous cell carcinoma, which originates in the squamous epithelium. Adenocarcinomas generally occur in mucus membranes, and are observed as a thickened plaque-like white mucosa. They often spread easily through the soft tissue where they occur. Exemplary adenocarcinomas include, but are not limited to, lung cancer, prostate cancer, pancreatic cancer, esophageal cancer, and colorectal cancer. Squamous cell carcinomas can originate from any region of the body. Examples of carcinomas include, but are not limited to, prostate cancer, colorectal cancer, microsatellite stable colon cancer, microsatellite instable colon cancer, hepatocellular carcinoma, breast cancer, lung cancer, small cell lung cancer, non-small cell lung cancer, lung adenocarcinoma, melanoma, basal cell carcinoma, squamous cell carcinoma, renal cell carcinoma, ductal carcinoma in situ, ductal carcinoma.

Sarcomas are cancers that originate in supportive and connective tissues, for example bones, tendons, cartilage, muscle, and fat. Sarcoma tumors usually resemble the tissue in which they grow. Non-limiting examples of sarcomas include, Osteosarcoma or osteogenic sarcoma (originating from bone), Chondrosarcoma (originating from cartilage), Leiomyosarcoma (originating from smooth muscle), Rhabdomyosarcoma (originating from skeletal muscle), Mesothelial sarcoma or mesothelioma (originate from membranous lining of body cavities), Fibrosarcoma (originating from fibrous tissue), Angiosarcoma or hemangioendothelioma (originating from blood vessels), Liposarcoma (originating from adipose tissue), Glioma or astrocytoma (originating from neurogenic connective tissue found in the brain), Myxosarcoma (originating from primitive embryonic connective tissue), or Mesenchymous or mixed mesodermal tumor (originating from mixed connective tissue types).

Melanoma is a type of cancer forming from pigment-containing melanocytes. Melanoma typically develops in the skin, but can occur in the mouth, intestine, or eye.

Myelomas are cancers that originate in plasma cells of bone marrow. Non-limiting examples of myelomas include multiple myeloma, plasmacytoma and amyloidosis.

Lymphomas develop in the glands or nodes of the lymphatic system (e.g., the spleen, tonsils, and thymus), which purifies bodily fluids and produces white blood cells, or lymphocytes. Unlike leukemia, lymphomas form solid tumors. Lymphoma can also occur in specific organs, for example the stomach, breast, or brain; this is referred to as extranodal lymphomas). Lymphomas are subclassified into two categories: Hodgkin lymphoma and Non-Hodgkin lymphoma. The presence of Reed-Sternberg cells in Hodgkin lymphoma diagnostically distinguishes Hodgkin lymphoma from Non-Hodgkin lymphoma. Non-limiting examples of lymphoma include Diffuse large B-cell lymphoma (DLBCL), Follicular lymphoma, Chronic lymphocytic leukemia (CLL), Small lymphocytic lymphoma (SLL), Mantle cell lymphoma (MCL), Marginal zone lymphomas, Burkitt lymphoma, hairy cell leukemia (HCL). In one embodiment, the cancer is DLBCL or Follicular lymphoma.

Leukemias (also known as "blood cancers") are cancers of the bone marrow, which is the site of blood cell production. Leukemia is often associated with the overproduction of immature white blood cells. Immature white blood cells do not function properly, rendering the patient prone to infection. Leukemia additionally affects red blood cells, and can cause poor blood clotting and fatigue due to anemia.

In one embodiment, the leukemia is acute myeloid leukemia (AML), Chronic myeloid leukemia (CML), Acute lymphocytic leukemia (ALL), and Chronic lymphocytic leukemia (CLL). Examples of leukemia include, but are not limited to, Myelogenous or granulocytic leukemia (malignancy of the myeloid and granulocytic white blood cell series), Lymphatic, lymphocytic, or lymphoblastic leukemia (malignancy of the lymphoid and lymphocytic blood cell series), and Polycythemia vera or erythremia (malignancy of various blood cell products, but with red cells predominating).

In one embodiment, the cancer is a solid tumor. Non-limiting examples of solid tumors include Adrenocortical Tumor, Alveolar Soft Part Sarcoma, Chondrosarcoma, Colorectal Carcinoma, Desmoid Tumors, Desmoplastic Small Round Cell Tumor, Endocrine Tumors, Endodermal Sinus Tumor, Epithelioid Hemangioendothelioma, Ewing Sarcoma, Germ Cell Tumors (Solid Tumor), Giant Cell Tumor of Bone and Soft Tissue, Hepatoblastoma, Hepatocellular Carcinoma, Melanoma, Nephroma, Neuroblastoma, Non-Rhabdomyosarcoma Soft Tissue Sarcoma (NRSTS), Osteosarcoma, Paraspinal Sarcoma, Renal Cell Carcinoma, Retinoblastoma, Rhabdomyosarcoma, Synovial Sarcoma, and Wilms Tumor. Solid tumors can be found in bones, muscles, or organs, and can be sarcomas or carinomas.

In one embodiment, the cancer is resistant to a cancer therapy. In one embodiment, the cancer is resistant to an asparaginase. A cancer resistant to a therapy, for example, asparaginase, is one that previously responded to the treatment but is now capable of growing or persisting despite the presence of continued treatment. Resistance to a therapy can occur due to, e.g., acquired mutations in the cancer cell, gene amplification in the cancer cell, or the cancer cell develops mechanisms to prevent the uptake of the treatment. In one embodiment, the cancer is not resistant to a cancer therapy or asparaginase.

In one embodiment, the cancer is metastatic (e.g., the cancer has disseminated from its primary location to at least one secondary location).

In one embodiment, the cancer has relapsed following administration of a cancer therapy. A "relapsed cancer" is defined as the return of a disease or the signs and symptoms of a disease after a period of improvement.

### Asparaginase and Agents that inhibit GSK3α

Asparaginase, an antileukemic enzyme that degrades the nonessential amino acid asparagine is a chemotherapy drug used to treat acute lymphoblastic leukemia (ALL). It can also be used to treat some other blood disorders. Asparaginase is also known in the art as, e.g., Erwinase, Crisantaspase or L-asparaginase. Asparaginase catalyzes the conversion of L-asparagine to aspartic acid and ammonia, thus depriving the leukemic cell of circulating asparagine, which leads to cell death.

In one embodiment, the asparaginase is L-asparaginase (Elspar), pegaspargase (PEG-asparaginase; Oncaspar), SC-PEG asparaginase (Calaspargase pegol), Erwinia asparaginase (Erwinaze, Recombinant Crisantaspase, or Recombinant Crisantaspase with half-life extension or pegylation).

L-asparaginase (Elspar), pegaspargase (PEG-asparaginase; Oncaspar), and SC-PEG asparaginase (Calaspargase pegol) are all based on the Escherichia coli asparaginase gene ansB, either in its native form or conjugated to polyethylene glycol (pegylated), which encodes a gene product having a sequence of SEQ ID NO: 23.

MEFFKKTALAALVMGFSGAALALPNITILATGGTIAGGGDSATKSNYTVGKVGVEN
LVNAVPQLKDIANVKGEQVVNIGSQDMNDNVWLTLAKKINTDCDKTDGFVITHGT
DTMEETAYFLDLTVKCDKPVVMVGAMRPSTSMSADGPFNLYNAVVTAADKASAN
RGVLVVMNDTVLDGRDVTKTNTTDVATFKSVNYGPLGYIHNGKIDYQRTPARKHT
SDTPFDVSKLNELPKVGIVYNYANASDLPAKALVDAGYDGIVSAGVGNGNLYKSVF
DTLATAAKTGTAVVRSSRVPTGATTQDAEVDDAKYGFVASGTLNPQKARVLLQLA
LTQTKDPQQIQQIFNQY (SEQ ID NO: 23)

The Erwinia asparaginases (Erwinaze, Recombinant Crisantaspase, or Recombinant Crisantaspase with half-life extension) are based on the ansB gene from Erwinia chrysanthemi (also known as Dickeya chrysanthemi), either in its native form or conjugated to polyethylene glycol (pegylated), which encodes a gene product having a sequence of SEQ ID NO: 24.

MERWFKSLFVLVLFFVFTASAADKLPNIVILATGGTIAGSAATGTQTTGYKAGALGV
DTLINAVPEVKKLANVKGEQFSNMASENMTGDVVLKLSQRVNELLARDDVDGVVI
THGTDTVEESAYFLHLTVKSDKPVVFVAAMRPATAISADGPMNLLEAVRVAGDKQS
RGRGVMVVLNDRIGSARYITKTNASTLDTFKANEEGYLGVIIGNRIYYQNRIDKLHT
TRSVFDVRGLTSLPKVDILYGYQDDPEYLYDAAIQHGVKGIVYAGMGAGSVSVRGI
AGMRKAMEKGVVVIRSTRTGNGIVPPDEELPGLVSDSLNPAHARILLMLALTRTSDP
KVIQEYFHTY (SEQ ID NO: 24)

In one embodiment, the asparaginase encodes a gene product having a sequence that comprises a sequence with at least 80%, at least 85%, at least 90%, at least 95%, at least 96%, at least 97%, at least 98%, at least 99% or greater sequence identity to the sequence of SEQ ID NO: 23 or 24. In one embodiment, the asparaginase encodes a gene product having a sequence that comprises the entire sequence of SEQ ID NO: 23 or 24. In another embodiment, the asparaginase encodes a gene product having a sequence of SEQ ID NO: 23 or 24, wherein the fragment retains the desired function of asparaginase, e.g., the antileukemic enzymatic activity.

Methods for purifying and delivering asparaginases, and compositions comprising asparaginases are further described in, e.g., U.S. Patent No. 3440142; 3511754; 3511755; 3597323; 3652402; 3620925; 3686072; 3773624; 4617271; 6368845; 7666652; 9181552; 9920311; 10273444; U.S. Patent Publication No. 2002/0102251; 2003/0186380; 2010/00183765; 2012/0100249; 2013/0023029; and international Application No. WO1999/039732.

An agent that inhibits GSK3α may be administered in combination with an asparaginase to a subject having cancer, e.g., leukemia. In one embodiment, the agent that inhibits GSK3α is a small molecule, an antibody or antibody fragment, a peptide, an antisense oligonucleotide, a genome editing system, or an RNAi.

An agent described herein targets GSK3α for its inhibition. An agent is considered effective for inhibiting GSK3α if, for example, upon administration, it inhibits the presence, amount, activity and/or level of GSK3α in the cell. In one embodiment, an agent that inhibits the level and/or activity of GSK3α by at least 10%, by at least 20%, by at least 30%, by at least 40%, by at least 50%, by at least 60%, by at least 70%, by at least 80%, by at least 90%, by at least 100% or more as compared to an appropriate control. As used herein, an "appropriate control" refers to the level and/or activity of GSK3α prior to administration of the agent, or the level and/or activity of GSK3α in a population of cells that was not in contact with the agent. Inhibition of GSK3α will prevent activation of the WNT signaling pathway. One skilled in the art can determine if the presence, amount or level of GSK3α has been reduced using PCR-based assays or western blotting to assess GSK3α mRNA or protein levels, respectively, or by visualizing the GSK3α protein via immunofluorescence of an anti-GSK3α antibody. One skilled in the art can determine if the activity of GSK3α has been reduced using functional assays that assess downstream effects of GSK3α, such as, determining if its downstream substrate, β-catenin, is phosphorylated via western blotting or immunofluorescence with an anti-phospho β-catenin specific antibody.

An agent can inhibit e.g., the transcription, or the translation of GSK3α in the cell. An agent can inhibit the activity or alter the activity (e.g., such that the activity no longer occurs, or occurs at a reduced rate) of GSK3α in the cell (e.g., GSK3α's expression).

In one embodiment, an agent that inhibits GSK3α promotes programmed cell death, e.g., kills the cell. To determine if an agent is effective at inhibiting GSK3α, mRNA and protein levels of a given target (e.g., GSK3α) can be assessed using RT-PCR and western-blotting, respectively. Biological assays that detect the activity of GSK3α (e.g., WNT activation) can be used to assess if programmed cell death has occurred.

The agent may function directly in the form in which it is administered. Alternatively, the agent can be modified or utilized intracellularly to produce something which inhibits GSK3α, such as introduction of a nucleic acid sequence into the cell and its transcription resulting in the production of the nucleic acid and/or protein inhibitor of GSK3α.In some embodiments, the agent is any chemical, entity or moiety, including without limitation synthetic and naturally-occurring non-proteinaceous entities. In certain embodiments the agent is a small molecule having a chemical moiety. For example, chemical moieties included unsubstituted or substituted alkyl, aromatic, or heterocyclyl moieties including macrolides, leptomycins and related natural products or analogues thereof. Agents can be known to have a desired activity and/or property, or can be identified from a library of diverse compounds.

In various embodiments, the agent is a small molecule that inhibits GSK3α. Exemplary small molecular inhibitors of GSK3α include BRD0705, BRD4963, BRD1652, BRD3731, CHIR-98014, LY2090314, AZD1080, CHIR-99021 (CT99021) HCl, CHIR-99021 (CT99021), BIO-acetoxime, SB216763, SB415286, Abemaciclib (LY2835210), AT-9283, RGB-286638, PHA-793887, AT-7519, AZD-5438, OTS-167, 9-ING-41, Tideglusib (NP031112), and AR-A014418. Chemical structures for exemplary small molecular inhibitors of GSK3α are shown in Table 2.

| Table 2. Chemical structures for exemplary small molecular inhibitors of GSK3α. | |
|---|---|
| BRD0705 | 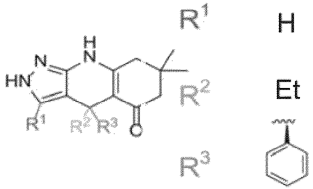 |
| BRD4963 | 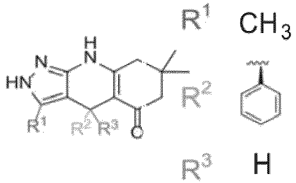 |
| BRD1652 | 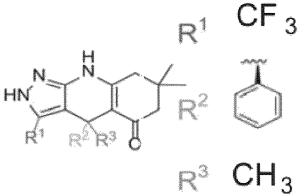 |
| BRD3731 | 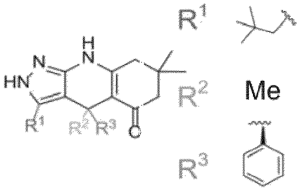 |
| CHIR-98014 | 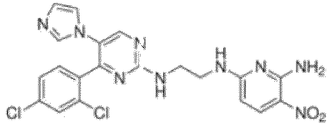 |
| LY2090314 | 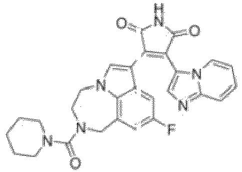 |
| AZD1080 | 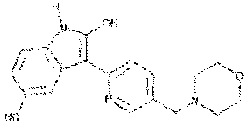 |
| CHIR-99021 (CT99021) | 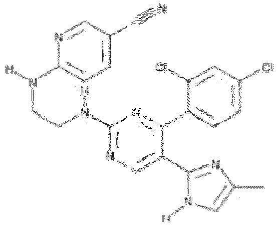 |
| BIO-acetoxime | 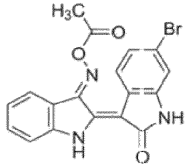 |
| SB216763 | 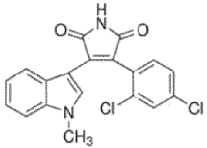 |
| SB415286 | 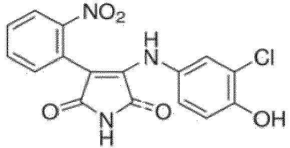 |
| Abemaciclib (LY2835210) | 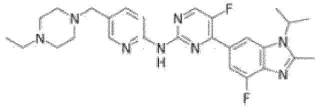 |
| AT-9283 | 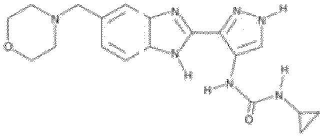 |
| RGB-286638 | 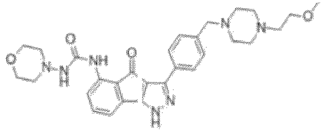 |
| PHA-793887 | 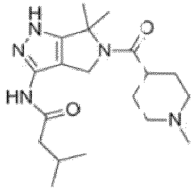 |
| AT-7519 | 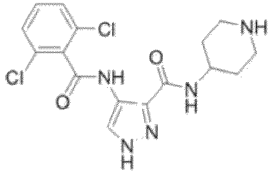 |
| AZD-5438 | 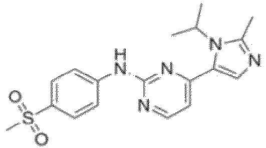 |
| OTS-167 | 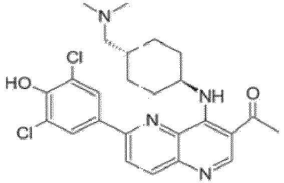 |
| 9-ING-41 | 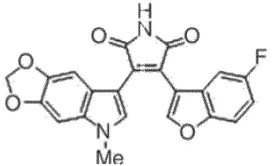 |
| Tideglusib (NP031112) | 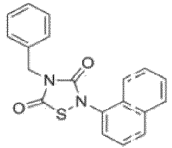 |
| AR-A014418 | 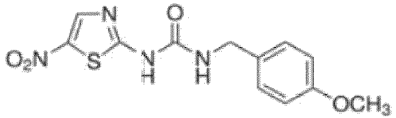 |

Further, in one embodiment, the small molecule is a derivative, a variant, or an analog of, or is substantially similar to any of the small molecules described herein, for example as listed in Table 2. A molecule is said to be a "derivative" of another molecule when it contains additional chemical moieties not normally a part of the molecule and/or when it has been chemically modified. Such moieties can improve the molecule's expression levels, enzymatic activity, solubility, absorption, biological half-life, etc. The moieties can alternatively decrease the toxicity of the molecule, eliminate or attenuate any undesirable side effect of the molecule, etc. Moieties capable of mediating such effects are disclosed in Remington's Pharmaceutical Sciences, 18th edition, A. R. Gennaro, Ed., MackPubl., Easton, PA (1990). A "variant" of a molecule is meant to refer to a molecule substantially similar in structure and function to either the entire molecule, or to a fragment thereof. A molecule is said to be "substantially similar" to another molecule if both molecules have substantially similar structures and/or if both molecules possess a similar biological activity. Thus, provided that two molecules possess a similar activity, they are considered variants as that term is used herein even if the structure of one of the molecules not found in the other, or if the structure is not identical. An "analog" of a molecule is meant to refer to a molecule substantially similar in function to either the entire molecule or to a fragment thereof.

In one embodiment, the small molecule inhibitor of GSK3α, e.g., a small molecule listed in Table 2, is conjugated to an E3 ubiquitin ligase recruitment element. As used herein, "conjugated" refers to two or more smaller entities (e.g., a small molecule and a E3 ubiquitin ligase recruitment element) that are linked, connected, associated, bonded (covalently or non-covalently), or any combination thereof, to form a larger entity. The conjugated E3 ubiquitin ligase recruitment element recruits an E3, which mediates the transfer of an ubiquitin from an E2 to the protein substrate. Binding of an ubiquitin to a protein substrate marks the protein for degradation via the ubiquitin proteasome system. Thus, a small molecule inhibitor of GSK3α conjugated to an E3 ubiquitin ligase recruitment element would, e.g., bind to GSK3α and subsequently promote its degradation. E3 ubiquitin ligase recruitment elements can include, but are not limited to, thalidomide, lenalidomide, pomalidomide, or a VHL ligand that mimics the hydroxyproline degradation motif of HIF1-alpha. Chemical structures for exemplary E3 ubiquitin ligase recruitment element are presented herein in Table 3, and are further described in, e.g., Pavia, SL, and Crews, CM. Current Opinion in Chemical Biology. 2019. 50; 111-119. Use of conjugated E3 ubiquitin ligase recruitment elements are further described in U.S. Patent Nos 7,208,157B2 and 9,770,512.

In one embodiment, a small molecule conjugated to an E3 ubiquitin ligase recruitment element further comprises a linker. It is specifically contemplated herein that the specifications of the linker (e.g., length, sequence, etc.) would be optimized for greatest efficacy of the small molecule and E3 ubiquitin ligase recruitment element. For example, a linker would be designed such that it does not interfere with binding of the small molecule to its target (e.g., the binding pocket on the protein of interest) or the transfer of the ubiquitin from the E2 to the protein substrate.

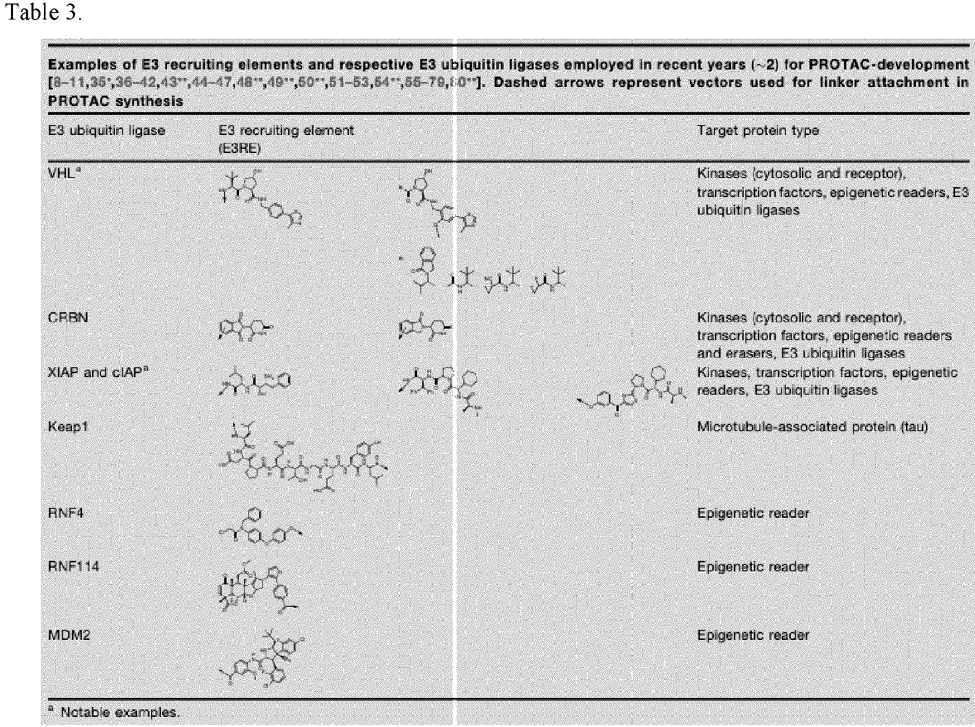

In various embodiments, the agent that inhibits GSK3α is an antibody or antigen-binding fragment thereof, or an antibody reagent that is specific for GSK3α.As used herein, the term "antibody reagent" refers to a polypeptide that includes at least one immunoglobulin variable domain or immunoglobulin variable domain sequence and which specifically binds a given antigen. An antibody reagent can comprise an antibody or a polypeptide comprising an antigen-binding domain of an antibody. In some embodiments, an antibody reagent can comprise a monoclonal antibody or a polypeptide comprising an antigen-binding domain of a monoclonal antibody. For example, an antibody can include a heavy (H) chain variable region (abbreviated herein as VH), and a light (L) chain variable region (abbreviated herein as VL). In another example, an antibody includes two heavy (H) chain variable regions and two light (L) chain variable regions. The term "antibody reagent" encompasses antigen-binding fragments of antibodies (e.g., single chain antibodies, Fab and sFab fragments, F(ab')2, Fd fragments, Fv fragments, scFv, CDRs, and domain antibody (dAb) fragments (see, e.g. de Wildt et al., Eur J. Immunol. 1996; 26(3):629-39)) as well as complete antibodies. An antibody can have the structural features of IgA, IgG, IgE, IgD, or IgM (as well as subtypes and combinations thereof). Antibodies can be from any source, including mouse, rabbit, pig, rat, and primate (human and non-human primate) and primatized antibodies. Antibodies also include midibodies, nanobodies, humanized antibodies, chimeric antibodies, and the like.

In one embodiment, the antibody or antibody reagent binds to an amino acid sequence that corresponds to the amino acid sequence encoding GSK3α (SEQ ID NO: 2).

MSGGGPSGGGPGGSGRARTSSFAEPGGGGGGGGGPGGSASGPG
GTGGGKASVGAMGGGVGASSSGGGPGGSGGGGSGGPGAGTSFPPPGVKLGRDSGKVTT
VVATLGQGPERSQEVAYTDIKVIGNGSFGVVYQARLAETRELVAIKKVLQDKRFKNRE
LQIMRKLDHCNIVRLRYFFYSSGEKKDELYLNLVLEYVPETVYRVARHFTKAKLTIPI
LYVKVYMYQLFRSLAYIHSQGVCHRDIKPQNLLVDPDTAVLKLCDFGSAKQLVRGEPN
VSYICSRYYRAPELIFGATDYTSSIDVWSAGCVLAELLLGQPIFPGDSGVDQLVEIIK
VLGTPTREQIREMNPNYTEFKFPQIKAHPWTKVFKSRTPPEAIALCSSLLEYTPSSRL
SPLEACAHSFFDELRCLGTQLPNNRPLPPLFNFSAGELSIQPSLNAILIPPHLRSPAG
TTTLTPSSQALTETPTSSDWQSTDATPTLTNSS (SEQ ID NO: 2)

In another embodiment, the anti- GSK3α antibody or antibody reagent binds to an amino acid sequence that comprises the sequence of SEQ ID NO: 2; or binds to an amino acid sequence that comprises a sequence with at least 80%, at least 85%, at least 90%, at least 95%, at least 96%, at least 97%, at least 98%, at least 99% or greater sequence identity to the sequence of SEQ ID NO: 2. In one embodiment, the anti- GSK3α antibody or antibody reagent binds to an amino acid sequence that comprises the entire sequence of SEQ ID NO: 2. In another embodiment, the antibody or antibody reagent binds to an amino acid sequence that comprises a fragment of the sequence of SEQ ID NO: 2, wherein the fragment is sufficient to bind its target, e.g., GSK3α, and inhibit GSK3α activity and/or expression.

In one embodiment, an anti-GSK3α antibody or antibody reagent is conjugated to an E3 ubiquitin ligase recruitment element. In one embodiment, the anti-GSK3α antibody or antibody reagent conjugated to an E3 ubiquitin ligase recruitment element further comprises a linker.

In one embodiment, the agent that inhibits GSK3α is an antisense oligonucleotide. As used herein, an "antisense oligonucleotide" refers to a synthesized nucleic acid sequence that is complementary to a DNA or mRNA sequence, such as that of a microRNA. Antisense oligonucleotides are typically designed to block expression of a DNA or RNA target by binding to the target and halting expression at the level of transcription, translation, or splicing. Antisense oligonucleotides of the present invention are complementary nucleic acid sequences designed to hybridize under cellular conditions to a gene, e.g., GSK3α. Thus, oligonucleotides are chosen that are sufficiently complementary to the target, i.e., that hybridize sufficiently well and with sufficient specificity in the context of the cellular environment, to give the desired effect. For example, an antisense oligonucleotide that inhibits GSK3α may comprise at least 5, at least 10, at least 15, at least 20, at least 25, at least 30, or more bases complementary to a portion of the coding sequence of the human GSK3α gene (e.g., SEQ ID NO: 1), respectively.

In one embodiment, GSK3α is depleted from the cell's genome using any genome editing system including, but not limited to, zinc finger nucleases, TALENS, meganucleases, and CRISPR/Cas systems. In one embodiment, the genomic editing system used to incorporate the nucleic acid encoding one or more guide RNAs into the cell's genome is not a CRISPR/Cas system; this can prevent undesirable cell death in cells that retain a small amount of Cas enzyme/protein. It is also contemplated herein that either the Cas enzyme or the sgRNAs are each expressed under the control of a different inducible promoter, thereby allowing temporal expression of each to prevent such interference.

When a nucleic acid encoding one or more sgRNAs and a nucleic acid encoding an RNA-guided endonuclease each need to be administered *in vivo,* the use of an adenovirus associated vector (AAV) is specifically contemplated. Other vectors for simultaneously delivering nucleic acids to both components of the genome editing/fragmentation system (e.g., sgRNAs, RNA-guided endonuclease) include lentiviral vectors, such as Epstein Barr, Human immunodeficiency virus (HIV), and hepatitis B virus (HBV). Each of the components of the RNA-guided genome editing system (e.g., sgRNA and endonuclease) can be delivered in a separate vector as known in the art or as described herein.

In one embodiment, the agent inhibits GSK3α by RNA inhibition. Inhibitors of the expression of a given gene can be an inhibitory nucleic acid. In some embodiments, the inhibitory nucleic acid is an inhibitory RNA (iRNA). The RNAi can be single stranded or double stranded.

The iRNA can be siRNA, shRNA, endogenous microRNA (miRNA), or artificial miRNA. In one embodiment, an iRNA as described herein effects inhibition of the expression and/or activity of a target, e.g. GSK3α. In some embodiments, the agent is siRNA that inhibits GSK3α. In some embodiments, the agent is shRNA that inhibits GSK3α.

One skilled in the art would be able to design siRNA, shRNA, or miRNA to target GSK3α, e.g., using publicly available design tools. siRNA, shRNA, or miRNA is commonly made using companies such as Dharmacon (Layfayette, CO) or Sigma Aldrich (St. Louis, MO).

In some embodiments, the iRNA can be a dsRNA. A dsRNA includes two RNA strands that are sufficiently complementary to hybridize to form a duplex structure under conditions in which the dsRNA will be used. One strand of a dsRNA (the antisense strand) includes a region of complementarity that is substantially complementary, and generally fully complementary, to a target sequence. The target sequence can be derived from the sequence of an mRNA formed during the expression of the target. The other strand (the sense strand) includes a region that is complementary to the antisense strand, such that the two strands hybridize and form a duplex structure when combined under suitable conditions

The RNA of an iRNA can be chemically modified to enhance stability or other beneficial characteristics. The nucleic acids featured in the invention may be synthesized and/or modified by methods well established in the art, such as those described in "Current protocols in nucleic acid chemistry," Beaucage, S.L. et al. (Edrs.), John Wiley & Sons, Inc., New York, NY, USA.

In one embodiment, the agent is miRNA that inhibits GSK3α.microRNAs are small non-coding RNAs with an average length of 22 nucleotides. These molecules act by binding to complementary sequences within mRNA molecules, usually in the 3' untranslated (3'UTR) region, thereby promoting target mRNA degradation or inhibited mRNA translation. The interaction between microRNA and mRNAs is mediated by what is known as the "seed sequence", a 6-8-nucleotide region of the microRNA that directs sequence-specific binding to the mRNA through imperfect Watson-Crick base pairing. More than 900 microRNAs are known to be expressed in mammals. Many of these can be grouped into families on the basis of their seed sequence, thereby identifying a "cluster" of similar microRNAs. A miRNA can be expressed in a cell, e.g., as naked DNA. A miRNA can be encoded by a nucleic acid that is expressed in the cell, e.g., as naked DNA or can be encoded by a nucleic acid that is contained within a vector.

The agent may result in gene silencing of the target gene (e.g., GSK3α), such as with an RNAi molecule (e.g. siRNA or miRNA). This entails a decrease in the mRNA level in a cell for a target by at least about 5%, about 10%, about 20%, about 30%, about 40%, about 50%, about 60%, about 70%, about 80%, about 90%, about 95%, about 99%, about 100% of the mRNA level found in the cell without the presence of the agent. In one preferred embodiment, the mRNA levels are decreased by at least about 70%, about 80%, about 90%, about 95%, about 99%, about 100%. One skilled in the art will be able to readily assess whether the siRNA, shRNA, or miRNA effective target e.g., GSK3α, for its downregulation, for example by transfecting the siRNA, shRNA, or miRNA into cells and detecting the levels of a gene (e.g., GSK3α) found within the cell via western-blotting.

The agent may be contained in and thus further include a vector. Many such vectors useful for transferring exogenous genes into target mammalian cells are available. The vectors may be episomal, e.g. plasmids, virus-derived vectors such cytomegalovirus, adenovirus, etc., or may be integrated into the target cell genome, through homologous recombination or random integration, e.g. retrovirus-derived vectors such as MMLV, HIV-1, ALV, etc. In some embodiments, combinations of retroviruses and an appropriate packaging cell line may also find use, where the capsid proteins will be functional for infecting the target cells. Usually, the cells and virus will be incubated for at least about 24 hours in the culture medium. The cells are then allowed to grow in the culture medium for short intervals in some applications, e.g. 24-73 hours, or for at least two weeks, and may be allowed to grow for five weeks or more, before analysis. Commonly used retroviral vectors are "defective", i.e. unable to produce viral proteins required for productive infection. Replication of the vector requires growth in the packaging cell line.

The term "vector", as used herein, refers to a nucleic acid construct designed for delivery to a host cell or for transfer between different host cells. As used herein, a vector can be viral or non-viral. The term "vector" encompasses any genetic element that is capable of replication when associated with the proper control elements and that can transfer gene sequences to cells. A vector can include, but is not limited to, a cloning vector, an expression vector, a plasmid, phage, transposon, cosmid, artificial chromosome, virus, virion, etc.

As used herein, the term "expression vector" refers to a vector that directs expression of an RNA or polypeptide (e.g., an GSK3α inhibitor) from nucleic acid sequences contained therein linked to transcriptional regulatory sequences on the vector. The sequences expressed will often, but not necessarily, be heterologous to the cell. An expression vector may comprise additional elements, for example, the expression vector may have two replication systems, thus allowing it to be maintained in two organisms, for example in human cells for expression and in a prokaryotic host for cloning and amplification. The term "expression" refers to the cellular processes involved in producing RNA and proteins and as appropriate, secreting proteins, including where applicable, but not limited to, for example, transcription, transcript processing, translation and protein folding, modification and processing. "Expression products" include RNA transcribed from a gene, and polypeptides obtained by translation of mRNA transcribed from a gene. The term "gene" means the nucleic acid sequence which is transcribed (DNA) to RNA in vitro or in vivo when operably linked to appropriate regulatory sequences. The gene may or may not include regions preceding and following the coding region, e.g. 5' untranslated (5'UTR) or "leader" sequences and 3' UTR or "trailer" sequences, as well as intervening sequences (introns) between individual coding segments (exons).

Integrating vectors have their delivered RNA/DNA permanently incorporated into the host cell chromosomes. Non-integrating vectors remain episomal which means the nucleic acid contained therein is never integrated into the host cell chromosomes. Examples of integrating vectors include retroviral vectors, lentiviral vectors, hybrid adenoviral vectors, and herpes simplex viral vector.

One example of a non-integrative vector is a non-integrative viral vector. Non-integrative viral vectors eliminate the risks posed by integrative retroviruses, as they do not incorporate their genome into the host DNA. One example is the Epstein Barr oriP/Nuclear Antigen-1 ("EBNA1") vector, which is capable of limited self-replication and known to function in mammalian cells. As containing two elements from Epstein-Barr virus, oriP and EBNA1, binding of the EBNA1 protein to the virus replicon region oriP maintains a relatively long-term episomal presence of plasmids in mammalian cells. This particular feature of the oriP/EBNA1 vector makes it ideal for generation of integration-free iPSCs. Another non-integrative viral vector is adenoviral vector and the adeno-associated viral (AAV) vector.

Another non-integrative viral vector is RNA Sendai viral vector, which can produce protein without entering the nucleus of an infected cell. The F-deficient Sendai virus vector remains in the cytoplasm of infected cells for a few passages, but is diluted out quickly and completely lost after several passages (e.g., 10 passages).

Another example of a non-integrative vector is a minicircle vector. Minicircle vectors are circularized vectors in which the plasmid backbone has been released leaving only the eukaryotic promoter and cDNA(s) that are to be expressed.

As used herein, the term "viral vector" refers to a nucleic acid vector construct that includes at least one element of viral origin and has the capacity to be packaged into a viral vector particle. The viral vector can contain a nucleic acid encoding a polypeptide as described herein in place of non-essential viral genes. The vector and/or particle may be utilized for the purpose of transferring nucleic acids into cells either *in vitro* or *in vivo.* Numerous forms of viral vectors are known in the art.

### Administration

In some embodiments, the asparaginase for use as described herein relates to treating a subject having or diagnosed as having cancer (e.g., leukemia, colon cancer, or pancreatic cancer) comprising administering an agent that inhibits GSK3α in combination with said asparaginase as described herein. The asparaginase for use in the treatment of cancer may relate to treating a subject having or diagnosed as having cancer comprising a mutation that results in inhibition of GSK3α the treatment comprising administering said asparaginase as described herein. Subjects having cancer can be identified by a physician using current methods of diagnosing a condition. Symptoms and/or complications of cancer, which characterize this disease and aid in diagnosis are well known in the art. Tests that may aid in a diagnosis of, e.g., cancer, include blood tests and non-invasive imaging. A family history of a particular cancer will also aid in determining if a subject is likely to have the condition or in making a diagnosis of cancer.

The agents described herein (e.g., an agent that inhibits GSK3α) and an asparaginase can be administered in combination to a subject having or diagnosed as having cancer (e.g., leukemia, colon cancer, or pancreatic cancer). Administration of an agent or asparaginase described herein can be performed in a variety of manners, for example, in a single dose, in reoccurring multiple doses, via continuous infusion, via pulsed administration. In one embodiment, an agent or asparaginase described herein can be administered to a subject at least once every 1, 2, 3, 4, 5, 6, 7, 8, 9, 10, 11, 12, 13, 14, 15, 16, 17, 18, 19, 20, 21, 22, 23, or 24 hours; or every 1, 2, 3, 4, 5, 6, or 7 days; or every 1, 2, 3, or 4 weeks; or every 1, 2, 3, 4, 5, 6, 7, 8, 9, 10, 11, or 12 months, or more. It is specifically contemplated herein that the dosing of an agent or asparaginase described herein is determined based on the half-life of the agent, e.g., such that the effect of the agent (for example, inhibition of GSKα) is continuous, or nearly continuous, in the subject. For example, if the half-life of a given GSKα inhibitor is 12 hours, it would be administered every 12 hours to the subject such that it maintains continuous inhibition of GSKα in the subject.

In one embodiment, the agent that inhibits GSK3α and the asparaginase are administered in the same manner, e.g., the agent that inhibits GSK3α and the asparaginase are administered in a single dose, in multiple doses, via continuous infusion, via pulsed administration. In one embodiment, the agent that inhibits GSK3α and the asparaginase are administered in different manners, e.g., the agent that inhibits GSK3α is administered via continuous infusion, and the asparaginase is administered in a single dose.

In one embodiment the agent that inhibits GSK3α and the asparaginase are formulated together in one composition for administration. In one embodiment the agent that inhibits GSK3α and the asparaginase are formulated in separate compositions and are administered as separate compositions.

In some embodiments, the asparaginase for use described herein comprise administering an effective amount of the agents to a subject in order to alleviate at least one symptom of a given cancer. As used herein, "alleviating at least one symptom of a given cancer" is ameliorating any condition or symptom associated with cancer. As compared with an equivalent untreated control, such reduction is by at least 5%, 10%, 20%, 40%, 50%, 60%, 80%, 90%, 95%, 99% or more as measured by any standard technique. A variety of means for administering the agents and/or an asparaginase described herein to subjects are known to those of skill in the art. In one embodiment, the agent is administered systemically or locally (e.g., to the affected organ, e.g., the colon). In one embodiment, the agent is administered intravenously. In one embodiment, the agent is administered continuously, in intervals, or sporadically. The route of administration of the agent will be optimized for the type of agent being delivered (e.g., an antibody, a small molecule, an RNAi), and can be determined by a skilled practitioner.

The term "effective amount" as used herein refers to the amount of an agent (e.g., an agent that inhibits GSK3α) and/or an asparaginase that can be administered to a subject having or diagnosed as having cancer (e.g., leukemia, colon cancer, or pancreatic cancer) needed to alleviate at least one or more symptom of cancer. The term "therapeutically effective amount" therefore refers to an amount of an agent and/or an asparaginase that is sufficient to provide a particular anti-cancer effect when administered to a typical subject. An effective amount as used herein, in various contexts, would also include an amount of an agent and/or an asparaginase sufficient to delay the development of a symptom of cancer, alter the course of a symptom of cancer (e.g., slowing the progression of cancer), or reverse a symptom of cancer. Thus, it is not generally practicable to specify an exact "effective amount". However, for any given case, an appropriate "effective amount" can be determined by one of ordinary skill in the art using only routine experimentation.

In one embodiment, the agent and/or an asparaginase is administered continuously (e.g., at constant levels over a period of time). Continuous administration of an agent can be achieved, e.g., by epidermal patches, continuous release formulations, or on-body injectors.

Effective amounts, toxicity, and therapeutic efficacy can be evaluated by standard pharmaceutical procedures in cell cultures or experimental animals. The dosage can vary depending upon the dosage form employed and the route of administration utilized. The dose ratio between toxic and therapeutic effects is the therapeutic index and can be expressed as the ratio LD50/ED50. Compositions and methods that exhibit large therapeutic indices are preferred. A therapeutically effective dose can be estimated initially from cell culture assays. Also, a dose can be formulated in animal models to achieve a circulating plasma concentration range that includes the IC50 (*i.e.,* the concentration of the agent, which achieves a half-maximal inhibition of symptoms) as determined in cell culture, or in an appropriate animal model. Levels in plasma can be measured, for example, by high performance liquid chromatography. The effects of any particular dosage can be monitored by a suitable bioassay, e.g., measuring neurological function, or blood work, among others. The dosage can be determined by a physician and adjusted, as necessary, to suit observed effects of the treatment.

### Dosage

"Unit dosage form" as the term is used herein refers to a dosage for suitable one administration. By way of example a unit dosage form can be an amount of therapeutic disposed in a delivery device, e.g., a syringe or intravenous drip bag. In one embodiment, a unit dosage form is administered in a single administration. In another, embodiment more than one unit dosage form can be administered simultaneously.

Typically, the dosage ranges are between 0.001mg/kg body weight to 5 g/kg body weight, inclusive. In some embodiments, the dosage range is from 0.001 mg/kg body weight to 1g/kg body weight, from 0.001 mg/kg body weight to 0.5 g/kg body weight, from 0.001 mg/kg body weight to 0.1 g/kg body weight, from 0.001 mg/kg body weight to 50 mg/kg body weight, from 0.001 mg/kg body weight to 25 mg/kg body weight, from 0.001 mg/kg body weight to 10 mg/kg body weight, from 0.001 mg/kg body weight to 5 mg/kg body weight, from 0.001 mg/kg body weight to 1 mg/kg body weight, from 0.001 mg/kg body weight to 0.1 mg/kg body weight, from 0.001 mg/kg body weight to 0.005 mg/kg body weight. Alternatively, in some embodiments the dosage range is from 0.1 g/kg body weight to 5 g/kg body weight, from 0.5 g/kg body weight to 5 g/kg body weight, from 1 g/kg body weight to 5 g/kg body weight, from 1.5 g/kg body weight to 5 g/kg body weight, from 2 g/kg body weight to 5 g/kg body weight, from 2.5 g/kg body weight to 5 g/kg body weight, from 3 g/kg body weight to 5 g/kg body weight, from 3.5 g/kg body weight to 5 g/kg body weight, from 4 g/kg body weight to 5 g/kg body weight, from 4.5 g/kg body weight to 5 g/kg body weight, from 4.8 g/kg body weight to 5 g/kg body weight. In one embodiment, the dose range is from 5µg/kg body weight to 30µg/kg body weight. Alternatively, the dose range will be titrated to maintain serum levels between 5µg/mL and 30µg/mL.

The dosage of the agent and/or an asparaginase as described herein can be determined by a physician and adjusted, as necessary, to suit observed effects of the treatment. With respect to duration and frequency of treatment, it is typical for skilled clinicians to monitor subjects in order to determine when the treatment is providing therapeutic benefit, and to determine whether to administer further cells, discontinue treatment, resume treatment, or make other alterations to the treatment regimen. The dosage should not be so large as to cause adverse side effects, such as cytokine release syndrome. Generally, the dosage will vary with the age, condition, and sex of the patient and can be determined by one of skill in the art. The dosage can also be adjusted by the individual physician in the event of any complication.

### Combination treatment

The agent and an asparaginase described herein may be administered in combination for use in the treatment of cancer. Administered "in combination," as used herein, means that two (or more) different treatments (e.g., an asparaginase and an agent that inhibits GSK3α, or a cancer therapy) are delivered to the subject during the course of the subject's affliction with the disorder, e.g., the two or more treatments are delivered after the subject has been diagnosed with the disorder (e.g., cancer) and before the disorder has been cured or eliminated or treatment has ceased for other reasons. In some embodiments, the delivery of one treatment is still occurring when the delivery of the second begins, so that there is overlap in terms of administration. This is sometimes referred to herein as "simultaneous" or "concurrent delivery." In other embodiments, the delivery of one treatment ends before the delivery of the other treatment begins. In some embodiments of either case, the treatment is more effective because of combined administration. For example, the second treatment is more effective, e.g., an equivalent effect is seen with less of the second treatment, or the second treatment reduces symptoms to a greater extent, than would be seen if the second treatment were administered in the absence of the first treatment, or the analogous situation is seen with the first treatment. In some embodiments, delivery is such that the reduction in a symptom, or other parameter related to the disorder is greater than what would be observed with one treatment delivered in the absence of the other. The effect of the two treatments can be partially additive, wholly additive, or greater than additive. The delivery can be such that an effect of the first treatment delivered is still detectable when the second is delivered. The agents described herein and the at least one additional therapy can be administered simultaneously, in the same or in separate compositions, or sequentially. For sequential administration, the agent and/or an asparaginase described herein can be administered first, and the additional agent can be administered second, or the order of administration can be reversed. The agent and/or other therapeutic agents, procedures or modalities can be administered during periods of active disorder, or during a period of remission or less active disease. The agent can be administered before another treatment, concurrently with the treatment, post-treatment, or during remission of the disorder.

When administered in combination, the agent and an asparaginase, or all, can be administered in an amount or dose that is higher, lower or the same as the amount or dosage of each agent used individually, e.g., as a monotherapy. In certain embodiments, the administered amount or dosage of the agent, the additional agent (e.g., second or third agent), or all, is lower (e.g., at least 20%, at least 30%, at least 40%, or at least 50%) than the amount or dosage of each agent used individually. In other embodiments, the amount or dosage of agent, the additional agent (e.g., second or third agent), or all, that results in a desired effect (e.g., treatment of cancer) is lower (e.g., at least 20%, at least 30%, at least 40%, or at least 50% lower) than the amount or dosage of each agent individually required to achieve the same therapeutic effect.

In one embodiment, the cancer therapy is selected from the group consisting of chemotherapy, radiation therapy, immunotherapy, surgery, hormone therapy, stem cell therapy, targeted therapy, gene therapy, and precision therapy.

The cancer therapy may be selected from the group consisting of growth inhibitory agents, cytotoxic agents, anti-angiogenesis agents, apoptotic agents, anti-tubulin agents, anti-HER-2 antibodies, anti-CD20 antibodies, an epidermal growth factor receptor (EGFR) antagonist, a HER1/EGFR inhibitor, a platelet derived growth factor inhibitor, a COX-2 inhibitor, an interferon, and a cytokine (e.g., G-CSF, granulocyte - colony stimulating factor).

In other embodiments, the cancer therapy is selected from the group consisting of 13-cis-retinoic acid, 2-CdA, 2-Chlorodeoxyadenosine, 5-Azacitidine, azacytidine, 5-Fluorouracil, 5-FU, 6-Mercaptopurine, 6-MP, 6-TG, 6-Thioguanine, abiraterone acetate, Abraxane, Accutane ^{®}, Actinomycin-D, Adriamycin ^{®}, Adrucil ^{®}, Afinitor ^{®}, Agrylin ^{®}, Ala-Cort ^{®}, Aldesleukin, Alemtuzumab, ALIMTA, Alitretinoin, Alkaban-AQ ^{®}, Alkeran ^{®}, All-transretinoic Acid, Alpha Interferon, Altretamine, Amethopterin, Amifostine, Aminoglutethimide, Anagrelide, Anandron ^{®}, Anastrozole, Arabinosylcytosine, Ara-C, Aranesp ^{®}, Aredia ^{®}, Arimidex ^{®}, Aromasin ^{®}, Arranon ^{®}, Arsenic Trioxide, Arzerra^{™}, Asparaginase, ATRA, Avastin ^{®}, Axitinib, Azacitidine, BCG, BCNU, Bendamustine, Bevacizumab, Bexarotene, BEXXAR ^{®}, Bicalutamide, BiCNU, Blenoxane ^{®}, Bleomycin, Bortezomib, Busulfan, Busulfex ^{®}, C225, Cabazitaxel, Calcium Leucovorin, Campath ^{®} Camptosar ^{®} Camptothecin-11, Capecitabine, Caprelsa^{®} Carac ^{™} Carboplatin, Carmustine, Carmustine Wafer, Casodex ^{®}, CC-5013, CCI-779, CCNU, CDDP, CeeNU, Cerubidine ^{®}, Cetuximab, Chlorambucil,Cisplatin, Citrovorum Factor, Cladribine, Cortisone, Cosmegen ^{®}, CPT-11, Crizotinib, Cyclophosphamide, Cytadren ^{®}, Cytarabine, Cytarabine Liposomal, Cytosar-U ^{®}, Cytoxan ^{®}, Dacarbazine, Dacogen, Dactinomycin, Darbepoetin Alfa, Dasatinib, Daunomycin, Daunorubicin, Daunorubicin Hydrochloride, Daunorubicin Liposomal, DaunoXome ^{®}, Decadron, Decitabine, Delta-Cortef ^{®}, Deltasone ^{®},Denileukin Diftitox, Denosumab, DepoCyt ^{™}, Dexamethasone, Dexamethasone Acetate, Dexamethasone Sodium Phosphate, Dexasone, Dexrazoxane, DHAD, DIC, Diodex, Docetaxel, Doxil ^{®}, Doxorubicin, Doxorubicin Liposomal, Droxia ^{™}, DTIC, DTIC-Dome ^{®}, Duralone ^{®}, Eculizumab, Efudex ^{®}, Eligard ^{™}, Ellence ^{™}, Eloxatin ^{™}, Elspar ^{®}, Emcyt ^{®}, Epirubicin, Epoetin Alpha, Erbitux, Eribulin, Erlotinib, Erwinia L-asparaginase, Estramustine, Ethyol, Etopophos ^{®}, Etoposide, Etoposide Phosphate, Eulexin ^{®}, Everolimus, Evista ^{®}, Exemestane, Fareston ^{®}, Faslodex ^{®}, Femara ^{®}, Filgrastim, Floxuridine, Fludara ^{®}, Fludarabine, Fluoroplex ^{®}, Fluorouracil, Fluorouracil (cream), Fluoxymesterone, Flutamide, Folinic Acid, FUDR ^{®}, Fulvestrant, Gefitinib, Gemcitabine, Gemtuzumab ozogamicin, Gemzar, Gleevec ^{™}, Gliadel ^{®} Wafer, Goserelin, Granulocyte - Colony Stimulating Factor (G-CSF), Granulocyte Macrophage Colony Stimulating Factor (GM-CSF), Halaven ^{®}, Halotestin ^{®}, Herceptin ^{®}, Hexadrol, Hexalen ^{®}, Hexamethylmelamine, HMM, Hycamtin ^{®}, Hydrea ^{®}, Hydrocort Acetate ^{®}, Hydrocortisone, Hydrocortisone Sodium Phosphate, Hydrocortisone Sodium Succinate, Hydrocortone Phosphate, Hydroxyurea, Ibritumomab, Ibritumomab Tiuxetan, Idamycin ^{®}, Idarubicin, Ifex ^{®}, IFN-alpha, Ifosfamide, IL-11, IL-2, Imatinib mesylate, Imidazole Carboxamide, Inlyta ^{®}, Interferon alpha, Interferon Alpha-2b (PEG Conjugate), Interleukin - 2, Interleukin-11, Intron A^{®} (interferon alpha-2b), Ipilimumab, Iressa ^{®}, Irinotecan, Isotretinoin, Ixabepilone, Ixempra ^{™}, Jevtana^{®}, Kidrolase (t), Lanacort ^{®}, Lapatinib, L-asparaginase, LCR, Lenalidomide, Letrozole, Leucovorin, Leukeran, Leukine ^{™}, Leuprolide, Leurocristine, Leustatin ^{™}, Liposomal Ara-C, Liquid Pred ^{®}, Lomustine, L-PAM, L-Sarcolysin, Lupron ^{®}, Lupron Depot ^{®}, Matulane ^{®}, Maxidex, Mechlorethamine, Mechlorethamine Hydrochloride, Medralone ^{®}, Medrol ^{®}, Megace ^{®}, Megestrol, Megestrol Acetate, Melphalan, Mercaptopurine, Mesna, Mesnex ^{™}, Methotrexate, Methotrexate Sodium, Methylprednisolone, Meticorten ^{®}, Mitomycin, Mitomycin-C, Mitoxantrone, M-Prednisol ^{®}, MTC, MTX, Mustargen ^{®}, Mustine, Mutamycin ^{®}, Myleran ^{®}, Mylocel ^{™}, Mylotarg ^{®}, Navelbine ^{®}, Nelarabine, Neosar ^{®}, Neulasta ^{™}, Neumega ^{®}, Neupogen ^{®}, Nexavar ^{®}, Nilandron ^{®}, Nilotinib, Nilutamide, Nipent ^{®}, Nitrogen Mustard, Novaldex ^{®}, Novantrone ^{®}, Nplate, Octreotide, Octreotide acetate, Ofatumumab, Oncospar ^{®}, Oncovin ^{®}, Ontak ^{®}, Onxal ^{™}, Oprelvekin, Orapred ^{®}, Orasone ^{®}, Oxaliplatin, Paclitaxel, Paclitaxel Protein-bound, Pamidronate, Panitumumab, Panretin ^{®}, Paraplatin ^{®}, Pazopanib, Pediapred ^{®}, PEG Interferon, Pegaspargase, Pegfilgrastim, PEG-INTRON ^{™}, PEG-L-asparaginase, PEMETREXED, Pentostatin, Phenylalanine Mustard, Platinol ^{®}, Platinol-AQ ^{®}, Prednisolone, Prednisone, Prelone ^{®}, Procarbazine, PROCRIT ^{®}, Proleukin ^{®}, Prolia ^{®}, Prolifeprospan 20 with Carmustine Implant, Provenge ^{®}, Purinethol ^{®}, Raloxifene, Revlimid ^{®}, Rheumatrex ^{®}, Rituxan ^{®}, Rituximab, Roferon-A ^{®} (Interferon Alfa-2a), Romiplostim, Rubex ^{®}, Rubidomycin hydrochloride, Sandostatin ^{®}, Sandostatin LAR ^{®}, Sargramostim, Sipuleucel-T, Soliris ^{®}, Solu-Cortef ^{®}, Solu-Medrol ^{®}, Sorafenib, SPRYCEL ^{™}, STI-571, Streptozocin, SU11248, Sunitinib, Sutent ^{®}, Tamoxifen, Tarceva ^{®}, Targretin ^{®}, Tasigna ^{®}, Taxol ^{®}, Taxotere ^{®}, Temodar ^{®}, Temozolomide, Temsirolimus, Teniposide, TESPA, Thalidomide, Thalomid ^{®}, TheraCys ^{®}, Thioguanine, Thioguanine Tabloid ^{®}, Thiophosphoamide, Thioplex ^{®}, Thiotepa, TICE ^{®}, Toposar ^{®}, Topotecan, Toremifene, Torisel ^{®}, Tositumomab, Trastuzumab, Treanda ^{®}, Tretinoin, Trexall ^{™}, Trisenox ^{®}, TSPA, TYKERB ^{®}, Valrubicin, Valstar, vandetanib, VCR, Vectibix ^{™}, Velban ^{®}, Velcade ^{®}, Vemurafenib, VePesid ^{®}, Vesanoid ^{®}, Viadur ^{™}, Vidaza ^{®}, Vinblastine, Vinblastine Sulfate, Vincasar Pfs ^{®}, Vincristine, Vinorelbine, Vinorelbine tartrate, VLB, VM-26, Vorinostat, Votrient, VP-16, Vumon ^{®}, Xalkori capsules, Xeloda ^{®}, Xgeva^{®}, Yervoy^{®}, Zanosar ^{®}, Zelboraf, Zevalin ^{™}, Zinecard ^{®}, Zoladex ^{®}, Zoledronic acid, Zolinza, Zometa ^{®}, and Zytiga^{®}.

### Parenteral Dosage Forms

Parenteral dosage forms of an agents described herein and/or an asparaginase can be administered to a subject by various routes, including, but not limited to, subcutaneous, intravenous (including bolus injection), intramuscular, and intraarterial. Since administration of parenteral dosage forms typically bypasses the patient's natural defenses against contaminants, parenteral dosage forms are preferably sterile or capable of being sterilized prior to administration to a patient. Examples of parenteral dosage forms include, but are not limited to, solutions ready for injection, dry products ready to be dissolved or suspended in a pharmaceutically acceptable vehicle for injection, suspensions ready for injection, controlled-release parenteral dosage forms, and emulsions.

Suitable vehicles that can be used to provide parenteral dosage forms of the disclosure are well known to those skilled in the art. Examples include, without limitation: sterile water; water for injection USP; saline solution; glucose solution; aqueous vehicles such as but not limited to, sodium chloride injection, Ringer's injection, dextrose Injection, dextrose and sodium chloride injection, and lactated Ringer's injection; water-miscible vehicles such as, but not limited to, ethyl alcohol, polyethylene glycol, and propylene glycol; and non-aqueous vehicles such as, but not limited to, corn oil, cottonseed oil, peanut oil, sesame oil, ethyl oleate, isopropyl myristate, and benzyl benzoate.

### Controlled and Delayed Release Dosage Forms

In some embodiments, an agent and/or an asparaginase is administered to a subject by controlled- or delayed-release means. Ideally, the use of an optimally designed controlled-release preparation in medical treatment is characterized by a minimum of drug substance being employed to cure or control the condition in a minimum amount of time. Advantages of controlled-release formulations include: 1) extended activity of the drug; 2) reduced dosage frequency; 3) increased patient compliance; 4) usage of less total drug; 5) reduction in local or systemic side effects; 6) minimization of drug accumulation; 7) reduction in blood level fluctuations; 8) improvement in efficacy of treatment; 9) reduction of potentiation or loss of drug activity; and 10) improvement in speed of control of diseases or conditions. (Kim, Cherng-ju, Controlled Release Dosage Form Design, 2 (Technomic Publishing, Lancaster, Pa.: 2000)). Controlled-release formulations can be used to control a compound of formula (I)'s onset of action, duration of action, plasma levels within the therapeutic window, and peak blood levels. In particular, controlled- or extended-release dosage forms or formulations can be used to ensure that the maximum effectiveness of an agent is achieved while minimizing potential adverse effects and safety concerns, which can occur both from under-dosing a drug (i.e., going below the minimum therapeutic levels) as well as exceeding the toxicity level for the drug.

A variety of known controlled- or extended-release dosage forms, formulations, and devices can be adapted for use with any agent described herein. Examples include, but are not limited to, those described in U.S. Pat. Nos.: 3,845,770; 3,916,899; 3,536,809; 3,598,123; 4,008,719; 5674,533; 5,059,595; 5,591 ,767; 5,120,548; 5,073,543; 5,639,476; 5,354,556; 5,733,566; and 6,365,185. These dosage forms can be used to provide slow or controlled-release of one or more active ingredients using, for example, hydroxypropylmethyl cellulose, other polymer matrices, gels, permeable membranes, osmotic systems (such as OROS^{®} (Alza Corporation, Mountain View, Calif. USA)), multilayer coatings, microparticles, liposomes, or microspheres or a combination thereof to provide the desired release profile in varying proportions. Additionally, ion exchange materials can be used to prepare immobilized, adsorbed salt forms of the disclosed compounds and thus effect controlled delivery of the drug. Examples of specific anion exchangers include, but are not limited to, DUOLITE^{®} A568 and DUOLITE^{®} AP143 (Rohm&Haas, Spring House, Pa. USA).

### Efficacy

The efficacy of an agent described herein and/or an asparaginase, e.g., for the treatment of cancer, can be determined by the skilled practitioner. However, a treatment is considered "effective treatment," as the term is used herein, if one or more of the signs or symptoms of cancer are altered in a beneficial manner, other clinically accepted symptoms are improved, or even ameliorated, or a desired response is induced e.g., by at least 10% following treatment according to the methods described herein. Efficacy can be assessed, for example, by measuring a marker, indicator, symptom, and/or the incidence of a condition treated (e.g., cancer) according to the methods described herein or any other measurable parameter appropriate. Efficacy can also be measured by a failure of an individual to worsen as assessed by hospitalization, or need for medical interventions (i.e., progression of cancer). Methods of measuring these indicators are known to those of skill in the art and/or are described herein.

Efficacy can be assessed in animal models of a condition described herein, for example, a mouse model or an appropriate animal model of a given cancer, as the case may be. When using an experimental animal model, efficacy of treatment is evidenced when a statistically significant change in a marker is observed, e.g., a reduction in tumor size, or prevention of metastasis.

All patents, patent applications, and publications identified are for the purpose of describing and disclosing, for example, the methodologies described in such publications that might be used in connection with the present invention. These publications are provided solely for their disclosure prior to the filing date of the present application. Nothing in this regard should be construed as an admission that the inventors are not entitled to antedate such disclosure by virtue of prior invention or for any other reason. All statements as to the date or representation as to the contents of these documents is based on the information available to the applicants and does not constitute any admission as to the correctness of the dates or contents of these documents.

### EXAMPLES

### Example 1

To identify molecular pathways that promote fitness of asparaginase-treated leukemia cells, a genome-wide CRISPR/Cas9 loss-of-function genetic screen was performed in the T-ALL cell line CCRF-CEM, which is asparaginase-resistant (Fig. 5). Conditions for a drop-out screen were optimized using positive control guide RNAs targeting asparagine synthetase (ASNS), the enzyme that synthesizes asparagine (Fig. 6)⁶.

Upregulation of ASNS can induce resistance to asparaginase⁷, but this is not the sole determinant of asparaginase response^{8,9}. Cas9-expressing CCRF-CEM cells were then transduced with the GeCKO genome-wide guide RNA library¹⁰ (Fig. 1a and Fig. 7), treated with either vehicle or a 10 U/L dose of asparaginase that lacked detectable toxicity, and guide RNA representation was assessed. ASNS was the gene most significantly depleted in asparaginase-treated cells, followed closely by two regulators of Wnt signaling, NKD2 and LGR6 (Fig. 1b).

NKD2 negatively regulates Wnt signaling by binding and repressing dishevelled proteins¹¹, whereas LGR proteins are R-spondin receptors reported to either activate or repress Wnt signaling^{12,13}. To test how these genes regulate Wnt signaling, CCRF-CEM cells were transduced with shRNAs targeting NKD2 or LGR6, or with an shLuciferase control (Fig. 1c). Knockdown of NKD2 or LGR6 increased levels of active (nonphosphorylated) β-catenin (Fig. 1d), as well as the activity of a TopFLASH reporter of canonical Wnt/β-catenin transcriptional activity¹⁴ (Fig. 1e). Thus, NKD2 and LGR6 are negative regulators of Wnt signaling in T-ALL cells. To validate that loss of NKD2 or LGR6 sensitizes these cells to asparaginase, CCRF-CEM cells were transduced with these shRNAs and treated with asparaginase. Knockdown of NDK2 or LGR6 profoundly sensitized the cells to asparaginase (Fig. 1f), and potentiated asparaginase-induced caspase activation, indicating induction of apoptosis (Fig. 1g). This effect was phenocopied by treatment with the Wnt ligand Wnt3A (Fig. 1h). Thus, Wnt pathway activation sensitizes T-ALL cells to asparaginase.

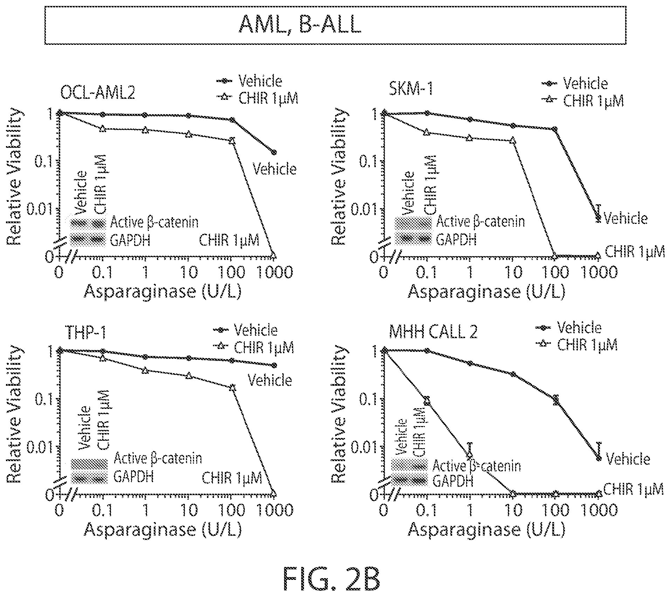

Inhibition of glycogen synthase kinase 3 (GSK3) activity is a key event in Wnt-induced signal transduction¹⁵, prompting the determination as to whether this effect could be phenocopied by CHIR99021, an ATP-competitive inhibitor of both GSK3 paralogs, GSK3α and GSK3β16. CHIR99021 induced significant asparaginase sensitization across a panel of cell lines representing distinct subtypes of treatment-resistant acute leukemia, including T-ALL, acute myeloid leukemia and hypodiploid B-ALL (Fig. 2a, b). Importantly, CHIR99021 did not increase the toxicity of asparaginase to normal human CD34+ hematopoietic progenitors (Fig. 2c), suggesting a selective effect on leukemic cells. Nor did it sensitize leukemic cells to other commonly used antileukemic drugs, including the microtubule inhibitor vincristine, the nucleoside analog 6-mercaptopurine, the glucocorticoid receptor agonist dexamethasone, and the DNA-damaging agent doxorubicin (Fig. 2d). Thus, GSK3 inhibition can enhance asparaginase toxicity not only in T-ALL, but in other common acute leukemia variants as well.

Canonical Wnt signaling is best known as an activator of β-catenin-dependent transcriptional activity^{17,18}, leading to the question of whether β-catenin activation is sufficient to induce asparaginase sensitization. Thus, CCRF-CEM cells were transduced with a constitutively active ΔN90 β-catenin allele, or shRNA targeting the β-catenin antagonist gene APC19, and treated them with asparaginase. Surprisingly, these modifications lacked any discernible effect on asparaginase sensitivity, despite effective activation of β-catenin-induced transcription, as assessed by TopFLASH reporter activity (Fig. 3a-b). The Wnt pathway also regulates protein synthesis and metabolism by activating both mTOR complexes, mTORC1²⁰ and mTORC2²¹. However, treatment with the mTORC1 inhibitors rapamycin and RAD001, or with the dual mTORC1/2 inhibitor AZD2014, had no effect on Wnt-induced sensitization to asparaginase (Fig. 3c and Fig. 8). On balance, these data indicate that asparaginase sensitization is independent of β-catenin or mTOR activity.

Activation of Wnt signaling increases total cellular protein content and cell size by inhibiting GSK3-dependent protein ubiquitination and proteasomal degradation, an effect termed Wnt-dependent stabilization of proteins (Wnt/STOP)^{4,22,23}. This function of the Wnt pathway appears relevant to asparaginase sensitization. A measurable decrease in cell size after treatment with the enzyme was observed, even in CCRF-CEM cells, which are highly resistant to asparaginase-induced cytotoxicity (Fig. 3d), and this effect was reversed by Wnt pathway activation (Fig. 3e). To test whether Wnt pathway activation inhibits protein degradation in asparaginase-treated cells, a pulse-chase experiment was used to measure total protein half-life. CCRF-CEM cells were first transduced with Wnt-activating shRNAs targeting NKD2 and LGR6, or with an shLuciferase control. Cells were then incubated with a pulse of the methionine analog azidohomoalanine (AHA), followed by a chase in which the cells were treated with asparaginase, and the rate of AHA release due to protein degradation was measured by flow cytometry. Wnt pathway activation did not affect the degree of AHA label incorporation during the pulse period (Fig. 9); however, shRNA knockdown of LGR6 or NDK2 increased total protein half-life by approximately 40% in these cells (Fig. 3f). These findings indicate that Wnt pathway activation inhibits protein degradation in asparaginase-treated cells.

The E3 ubiquitin ligase component FBXW7 recognizes a canonical phosphodegron that is phosphorylated by GSK3, and overexpression of FBXW7 restores the degradation of a subset of proteins stabilized by Wnt/STOP signaling⁴. Thus, it was contemplated that FBXW7 overexpression can also reverse Wnt/STOP induced asparaginase sensitization. After transducing CCRF-CEM with Wnt-activating shRNAs or an shLuciferase control, the same cells were transduced with expression constructs encoding either wild-type FBXW7, or an FBXW7 R465C point mutant allele with impaired binding to its canonical phospho-degron²⁴. Overexpression of wild-type FBXW7 blocked the ability of Wnt pathway activation to trigger asparaginase hypersensitivity, whereas the R465C mutant had no effect (Fig. 3g). However, FBXW7 is a T-ALL tumor suppressor that can target specific oncoproteins²⁵, prompting the question of whether direct manipulation of proteasomal activity can modulate Wnt-induced sensitization to asparaginase. Inhibiting the catalytic activity of the 26S proteasome using bortezomib²⁶, using a 10 nM dose that lacked single-agent toxicity, phenocopied Wnt-induced sensitization to asparaginase (Fig. 3h). Direct stimulation of proteasomal activity was tested to determine if this effect was sufficient to reverse the asparaginase-selective toxicity of Wnt pathway activation, leveraging a hyperactive open-gate (ΔN3) mutant of the proteasomal subunit PSMA4, whose expression is sufficient to stimulate degradation of a wide range of proteasomal substrates⁵. CCRF-CEM cells were first transduced with control or Wnt-activating shRNAs, and then transduced with the constitutively active proteasomal subunit ΔN3-PSMA4, or a GFP control. Expression of the constitutively active ΔN3-PSMA4 proteasomal subunit completely blocked Wnt-induced sensitization to asparaginase (Fig. 3i). These findings indicate that Wnt pathway activation sensitizes leukemic cells to asparaginase by inhibiting proteasomal degradation of proteins.

To explore the therapeutic potential of the studies previously described, GSK3 was the focus as a therapeutic target because its inhibition had no detectable toxicity to these cells, in contrast to the proteasome inhibitor bortezomib, which was highly toxic at intermediate doses (Fig. 10). Although the pan-GSK3 inhibitor that was used in cell lines lack the suitable pharmacokinetic properties for in vivo studies, isoform-selective inhibitors of each GSK3 paralog with favorable pharmacologic properties have recently been developed²⁷. The GSK3α-selective inhibitor BRD0705 effectively sensitizes CCRF-CEM cells to asparaginase, while the GSK3β-selective inhibitor BRD0731 has only modest effects (Fig. 4a). To determine whether this result reflects redundancy between GSK3 isoforms, or partial inhibition of GSK3α by the GSK3β inhibitor BRD0731 shRNAs were used to selectively deplete GSK3α or GSK3β. Depletion of GSK3α phenocopied Wnt-induced sensitization to asparaginase, whereas GSK3β knockdown had no effect (Fig. 4b and Fig. 11). The effect of GSK3α-targeting shRNAs was rescued by transduction of a GSK3α expression construct that escapes shRNA targeting (Fig. 4c).

Combined treatment with the GSK3α inhibitor BRD0705 and asparaginase was well-tolerated by NRG mice, with no appreciable weight changes or increases in serum bilirubin levels, an important dose-limiting toxicity of asparaginase in adults (data not shown). Finally, a cohort of NRG mice were injected with leukemic cells from a primary asparaginase-resistant T-ALL patient-derived xenograft. Once leukemia engraftment was detected in the peripheral blood, the mice were treated with vehicle, asparaginase, BRD0705, or the combination of asparaginase and BRD0705. Although the xenografts proved highly resistant to asparaginase and BRD0705 monotherapy, the combination of asparaginase and BRD0705 was both highly efficacious and well-tolerated (Fig. 4d-e and Fig. 12).

These studies demonstrate a previously unrecognized synthetic lethal interaction between activation of Wnt/STOP signaling and asparaginase in acute leukemias resistant to this enzyme. The findings support a model in which asparaginase-resistant leukemias adapt to asparagine depletion by increasing protein degradation to replenish the pool of intracellular asparagine, thus repressing cell death (Fig. 4f). Interestingly, the combination of Wnt/STOP activation and asparaginase is selectively toxic to leukemia cells, whereas normal cells appear to have effective compensatory mechanisms. This result conceivably reflects checkpoints that normally couple proliferation to asparagine availability, a link that could be readily disrupted by oncogenic mutations. The combination of Wnt/STOP activation and asparaginase could have broad efficacy against tumors harboring relevant gene mutations. It is specifically contemplated herein that asparaginase monotherapy has therapeutic use in tumors driven by mutations that activate Wnt/STOP signaling, such as R-spondin translocations or inactivating mutations of RNF43 that potentiate ligand-induced Wnt signaling in colorectal carcinomas²⁸⁻³⁰.

### Material and Methods

**Cell lines and cell culture.** 293T cells, T-ALL cell lines, AML cell lines and B-ALL cell lines were obtained from ATCC (Manassas, VA, USA), DSMZ (Braunschweig, Germany), the A. Thomas Look laboratory (Boston, MA, USA) or Alex Kentsis laboratory (New York, NY, USA) and cultured in DMEM, RPMI 1640 or MEM alpha (Thermo Fisher Scientific) with 10% or 20% fetal bovine serum (FBS, Sigma-Aldrich, Saint Louis, MO) or TET system approved FBS (Clontech, Mountain View, CA) and 1% penicillin/streptomycin (Thermo Fisher Scientific) at 37°C, 5% CO2. Human CD34+ progenitor cells from mobilized peripheral blood of healthy donors were obtained from Fred Hutchinson Cancer Research Center (Seattle, WA, USA) ( e.g., found on the world wide web at sharedresources.fredhutch.org/products/cd34-cells). CD34+ progenitors were cultured in IMDM (Thermo Fisher Scientific) supplemented with 20% FBS and recombinant human interleukin-3 (R&D systems, Minneapolis, MN), recombinant human interleukin-6 (R&D systems, Minneapolis, MN) and recombinant human stem cell factor (R&D systems, Minneapolis, MN) to a final concentration of 50 ng/ml each.

Cell line identities were validated using STR profiling at the Dana-Farber Cancer Institute Molecular Diagnostics Laboratory (most recently in June 2018), and mycoplasma contamination was excluded using the MycoAlert Mycoplasma Detection Kit according to the manufacturer's instructions (Lonza, Portsmouth, NH; most recently in March 2018).

**Lentiviral and retroviral production, transduction and selection.** Lentiviruses were generated by co-transfecting pLKO.1 plasmids of interest together with packaging vectors psPAX2 (a gift from Didier Trono; addgene plasmid # 12260) and VSV.G (a gift from Tannishtha Reya¹; addgene plasmid # 14888) using OptiMEM (Invitrogen, Carlsbad, CA) and Fugene (Promega, Madison, WI), as previously described². Retrovirus was produced by co-transfecting plasmids with packaging vectors gag/pol¹ (a gift from Tannishtha Reya; addgene plasmid # 14887) and VSV.G.

Lentiviral and retroviral infections were performed by spinoculating T-ALL cell lines with virus-containing media (1,500 g x 90 minutes) in the presence of 8 µg/ml polybrene (Merck Millipore, Darmstadt, Germany). Selection with antibiotics was started 24 hours after infection with neomycin (700 µg/ml for a minimum of 5 days; Thermo Fisher Scientific), puromycin (1 µg/ml for a minimum of 48 hours; Thermo Fisher Scientific), or blasticidin (15 □g/ml for a minimum of 5 days; Invivogen).

Transient transfection was performed using Lipofectamine 2000 reagent (invitrogen). Briefly, 800,000 cells were seeded in 2ml of growth medium in 24 well plates. Five µg plasmid of interest and 10 µl lipofectamine were mixed with 300 µl OptiMEM, incubated for 10 minutes and added to the wells. Antibiotic selection was begun after 48 hours of incubation.

**Pooled ASNS/AAVS1 library.** A pooled guide RNA library with 3 unique guide RNAs targeting genomic loci encoding the catalytic domain of *ASNS* (e.g., found on the world wide web at www.uniprot.org/uniprot/P08243), and 3 unique guide RNAs targeting the safeharbor AAVS1 locus located in intron 1 of the *PPPIR12C* gene³, were designed as described⁴. The oligos were cloned to a modified version of lentiGuide-Puro (a gift from Feng Zhang, addgene plasmid # 52963) in which the guide RNA scaffold was replaced by a structurally optimized form (A-U flip and stem extension, called combined modification) previously reported to increase the efficiency of Cas9 targeting.⁵ Briefly, guide RNAs targeting the relevant genomic loci were designed using the Zhang lab CRISPR design tool (e.g., found on the world wide web at crispr.mit.edu/). One µl of 100 µm forward and reverse oligo was mixed with 1 µl 10X T4 DNA Ligation Buffer (NEB, Ipswich, MA), 6.5 µl ddH2O, 0.5 µl T4 PNK (NEB) and annealed for 30 minutes at 37°C and 5 min 95°C. 1 µl phosphoannealed oligo (diluted 1:500) was then ligated into 1 µl of BsmBI-digested lentiviral pHK09-puro plasmid using 1 µl Quick ligase (NEB), 5 µl 2x Quick Ligase buffer (NEB) and 2 µl ddH2O for 5 minutes at room temperature.

Guide RNA target sequences were as follows:
ASNS_a- GGAAGACAGCCCCGATTTAC (SEQ ID NO: 3); ASNS_b-AGGATCAGATGAACTTACGC (SEQ ID NO: 4); ASNS_c-CAGCAGTAGTTCGATCTGCG (SEQ ID NO: 5); AAVS1_a-AGCGGCTCCAATTCGGAAGT (SEQ ID NO: 6); AAVS1_b-GAGAGGTGACCCGAATCCAC (SEQ ID NO: 7); AAVS1_c-AGTTCTTAGGGTACCCCACG (SEQ ID NO: 8).

Pooled lentivirus was produced by co-transfecting equal amounts of each of these guide RNAs together with the psPAX2 and VSV.G vectors as described above. Virus was concentrated using a Beckmann XL-90 ultracentrifuge (Beckman Coulter) at 100,000 g (24,000 rpm) for 2 hours at 4°C. Viral titers were determined using alamarBlue staining, as described (e.g., found on the world wide web at portals.broadinstitute.org/gpp/public/resources/protocols). CCRF-CEM cells (40,000 per well in 100 µl RPMI medium) infected with lentiCas9-blast (a gift from Feng Zhang; addgene plasmid # 52962) were plated in 96-well format and transduced with lentivirus at multiplicity of infection (MOI) of 0.3. Infected cells were selected 48 hours postinfection with puromycin at 1 µg/ml for 7 days. Infected cells were treated with vehicle (PBS) or asparaginase in 24-well format (400,000 cell per well in 1 ml RPMI medium). Cells were harvested 5 days after start of asparaginase treatment, and genomic DNA was extracted using DNeasy Blood and Tissue Kit (Qiagen). Guide RNA sequences were PCR-amplified using pHKO9 sequencing primers (pHKO9 F - TCTTGTGGAAAGGACGAAACACCG (SEQ ID NO: 9); pHKO9 R - TCTACTATTCTTTCCCCTGCACTGT (SEQ ID NO: 10)), PCR-purified using the QIAquick PCR purification Kit (Qiagen), and next-generation "CRISPR sequencing" was performed at the MGH CCIB DNA Core facility (e.g., found on the world wide web at dnacore.mgh.harvard.edu/new-cgi-bin/site/pages/crispr_sequencing_main.jsp). Cutting efficiency was assessed using CrispRVariantsLite v1.1⁶.

**Genome-wide loss of function screen and data analysis.** The genome-wide loss-of function screen was performed using the GeCKO v2 human library, as described^{4,7}. CCRF-CEM cells were first transduced with lentiCas9-blast, selected with blasticidin, and Cas9 activity was confirmed using a self-excising GFP construct, pXPR_011⁸ (a gift from John Doench & David Root; addgene plasmid # 59702). GeCKO v2 consists of two half-libraries (A and B), each of which was transduced in biologic duplicates into 1.8 x 10⁸ Cas9-expressing CCRF-CEM cells at MOI = 0.3. Cells were selected with puromycin (1 µg/ml) beginning 24 hours post transduction, which was continued for 8 days. Based on the number of cells that survived selection and estimated growth rate, coverage was estimated at 663x for GeCKO half-library A, and 891x for GeCKO half-library B. Cells were split every other day, and the minimum number of cells kept at each split was 84 million for each half-library, in order to minimize loss of guide RNA coverage. Cells were treated with 10 U/L asparaginase beginning on day 10, and cells were harvested after 5 days of treatment. Genomic DNA was extracted using the Blood & Cell Culture DNA Maxi Kit (Qiagen). Samples were sequenced using CRISPR sequencing at the MGH CCIB DNA Core facility as described above. Significance of gene depletion based on guide RNA drop-out was calculated using MAGeCK software (e.g., found on the world wide web at sourceforge.net/projects/mageck/)⁹.

**shRNA and expression plasmids.** The following lentiviral shRNA vectors in pLKO.1 with puromycin resistance were generated by the RNAi Consortium library of the Broad Institute, and obtained from Sigma-Aldrich: shLuciferase (TRCN0000072243), shNKD2#1 (TRCN0000187580), shNKD2#3 (TRCN0000428381), shLGR6#2 (TRCN0000063619) shLGR6#4 (TRCN0000063621), shAPC#1 (TRCN0000010296), shAPC#2 (TRCN0000010297), shGSK3α#1 (TRCN0000010340), shGSK3α#4 (TRCN0000038682), shGSK3β#2 (TRCN0000039564), shGSK3β#6 (TRCN0000010551).

The construct encoding a constitutively active β-catenin (ΔN90) allele was a gift from Bob Weinberg (e.g., found on the world wide web at www.addgene.org/36985/)¹⁰. Expression constructs expressing wild-type FBXW7 (also known as CDC4) or its R465C mutant were a gift from Bert Vogelstein (e.g., found on the world wide web at www.addgene.org/16652/ and www.addgene.org/16653/)¹¹. The 7TGC (TxTcf-eGFP//SV40-mCherry) TopFLASH reporter was a gift from Roel Nusse (e.g., found on the world wide web at www.addgene.org/24304/)12. A hyperactive open-gate mutant of the human proteasomal subunit PSMA4 harboring a deletion of the N-terminal amino acids 2-10 (based on isoform NP_002780.1), termed ΔN-PSMA4, was designed based on the data of Choi and colleagues¹³, and synthesized with a C-terminal V5 tag in a pLX304 lentiviral expression vector by GeneCopoeia (Rockville, MD). The GSK3α pWZL expression vector was previously described¹⁴.

### Assessment of chemotherapy response and chemotherapy-induced apoptosis

Cells (25,000 per well) were seeded in 100µl of complete growth medium in 96-well plates and incubated with chemotherapeutic agents or vehicle. T-ALL cells were split every 48 hours in a 1:5 ratio. Briefly, 20 µl of cells were mixed with 80 µl fresh culture medium, supplemented with vehicle or chemotherapeutic drugs at the indicated doses. AML cells were split every 72 hours as described above. Cell viability was assessed by counting viable cells based on trypan blue vital dye staining (Invitrogen), according to the manufacturer's instructions. Chemotherapeutic drugs included: PEG-asparaginase ("oncaspar", Shire, Lexington, MA), dexamethasone (Sigma-Aldrich), vincristine (Selleckchem, Houston, TX), doxorubicin (Sigma-Aldrich), 6-mercaptopurine (Abcam, Cambridge, UK), CHIR99021 (Selleckchem), bortezomib (Selleckchem), rapamycin (Selleckchem), RAD001 (Selleckchem), AZD2014 (Selleckchem), and Wnt3A (R&D systems, Minneapolis, MN). BRD0705 and BRD0731 were synthesized as described¹⁵. Caspase 3/7 activity was assessed using the Caspase Glo 3/7 Assay (Promega, Madison, WI) according to the manufacturer's instructions.

**TopFLASH reporter.** Briefly, 400,000 CCRF-CEM cells were transduced with the TopFLASH reporter 7TGC¹², as described in the lentiviral infection section above. Cells expression the constitutive mCherry selection marker were selected by fluorescence activated cell sorting (FACS), treated with the Wnt ligand Wnt3A for 5 days, and cells with Wnt-inducible GFP expression were selected by sorting for mCherry and GFP double-positive cells. Selected cells were released from the Wnt ligand for a minimum of 7 days and then further manipulated with expression plasmids. GFP positivity of the mCherry positive cell fraction was assessed on a Beckton-Dickinson LSR-II instrument.

**Western blot and antibodies.** Cells were lysed in RIPA buffer (Merck Millipore) supplemented with cOmplete protease inhibitor (Roche, Basel, Switzerland) and PhosSTOP phosphatase inhibitor (Roche). Laemmli sample buffer (Bio-Rad, Hercules, CA) and β-mercaptoethanol (Sigma-Aldrich) were mixed with 20 µg of protein lysate before being run on a 4-12% Novex bis-tris polyacrylamide gel (Thermo Fisher Scientific). Blots were transferred to PVDF membrane (Thermo Fisher Scientific) and blocked with 5% BSA (New England Biolabs) in phosphate-buffered saline with 0.1% Tween (Boston Bioproducts, Ashland, MA) and probed with the following antibodies: Non-phospho β-catenin (Ser33/37/Thr41) antibody (1:1000, Cell Signaling Technologies #8814), total β-catenin antibody (1:1000, Cell Signaling Technologies #8480), total GSK3α/β antibody (1:1000, Cell Signaling Technologies #5676), phospho-GSK3α/β (Tyr279/216) antibody (Thermo Fisher Scientific #OPA1-03083), Myc-tag antibody (1:1000, Cell Signaling Technologies #2272), phospho-p70 S6 kinase (Thr389) antibody (1:1000 Cell Signaling Technologies #9234) or GAPDH (1:1000, Cell Signaling Technologies #2118). Secondary detection of horseradish peroxidase-linked antibodies (1:2000, Cell Signaling Technologies #7074S) with horseradish peroxidase substrate (Thermo Fisher Scientific) was visualized using Amersham Imager 600 (GE Healthcare Life Sciences, Marlborough, MA).

**Assessment of cell size.** Cells (400,000 per well) were plated in 1ml of complete growth medium, containing a final concentration of 10 U/L asparaginase in a 24-well format. After 48 hours of treatment, forward scattering (FSC-H) was assessed by flow cytometry.

### Quantitative reverse transcriptase PCR (qRT-PCR) and primers

RNA was isolated using RNeasy kit (Qiagen) and cDNA was made using SuperScript III first-strand cDNA synthesis kit (Thermo Fisher Scientific). qRT-PCR was performed using Power SYBR^{®} Green PCR Master Mix (Thermo Fisher Scientific) and 7500 real-time PCR system (Applied Biosystems). Primers used were as follows:

| | |
|---|---|
| Human β-actin F: | CTGGCACCCAGCACAATG (SEQ ID NO:11) |
| Human β-actin R: | GCCGATCCACACGGAGTACT (SEQ ID NO: 12) |
| Human NKD2 F: | ACCCTCCGTGTGAAGCTAAC (SEQ ID NO: 13) |
| Human NKD2 R: | GGGCCTCCTGACGTGTG (SEQ ID NO: 14) |
| Human LGR6 F: | TCGGGCTGTCCGCCGTTC (SEQ ID NO: 15) |
| Human LGR6 R: | GCTCCTCCAAGAAGCGCAGGTG (SEQ ID NO: 16) |
| Human APC F: | AACCGCTGCAGATGGCTGATGTG (SEQ ID NO: 17) |
| Human APC R: | TGCAGCCATCCTTGGCTACCCT (SEQ ID NO: 18) |
| Human GSK3α F: | TCCCCAGCGGGCACTACCA (SEQ ID NO: 19) |
| Human GSK3α R: | TGAGGGTAGGTGTGG CATCGGT (SEQ ID NO: 20) |
| Human GSK3β F: | CCAGGGGATAGTGGTGTGGATCAGT (SEQ ID NO: 21) |
| Human GSK3β R: | GAAGACCCGCACTCCTGAGCTGA (SEQ ID NO: 22) |

**Mice.** NOD rag gamma (NRG) mice 6-8 weeks of age were purchased from the Jackson Laboratories (Bar Harbor, ME; Stock # 007799). Mice were handled in strict accordance with Good Animal Practice as defined by the Office of Laboratory Animal Welfare. All animal work was done with Boston Children's Hospital (BCH) Institutional Animal Care and Use Committee approval (protocol # 15-10-3058R).

Mice were exposed to a sub-lethal dose of radiation (4.5 Gy) and subsequently injected with human leukemia cells. All injections were performed by tail vein injection. Blood collections were performed to monitor leukemia onset in mice injected with leukemic cells, which was assessed by staining for human CD45 expression using anti-human CD45-PE-Cy7 (1:400, BD# 560915) antibody staining. As soon as leukemia engraftment was confirmed based on ≥5% human leukemia cells in the peripheral blood, treatment of mice was begun. Asparaginase (1,000 U/kg) or PBS were injected by tail vein injection as a single dose on day 1 of drug treatment, and BRD0705 (15 mg/kg) or vehicle were given every 12 hours for 12 days by oral gavage. Vehicle was formulated as previously described.¹⁵ Mice were anesthetized with isoflurane (Patterson Veterinary, Greeley, CO) prior to gavage. After start of treatment, body weight was monitored every third day. To assess bilirubin levels, mandibular blood vein collection was performed, and bilirubin levels were measured in the Boston Children's Hospital clinical laboratory. The primary endpoint of this experiment was survival. Mice were euthanized as soon as they developed weight loss greater than 15% or signs/symptoms of progressing disease. Post-mortem analysis of leukemic burden was performed by extracting bone marrow cells, which were filtered through a 40 µM mesh filter and red blood cells were lysed using the BD Biosciences Red Blood Cell Lysis reagent (BD #555899). Isolated bone marrow cells were stained for assessment of leukemic burden using following antibodies from BD Biosciences: Anti-human CD4-APC-Cy7 (1: 100, BD# 561839) and anti-human CD8-PerCP-Cy5.5 (1: 100, BD# 560662).

**Assessment of protein stability by pulse-chase.** Protein degradation was assessed using a non-radioactive quantification of the methionine analog L-azidohomoalanine (AHA), as previously described¹⁶. Briefly, 400,000 CCRF-CEM cells were seeded in 1 ml of methionine-free RPMI containing 10% dialyzed FBS. After 30 minutes, the pulse step was performed by replacing this media with 1 ml RPMI supplemented with 10% dialyzed FBS and AHA at a final concentration of 50 µM for 18 hours. In the chase step, cells were released from AHA by replacing media with RPMI containing 10% dialyzed FBS and 10x _{L}-methionine (2 mM) for 2 hours. Subsequently, media was replaced with regular growth medium and cells were treated with a final concentration of 10 U/L asparaginase, followed by fixation of cells. AHA labeled proteins were tagged using TAMRA alkyne click chemistry, and fluorescence intensity was measured by flow cytometry. A sample without AHA labeling but TAMRA alkyne tag was included as a negative control to account for background fluorescence.

**Statistical analyses.** For two-group comparisons of continuous measures, a two-tailed Welch unequal variances t-test was used. For 3-group comparisons, a one-way analysis of variance model (ANOVA) was performed and a Dunnett adjustment for multiple comparisons was used. For analysis of two effects, a two-way ANOVA model was constructed and unless indicated included an interaction term between the two effects. Post-hoc adjustment for multiple comparisons for two-way ANOVA included Tukey's adjustment. The log rank test was used to test for differences in survival between groups, and the method of Kaplan and Meier was used to construct survival curves. Data shown as bar graphs represent the mean and standard error of the mean (s.e.m) of a minimum of 3 biologic replicates. All p-values reported are two-sided and considered as significant if <0.05.

### References for Example 1

1. Wells, R. J. et al. Impact of high-dose cytarabine and asparaginase intensification on childhood acute myeloid leukemia: a report from the Childrens Cancer Group. J Clin Oncol 11, 538-545 (1993).
2. Siegel, S. E. et al. Pediatric-Inspired Treatment Regimens for Adolescents and Young Adults With Philadelphia Chromosome-Negative Acute Lymphoblastic Leukemia: A Review. JAMA Oncol 4, 725-734 (2018).
3. Lord, C. J. & Ashworth, A. PARP inhibitors: Synthetic lethality in the clinic. Science 355, 1152-1158 (2017).
4. Acebron, S. P., Karaulanov, E., Berger, B. S., Huang, Y. L. & Niehrs, C. Mitotic wnt signaling promotes protein stabilization and regulates cell size. Mol Cell 54, 663-674 (2014).
5. Choi, W. H. et al. Open-gate mutants of the mammalian proteasome show enhanced ubiquitin-conjugate degradation. Nat Commun 7, 10963 (2016).
6. Van Heeke, G. & Schuster, S. M. The N-terminal cysteine of human asparagine synthetase is essential for glutamine-dependent activity. J Biol Chem 264, 19475-19477 (1989).
7. Horowitz, B. et al. Asparagine synthetase activity of mouse leukemias. Science 160, 533-535 (1968).
8. Hermanova, I., Zaliova, M., Trka, J. & Starkova, J. Low expression of asparagine synthetase in lymphoid blasts precludes its role in sensitivity to L-asparaginase. Exp Hematol 40, 657-665 (2012).
9. Appel, I. M. et al. Up-regulation of asparagine synthetase expression is not linked to the clinical response L-asparaginase in pediatric acute lymphoblastic leukemia. Blood 107, 4244-4249 (2006).
10. Shalem, O. et al. Genome-scale CRISPR-Cas9 knockout screening in human cells. Science 343, 84-87 (2014).
11. Wharton, K. A., Jr., Zimmermann, G., Rousset, R. & Scott, M. P. Vertebrate proteins related to Drosophila Naked Cuticle bind Dishevelled and antagonize Wnt signaling. Dev Biol 234, 93-106 (2001).
12. de Lau, W. et al. Lgr5 homologues associate with Wnt receptors and mediate R-spondin signalling. Nature 476, 293-297 (2011).
13. Walker, F., Zhang, H. H., Odorizzi, A. & Burgess, A. W. LGR5 is a negative regulator of tumourigenicity, antagonizes Wnt signalling and regulates cell adhesion in colorectal cancer cell lines. PLoS One 6, e22733 (2011).
14. Fuerer, C. & Nusse, R. Lentiviral vectors to probe and manipulate the Wnt signaling pathway. PLoS One 5, e9370 (2010).
15. Siegfried, E., Chou, T. B. & Perrimon, N. wingless signaling acts through zeste-white 3, the Drosophila homolog of glycogen synthase kinase-3, to regulate engrailed and establish cell fate. Cell 71, 1167-1179 (1992).
16. Bennett, C. N. et al. Regulation of Wnt signaling during adipogenesis. J Biol Chem 277, 30998-31004 (2002).
17. van de Wetering, M. et al. Armadillo coactivates transcription driven by the product of the Drosophila segment polarity gene dTCF. Cell 88, 789-799 (1997).
18. Brunner, E., Peter, O., Schweizer, L. & Basler, K. pangolin encodes a Lef-1 homologue that acts downstream of Armadillo to transduce the Wingless signal in Drosophila. Nature 385, 829-833 (1997).
19. Moon, R. T. & Miller, J. R. The APC tumor suppressor protein in development and cancer. Trends Genet 13, 256-258 (1997).
20. Inoki, K. et al. TSC2 integrates Wnt and energy signals via a coordinated phosphorylation by AMPK and GSK3 to regulate cell growth. Cell 126, 955-968 (2006).
21. Esen, E. et al. WNT-LRP5 signaling induces Warburg effect through mTORC2 activation during osteoblast differentiation. Cell Metab 17, 745-755 (2013).
22. Taelman, V. F. et al. Wnt signaling requires sequestration of glycogen synthase kinase 3 inside multivesicular endosomes. Cell 143, 1136-1148 (2010).
23. Huang, Y. L., Anvarian, Z., Doderlein, G., Acebron, S. P. & Niehrs, C. Maternal Wnt/STOP signaling promotes cell division during early Xenopus embryogenesis. Proceedings of the National Academy of Sciences of the United States of America 112, 5732-5737 (2015).
24. Koepp, D. M. et al. Phosphorylation-dependent ubiquitination of cyclin E by the SCFFbw7 ubiquitin ligase. Science 294, 173-177 (2001).
25. Thompson, B. J. et al. The SCFFBW7 ubiquitin ligase complex as a tumor suppressor in T cell leukemia. J Exp Med 204, 1825-1835 (2007).
26. Shah, S. A. et al. 26S proteasome inhibition induces apoptosis and limits growth of human pancreatic cancer. Journal of cellular biochemistry 82, 110-122 (2001).
27. Wagner, F. F. et al. Exploiting an Asp-Glu "switch" in glycogen synthase kinase 3 to design paralog-selective inhibitors for use in acute myeloid leukemia. Sci Transl Med 10 (2018).
28. Koo, B. K. et al. Tumour suppressor RNF43 is a stem-cell E3 ligase that induces endocytosis of Wnt receptors. Nature 488, 665-669 (2012).
29. Giannakis, M. et al. RNF43 is frequently mutated in colorectal and endometrial cancers. Nat Genet 46, 1264-1266 (2014).
30. Seshagiri, S. et al. Recurrent R-spondin fusions in colon cancer. Nature 488, 660-664 (2012).

### References for Materials and Methods of Example 1

1 Reya, T. et al. A role for Wnt signalling in self-renewal of haematopoietic stem cells. Nature 423, 409-414 (2003).
2 Burns, M. A. et al. Hedgehog pathway mutations drive oncogenic transformation in high-risk T-cell acute lymphoblastic leukemia. Leukemia (2018).
3 Sadelain, M., Papapetrou, E. P. & Bushman, F. D. Safe harbours for the integration of new DNA in the human genome. Nat Rev Cancer 12, 51-58 (2011).
4 Sanjana, N. E., Shalem, O. & Zhang, F. Improved vectors and genome-wide libraries for CRISPR screening. Nature methods 11, 783-784 (2014).
5 Chen, B. et al. Dynamic imaging of genomic loci in living human cells by an optimized CRISPR/Cas system. Cell 155, 1479-1491 (2013).
6 Lindsay, H. et al. CrispRVariants charts the mutation spectrum of genome engineering experiments. Nat Biotechnol 34, 701-702 (2016).
7 Shalem, O. et al. Genome-scale CRISPR-Cas9 knockout screening in human cells. Science 343, 84-87 (2014).
8 Doench, J. G. et al. Rational design of highly active sgRNAs for CRISPR-Cas9-mediated gene inactivation. Nat Biotechnol 32, 1262-1267 (2014).
9 Li, W. et al. MAGeCK enables robust identification of essential genes from genome-scale CRISPR/Cas9 knockout screens. Genome Biol 15 (2014).
10 Guo, W. et al. Slug and Sox9 cooperatively determine the mammary stem cell state. Cell 148, 1015-1028 (2012).
11 Rajagopalan, H. et al. Inactivation of hCDC4 can cause chromosomal instability. Nature 428, 77-81 (2004).
12 Fuerer, C. & Nusse, R. Lentiviral vectors to probe and manipulate the Wnt signaling pathway. PLoS One 5, e9370 (2010).
13 Choi, W. H. et al. Open-gate mutants of the mammalian proteasome show enhanced ubiquitin-conjugate degradation. Nat Commun 7, 10963 (2016).
14 Banerji, V. et al. The intersection of genetic and chemical genomic screens identifies GSK-3alpha as a target in human acute myeloid leukemia. J Clin Invest 122, 935-947 (2012).
15 Wagner, F. F. et al. Exploiting an Asp-Glu "switch" in glycogen synthase kinase 3 to design paralog-selective inhibitors for use in acute myeloid leukemia. Sci Transl Med 10 (2018).
16 Wang, J. et al. Nonradioactive quantification of autophagic protein degradation with L-azidohomoalanine labeling. Nature protocols 12, 279-288 (2017).

### EXAMPLE 2

### INTRODUCTION

Colorectal cancer (CRC) remains the second leading cause of cancer deaths in the US, and outcomes are dismal for patients with metastatic disease (Siegel et al., 2017; Siegel et al., 2019). An estimated 96% of CRCs have mutations that activate canonical Wnt/β-catenin signaling (Yaeger et al., 2018), and these mutations promote intestinal transformation (Cheung et al., 2010; Su et al., 1992). Despite a compelling rationale for therapeutic inhibition of this pathway (Dow et al., 2015), oncogenic β-catenin transcription is difficult to inhibit directly (Nusse and Clevers, 2017). The discovery that approximately 15% of CRCs have mutations that drive ligand-dependent activation of Wnt signaling, such as intrachromosomal R-spondin (RSPO) rearrangements and RNF43 mutations (Giannakis et al., 2014; Han et al., 2017; Hao et al., 2012; Koo et al., 2012; Seshagiri et al., 2012), prompted considerable interest in therapeutic inhibition of Wnt ligand activity. This is much more tractable pharmacologically, and a number of therapeutic approaches targeting Wnt ligand-induced Wnt pathway activation have been developed [reviewed in (Nusse and Clevers, 2017)]. However, inhibiting Wnt ligand activity leads to significant bone toxicity with pathologic fractures (Tan et al., 2018). This is an on-target toxicity also caused by germline mutations of Wnt ligands (Fahiminiya et al., 2013; Zheng et al., 2012). While efforts to mitigate this toxicity are ongoing, whether inhibition of Wnt signaling has a sufficiently favorable therapeutic index for cancer therapy remains unclear.

Asparaginase, an antileukemic enzyme that degrades the nonessential amino acid asparagine (Rizzari et al., 2013), has little activity in unselected patients with CRC (Clarkson et al., 1970; Ohnuma et al., 1970; Wilson et al., 1975), most of whom have APC mutations (Yaeger et al., 2018). We recently found that activation of Wnt signaling upstream of GSK3 induces potent sensitization to asparaginase in drug-resistant acute leukemias, but not in normal hematopoietic progenitors (Hinze et al., 2019). Wnt-induced signal transduction is mediated by inhibition of the kinase GSK3 (Siegfried et al., 1992; Stamos et al., 2014; Taelman et al., 2010), and GSK3 inhibition was sufficient for asparaginase sensitization in leukemias. However, this effect appeared to be independent of APC or β-catenin. Instead, asparaginase sensitization was mediated by Wnt-dependent stabilization of proteins (Wnt/STOP), a β-catenin independent branch of Wnt signaling that inhibits GSK3-dependent protein ubiquitination and proteasomal degradation (Acebron et al., 2014). Protein ubiquitination and proteasomal degradation is a catabolic source of amino acids (Suraweera et al., 2012) required for asparaginase resistance in leukemia (Hinze et al., 2019), and this adaptive response is blocked by Wnt-induced inhibition of GSK3.

CRC provides a unique experimental context in which to test predictions from our model, because mutations that arise spontaneously in CRC are predicted to unlink Wnt/β-catenin from Wnt-induced sensitization to asparaginase. Indeed, approximately 80% of human CRC have loss-of-function mutations of APC (Yaeger et al., 2018), which activate β-catenin without inducing sensitivity to asparaginase. By contrast, ~15% of cases have RSPO fusions or RNF43 mutations that stimulate ligand-induced Wnt pathway activation (Chartier et al., 2016; de Lau et al., 2011; Giannakis et al., 2014; Han et al., 2017; Seshagiri et al., 2012; Storm et al., 2016). Our model predicts that Wnt ligand signaling, by inhibiting GSK3, will stimulate both β-catenin activation and Wnt-induced sensitization to asparaginase. The objective of this study was to test these predictions in the context of mutations that arise spontaneously in CRC.

### RESULTS

### Wnt pathway activation upstream of GSK3 induces asparaginase hypersensitivity

To test whether ligand-induced Wnt pathway activation induces asparaginase hypersensitivity in CRC, the inventors began with the human APC-mutant CRC cell lines HCT-15 and SW-480 (Barretina et al., 2012; Gayet et al., 2001). Both of these cell lines proved refractory to asparaginase monotherapy, but treatment with the recombinant ligands Rspo3 and Wnt3a induced significant sensitization to asparaginase (Figure 15A). Wnt-induced signal transduction is mediated by inhibition of the kinase GSK3 (Siegfried et al., 1992; Stamos et al., 2014; Taelman et al., 2010), and treatment of these cells with CHIR-99021, a small molecule inhibitor of both GSK3α and GSK3β (Bennett et al., 2002) was sufficient to induce asparaginase sensitization (Figure 15B). Importantly, the combination of GSK3 inhibition and asparaginase had little toxicity to CCD-841 cells derived from normal human colonic epithelium (Figure 15C) (Thompson et al., 1985).

Mammalian cells have two GSK3 paralogs (GSK3α and GSK3β), which are redundant for regulation of canonical Wnt/β-catenin signaling in several experimental contexts (Banerji et al., 2012; Doble et al., 2007; Wagner et al., 2018). However, it was found that knockdown of GSK3α was sufficient for asparaginase sensitization in CRC, whereas GSK3 β knockdown had little effect (Figure 15D, Figure 17A-17B), consistent with the inventors previous findings in leukemia (presented herein and in Hinze et al., 2019). Treatment of HCT-15 or SW-480 cells with asparaginase in combination with GSK3α shRNA knockdown led to induction of caspase 3/7 activity, a marker of apoptosis induction (Figure 17C-17D). Recently described isoform-selective GSK3 inhibitors were then used to validate these findings pharmacologically (Wagner et al., 2018). Indeed, treatment with the GSK3α-selective inhibitor BRD0705 sensitized both HCT-15 and SW-480 cells to asparaginase-induced cytotoxicity, whereas the GSK3β-selective inhibitor BRD3731 had little effect (Figure 15E).

It was then asked whether these findings are relevant in the context of endogenous mutations that arise spontaneously in CRC. The inventors aimed to test this in experimental models that were as similar as possible, beyond the type of endogenous Wnt-activating mutation. Thus, the inventors turned to genetically engineered mouse intestinal organoids designed to recapitulate the genetics of human CRC (Dow et al., 2015; Han et al., 2017). The combination of Kras, p53 and a Wnt-activating mutation is the most common genotype in metastatic CRC (Yaeger et al., 2018), thus triple-mutant organoids harboring these mutations were leveraged. The endogenous Wnt/β-catenin activating mutation was either Apc deficiency, which were predicted to activate β-catenin without inhibiting GSK3 or stimulating asparaginase sensitivity, or a Ptprk-Rspo3 fusion that potentiates Wnt ligand-induced inhibition of GSK3, which were predicted to both activate β-catenin and stimulate Wnt-induced sensitization to asparaginase. Treatment of these organoids revealed that asparaginase monotherapy was highly toxic to Rspo3 fusion organoids, whereas it had little activity against those that were Apc-deficient (Figure 15F-15G). In Apc-deficient organoids, monotherapy with the GSK3α inhibitor BRD0705 also had little toxicity, but the combination of BRD0705 and asparaginase was potently toxic (Figure 15G), indicating that inhibition of GSK3α is sufficient for asparaginase sensitization.

### Asparaginase Sensitization is Mediated by Wnt-Dependent Stabilization of Proteins

Work presented herein show that drug-resistant leukemias tolerate asparaginase therapy by relying on GSK3-dependent protein ubiquitination and proteasomal degradation as a catabolic source of asparagine. This adaptive response is blocked by Wnt-dependent stabilization of proteins (Wnt/STOP) (Hinze et al., 2019), a GSK3-dependent branch of Wnt signaling that inhibits protein degradation and increases cell size (Acebron et al., 2014; Huang et al., 2015; Taelman et al., 2010). The inventors found that asparaginase monotherapy significantly decreased cell size in the CRC cell line HCT-15, and this effect was reversed by treatment with Wnt ligands (Figure 18A-18B). To test whether Wnt ligand-induced sensitization to asparaginase is mediated by Wnt/STOP, the inventors first leveraged the fact that overexpression of the E3 ubiquitin ligase FBXW7 restores the degradation of a subset of proteins stabilized by Wnt-induced inhibition of GSK3 (Acebron et al., 2014). It was found that the toxicity of asparaginase combined with the GSK3α inhibitor BRD0705 to Apc-mutant organoids was reversed by overexpression of wild-type FBXW7, but not by an FBXW7 R465C point mutant allele that is impaired in its ability to bind its protein substrates (Figure 15H) (Koepp et al., 2001). Additionally, the toxicity of this combination was reversed by expression of a hyperactive open-gate mutant of the proteasomal subunit PSMA4 (Figure 15H), which directly stimulates proteasomal degradation of a range of proteasomal substrates (Choi et al., 2016). Thus, activation of Wnt signaling upstream of GSK3 induces asparaginase sensitization by inhibiting GSK3-dependent protein degradation.

### Exploiting Synthetic Lethality of Wnt Pathway Activation and Asparaginase for Colorectal Cancer Therapy

To test the in vivo therapeutic potential of these findings, subcutaneous tumors were generated in immunodeficient mice injected with triple-mutant mouse intestinal organoids that had Kras and p53 mutations, together with either Apc deficiency or an Rspo3 fusion. Once tumors engrafted, as assessed by clearly measurable tumor growth, mice were randomized to treatment with vehicle or a single dose of asparaginase (Figure 16A). Asparaginase had little effect on Apc-deficient tumors, but had significant therapeutic activity against Rspo3 fusion tumors. Indeed, asparaginase therapy not only markedly delayed disease progression in Rspo3 fusion tumors (Figure 16B), but also induced tumor regression in most treated mice (Figure 16C), and prolonged progression-free survival (Figure 16D), without inducing appreciable weight loss (Figure 19A).

It was then asked whether pharmacologic GSK3α inhibition could induce asparaginase sensitization in vivo using a patient-derived xenografts (PDX) of APC-mutant CRC. Immunodeficient mice were engrafted with a human patient-derived CRC xenograft harboring a truncating APC mutation (p.T1556fsX3) and a Kras p.G12R activating mutation. Following tumor engraftment, mice were randomized to treatment with vehicle, asparaginase (1000 U/kg x 1 dose), the GSK3α selective inhibitor BRD0705 (25 mg/kg every 12 hours x 21 days), or the combination of asparaginase and BRD0705 (Figure 16E). The combination treatment was well tolerated, with no appreciable weight loss or increases in serum bilirubin levels (Figure 19B and 19C), a common toxicity of asparaginase in adult humans. Monotherapy with asparaginase or BRD0705 had no activity, but treatment with the combination of asparaginase and BRD0705 had a potent effect on tumor growth, including tumor regression in all treated mice, and significant prolongation in progression-free survival (Figures 16F-16I).

### DISCUSSION

It is shown herein that the synthetic lethal interaction of GSK3 inhibition and asparaginase can be exploited for CRC therapy. Inhibition of GSK3 is a key mediator of Wnt-induced signal transduction (Siegfried et al., 1992; Stamos et al., 2014; Taelman et al., 2010), thus this kinase is predicted to be endogenously inhibited in a subset of CRCs as a consequence of upstream Wnt pathway mutations. Indeed, it was found that CRCs with chromosomal rearrangements leading to overexpression of Rspo3, a recurrent oncogenic alteration in CRC that potentiates Wnt ligand activity (Chartier et al., 2016; de Lau et al., 2011; Han et al., 2017; Seshagiri et al., 2012; Storm et al., 2016), were profoundly and selectively sensitized asparaginase monotherapy. The model presented herein predicts that tumors with other upstream Wnt-activating mutations, such as Rspo2 fusions or mutations of the Rspo receptor RNF43, should also be asparaginase sensitive.

It is specifically contemplated herein that asparaginase monotherapy, as described herein, would be effective in other tumor types with mutations predicted to stimulate Wnt-induced inhibition of GSK3, such as RNF43-mutant pancreatic, endometrial or gastric cancers (2014; Giannakis et al., 2014; Jiang et al., 2013; Wu et al., 2011).

It was found that APC-mutant CRCs were refractory to asparaginase monotherapy, unless GSK3 function was inhibited in these tumors. Selective inhibition of GSK3α was sufficient for this effect. While the ATP-binding pockets of GSK3α and GSK3β differ by a single amino acid, isoform-selective inhibitors of GSK3α have been developed (Wagner et al., 2018), which now provides a strategy to leverage this synthetic lethal therapeutic interaction for the majority of patients with CRC cases, who have mutations of APC or CTNNB1 (which encodes β-catenin). While the simultaneous inhibition of GSK3 β does not antagonize the therapeutic benefit of GSK3α inhibition, GSK3α and GSK3β are redundant for regulation of β-catenin activity in distinct experimental contexts (Banerji et al., 2012; Doble et al., 2007; Wagner et al., 2018). Thus, pan-GSK3 inhibitors are expected to have toxicity due to widespread activation of oncogenic β-catenin signaling, whereas isoform-selective inhibition of GSK3α is expected to be better tolerated.

Taken together with work presented herein in Example 1 in leukemia (Hinze et al., 2019), these data demonstrate that blocking GSK3-dependent protein degradation induces tumor-selective sensitization to asparaginase in both colon cancer and in drug-resistant leukemias. This leads to the unexpected conclusion that mechanisms of intrinsic asparaginase resistance in solid tumors overlap with those of acquired resistance in leukemia, but are fundamentally distinct from those that allow normal cells to tolerate treatment with the enzyme. Given that the synthetic lethal interaction of GSK3α inhibition and asparaginase is conserved in tumors derived from cellular lineages that are as diverse as intestinal epithelium and hematopoietic cells, this approach could have meaningful therapeutic activity in a broad range of human cancers, so long as these rely on GSK3-dependent protein degradation to tolerate treatment with asparaginase.

### MATERIALS DETAILS

### Patient-Derived Xenografts

Specimens were collected from a patient with APC-mutant colon cancer (Bullman et al., 2017), with informed consent in accordance with the Declaration of Helsinki. Human subject studies were approved by the Dana-Farber Cancer Institute Institutional Review board. Patient-derived xenografts were implanted into immunodeficient mice, as described below. Mouse studies were in accordance with all regulatory standards and approved by the Boston Children's Hospital Institutional Animal Care and Use Committee.

### Cell Lines, Cell Culture and Organoid Culture

293T cells, colorectal cancer cell lines and normal colon cells were purchased from ATCC (Manassas, VA, USA), DSMZ (Braunschweig, Germany) and cultured in DMEM, RPMI-1640 or Leibovitz's L-15 media (Thermo Fisher Scientific) with 10% or 20% fetal bovine serum (FBS, Sigma-Aldrich, Saint Louis, MO) or TET system approved FBS (Clontech, Mountain View, CA) and 1% penicillin/streptomycin (Thermo Fisher Scientific) at 37°C, 5% CO2.

Organoids carrying Ptprk-Rspo3 rearrangement and LSL-KrasG12D are derived from transgenic mice. Ptrpk-Rspo3 rearrangement was selected by culturing organoids without exogenous R-Spondin for 7 days, and validated by sequencing of Ptprk-Rspo3 fusion junction. KrasG12D activation and p53 loss were created using Lenti-sgTrp53-Cas9-Cre (Han et al., 2017). For selection of p53 loss, organoids were cultured with 5 µM Nutlin-3 for 7 days, and KrasG12D activation was indirectly selected during this selection. P53 loss was validated by sgRNA targeting site sequencing and Western Blot, and KrasG12D activation was validated by RNA sequencing.

Mouse colon organoids were cultured in basal organoid culture medium containing advanced DMEM/F-12 (Thermo Fisher Scientific), 1% penicillin/streptomycin, 2mM L-Glutamine (Sigma-Aldrich, Saint Louis, MO), 1mM N-acetylcysteine (Sigma-Aldrich, Saint Louis, MO) and 10mM HEPES (Sigma-Aldrich, Saint Louis, MO). Apc-deficient organoids were cultured in basal organoid medium supplemented with murine Wnt3A (50ng/ml, Merck Millipore, Darmstadt, Germany), murine Noggin (50ng/ml), murine EGF (R&D systems, Minneapolis, MN, 50ng/ml) and human R-Spondin 1 (R&D systems, Minneapolis, MN), as previously described (O'Rourke et al., 2016). Rspo3-fusion organoids were cultured in basal medium in the presence of murine Noggin (50ng/ml), murine EGF (R&D systems, Minneapolis, MN, 50ng/ml) and human R-Spondin 1 (R&D systems, Minneapolis, MN), as previously described (O'Rourke et al., 2016).

Cell line identities were validated using STR profiling at the Dana-Farber Cancer Institute Molecular Diagnostics Laboratory (most recently in December 2018), and mycoplasma contamination was excluded using the MycoAlert Mycoplasma Detection Kit according to the manufacturer's instructions (Lonza, Portsmouth, NH; most recently in November 2018).

### Mice

Nu/J mice were purchased from the Jackson Laboratories (Bar Harbor, ME; Stock # 0007850). 7-9 weeks-old male nude mice were used for experiments and littermates were kept in individual cages. Mice were randomly assigned to experimental groups and handled in strict accordance with Good Animal Practice as defined by the Office of Laboratory Animal Welfare. All animal work was done with Boston Children's Hospital (BCH) Institutional Animal Care and Use Committee approval (protocol # 18-09-3784R).

### Lentiviral Transduction of Colon Cancer Cell Lines

Lentiviruses were generated by co-transfecting pLKO.1 plasmids of interest together with packaging vectors psPAX2 (a gift from Didier Trono; Addgene plasmid # 12260) and VSV.G (a gift from Tannishtha Reya; Addgene plasmid # 14888) using OptiMEM (Invitrogen, Carlsbad, CA) and polyethyleneimine (VWR, Radnor, PA), as previously described (Burns et al., 2018).

Lentiviral infections were performed by spinoculating colorectal cancer cell lines with virus-containing media (1,500 g x 90 minutes) in the presence of 8 µg/ml polybrene (Merck Millipore, Darmstadt, Germany). Selection with antibiotics was started 24 hours after infection with puromycin (1 µg/ml for a minimum of 48 hours; Thermo Fisher Scientific), or blasticidin (15 µg/ml for a minimum of 5 days; Invivogen).

### Lentiviral Transduction of Mouse Intestinal Organoids

Prior to lentiviral transduction, a full 0.95cm2 well of mouse intestinal organoids was harvested by pipetting up and down the matrigel and organoid medium. Briefly, disrupted organoids were centrifuged at 300g for 5 minutes and the cell pellet was resuspended in 250 µl of cold 0.25% trypsin (Thermo Fisher Scientific) and incubated for 5 minutes at 37°C. Subsequently, trypsin was inactivated by adding 750 µl basal organoid culture medium and centrifuged (300g x 5 minutes). Cells were resuspended in 250 µl of concentrated lentivirus supplemented with 8 µg/ml polybrene (Merck Millipore, Darmstadt, Germany). For lentiviral infection, the organoid-virus mixture was incubated for 12 hours at 37°C, 5% CO2. Subsequently, 750 µl of organoid media was added to the well, and the mixture centrifuged at 300g for 5 minutes. The pellet was resuspended in 40 µl of ice-cold matrigel, and 250 µl of basal organoid culture media was added to each well after matrigel solidification. Selection with antibiotics was started 24 hours after infection.

### METHOD DETAILS

### shRNA and Expression Plasmids

The following lentiviral shRNA vectors in pLKO.1 with puromycin resistance were generated by the RNAi Consortium library of the Broad Institute, and obtained from Sigma-Aldrich: shLuciferase (TRCN0000072243), shGSK3α#1 (TRCN0000010340), shGSK3α#4 (TRCN0000038682), shGSK3β#2 (TRCN0000039564), shGSK3β#6 (TRCN0000010551).

Expression constructs expressing wild-type FBXW7 (also known as CDC4) or its R465C mutant were synthesized by gene synthesis, and cloned into the pLX304 lentiviral expression vector by GeneCopoeia (Rockville, MD).

A hyperactive open-gate mutant of the human proteasomal subunit PSMA4, termed ΔN-PSMA4, was designed by deleting the cDNA sequences encoding amino acids 2 to 10 (SRRYDSRTT) of PSMA4 isoform NP_002780.1 (encoded by the transcript NM_002789.6), based on the data of Choi and colleagues (Choi et al., 2016). This ΔN-PSMA4 coding sequence was synthesized by gene synthesis, and cloned into the pLX304 lentiviral expression vector inframe with the C-terminal V5 tag provided by this vector, by GeneCopoeia (Rockville, MD).

### Assessment of Chemotherapy Response and Apoptosis in Colon Cancer Cell Lines

Cells (100,000 per well) were seeded in 100 µl of complete growth medium in 12-well plates and incubated with chemotherapeutic agents or vehicle. Cells were split every 48 hours and cell viability was assessed by counting viable cells based on trypan blue vital dye staining (Invitrogen), according to the manufacturer's instructions. Chemotherapeutic drugs included: asparaginase (pegaspargase, Shire, Lexington, MA), CHIR99021 (Selleckchem), recombinant human Wnt3A protein (R&D systems, Minneapolis, MN) and recombinant human R-Spondin 3 protein (R&D systems, Minneapolis, MN). BRD0705 and BRD3731 were synthesized as described (Wagner et al., 2018). Caspase 3/7 activity was assessed using the Caspase Glo 3/7 Assay (Promega, Madison, WI) according to the manufacturer's instructions.

### Assessment of Chemotherapy Response in Mouse Intestinal Organoids

For assessment of chemotherapy response in mouse intestinal organoids, Apc-deficient and Rspo3 fusion organoids were cultured in basal organoid medium (medium not containing murine Wnt3A, murine Noggin and human R-spondin1 protein). A full 0.95cm2 well of organoids was split into new wells, aiming to obtain approximately 25 organoids per well. Organoids were split according to previously published protocols (O'Rourke et al., 2016). Matrigel and basal organoid culture medium were supplemented with vehicle or chemotherapeutic agents, and split every 48 hours. After 10 days in culture, total organoid numbers per wells were counted by light microscopy. Microscopy was performed using an 100x objective on an Axio Imager A1 microscope (Zeiss, Oberkochen, Germany), with images captured using a CV-A10 digital camera (Jai, Yokohama, Japan) and Cytovision software (Leica Biosystems, Wetzlar, Germany).

Organoids touching the edge of a well were excluded from counting. Images were taken from a representative of three independent experiments and analyzed with ImageJ Software (Schneider et al., 2012).

### Assessment of Cell Size

Cells (100,000 per well) were plated in 1 ml of complete growth medium, containing a final concentration of 10 U/L asparaginase or 100 ng/ml Wnt3A ligand in a 12-well format. After 48 hours of treatment, forward scatter height (FSC-H) was assessed by flow cytometry on a Beckton-Dickinson LSR-II instrument.

### Quantitative Reverse Transcriptase PCR (qRT-PCR)

RNA was isolated using RNeasy kit (Qiagen) and cDNA was made using SuperScript III first-strand cDNA synthesis kit (Thermo Fisher Scientific). qRT-PCR was performed using Power SYBR^{®} Green PCR Master Mix (Thermo Fisher Scientific) and 7500 real-time PCR system (Applied Biosystems). Primers used are described in Table S2.

### In Vivo Drug Treatment of Patient-Derived Xenografts (PDX) and Organoids

For implantation of APC mutant human CRC PDX, patient tumor material was collected in PBS and kept on wet ice for engraftment within 24 h after resection. Upon arrival, necrotic and supporting tissues were carefully removed using a surgical blade. Approximately 1mm x 1mm tissue fragments were implanted subcutaneously into the flank region of male nude mice, as described (Bullman et al., 2017). For injection of intestinal organoids (Rspo3; p53; Kras, or Apc; p53; Kras), per mouse one full 9.5cm2 well of organoids was injected subcutaneously.

As soon as tumors reached a volume of approximately 150mm3, treatment of mice was begun. For the APC mutant human CRC PDX, a single dose of asparaginase (1,000 U/kg) or PBS was injected by tail vein injection on day 1 of treatment, and BRD0705 (15 mg/kg) or vehicle was given every 12 hours for 21 days by oral gavage. Vehicle was formulated as previously described (Wagner et al., 2018).

After start of treatment, body weight and tumor size were evaluated every other day. Tumor size was assessed by caliper measurements and the approximate volume of the mass was calculated using the formula (1 × w × w) × (π/6), where l is the major tumor axis and w is the minor tumor axis. The response was determined by comparing tumor volume change at time t to its baseline: % tumor volume change = 100% × ((Vt - Vinitial) / Vinitia, as previously described (Gao et al., 2015). Mice were euthanized as soon as they reached a tumor volume of 1500 mm3, developed weight loss greater than 15% and/or showed signs of tumor ulceration. For Figure 16B and 2F, the last recorded tumor volume of mice that died

### Mice being censored due to tumor progression.

Mice that reached a tumor volume of 1500mm3 were Figure 16B and 16F.

To assess bilirubin levels, retro-orbital blood collections were performed, and bilirubin levels were measured in the Boston Children's Hospital clinical laboratory. (units, detection limit)

### Quantification and Statistical Analysis

For two-group comparisons of continuous measures, a two-tailed Welch unequal variances t-test was used. For 3-group comparisons, a one-way analysis of variance model (ANOVA) was performed and a Dunnett adjustment for multiple comparisons was used. For analysis of two effects, a two-way ANOVA model was constructed and included an interaction term between the two effects. Post-hoc adjustment for multiple comparisons for two-way ANOVA included Tukey-Kramer adjustment. The log rank test was used to test for differences in survival between groups, and the method of Kaplan and Meier was used to construct survival curves. Data shown as bar graphs represent the mean and standard error of the mean (s.e.m) of a minimum of 3 biologic replicates, unless otherwise indicated. All p-values reported are two-sided and considered as significant if <0.05.

### References for Example 2

(2014). Comprehensive molecular characterization of gastric adenocarcinoma. Nature 513, 202-209.
Acebron, S. P., Karaulanov, E., Berger, B. S., Huang, Y. L., and Niehrs, C. (2014). Mitotic wnt signaling promotes protein stabilization and regulates cell size. Mol Cell 54, 663-674. Banerji, V., Frumm, S. M., Ross, K. N., Li, L. S., Schinzel, A. C., Hahn, C. K., Kakoza, R. M., Chow, K. T., Ross, L., Alexe, G., et al. (2012). The intersection of genetic and chemical genomic screens identifies GSK-3alpha as a target in human acute myeloid leukemia. J Clin Invest 122, 935-947.
Barretina, J., Caponigro, G., Stransky, N., Venkatesan, K., Margolin, A. A., Kim, S., Wilson, C. J., Lehar, J., Kryukov, G. V., Sonkin, D., et al. (2012). The Cancer Cell Line Encyclopedia enables predictive modelling of anticancer drug sensitivity. Nature 483, 603-607.
Bennett, C. N., Ross, S. E., Longo, K. A., Bajnok, L., Hemati, N., Johnson, K. W., Harrison, S. D., and MacDougald, O. A. (2002). Regulation of Wnt signaling during adipogenesis. J Biol Chem 277, 30998-31004.
Bullman, S., Pedamallu, C. S., Sicinska, E., Clancy, T. E., Zhang, X., Cai, D., Neuberg, D., Huang, K., Guevara, F., Nelson, T., et al. (2017). Analysis of Fusobacterium persistence and antibiotic response in colorectal cancer. Science 358, 1443-1448.
Burns, M. A., Liao, Z. W., Yamagata, N., Pouliot, G. P., Stevenson, K. E., Neuberg, D. S., Thorner, A. R., Ducar, M., Silverman, E. A., Hunger, S. P., et al. (2018). Hedgehog pathway mutations drive oncogenic transformation in high-risk T-cell acute lymphoblastic leukemia. Leukemia.
Chartier, C., Raval, J., Axelrod, F., Bond, C., Cain, J., Dee-Hoskins, C., Ma, S., Fischer, M. M., Shah, J., Wei, J., et al. (2016). Therapeutic Targeting of Tumor-Derived R-Spondin Attenuates beta-Catenin Signaling and Tumorigenesis in Multiple Cancer Types. Cancer Res 76, 713-723.
Cheung, A. F., Carter, A. M., Kostova, K. K., Woodruff, J. F., Crowley, D., Bronson, R. T., Haigis, K. M., and Jacks, T. (2010). Complete deletion of Apc results in severe polyposis in mice. Oncogene 29, 1857-1864.
Choi, W. H., de Poot, S. A., Lee, J. H., Kim, J. H., Han, D. H., Kim, Y. K., Finley, D., and Lee, M. J. (2016). Open-gate mutants of the mammalian proteasome show enhanced ubiquitin-conjugate degradation. Nature communications 7, 10963.
Clarkson, B., Krakoff, I., Burchenal, J., Karnofsky, D., Golbey, R., Dowling, M., Oettgen, H., and Lipton, A. (1970). Clinical results of treatment with E. coli L-asparaginase in adults with leukemia, lymphoma, and solid tumors. Cancer 25, 279-305.
de Lau, W., Barker, N., Low, T. Y., Koo, B. K., Li, V. S., Teunissen, H., Kujala, P., Haegebarth, A., Peters, P. J., van de Wetering, M., et al. (2011). Lgr5 homologues associate with Wnt receptors and mediate R-spondin signalling. Nature 476, 293-297.
Doble, B. W., Patel, S., Wood, G. A., Kockeritz, L. K., and Woodgett, J. R. (2007). Functional redundancy of GSK-3alpha and GSK-3beta in Wnt/beta-catenin signaling shown by using an allelic series of embryonic stem cell lines. Dev Cell 12, 957-971.
Dow, L. E., O'Rourke, K. P., Simon, J., Tschaharganeh, D. F., van Es, J. H., Clevers, H., and Lowe, S. W. (2015). Apc Restoration Promotes Cellular Differentiation and Reestablishes Crypt Homeostasis in Colorectal Cancer. Cell 161, 1539-1552.
Fahiminiya, S., Majewski, J., Mort, J., Moffatt, P., Glorieux, F. H., and Rauch, F. (2013). Mutations in WNT1 are a cause of osteogenesis imperfecta. Journal of medical genetics 50, 345-348.
Gao, H., Korn, J. M., Ferretti, S., Monahan, J. E., Wang, Y., Singh, M., Zhang, C., Schnell, C., Yang, G., Zhang, Y., et al. (2015). High-throughput screening using patient-derived tumor xenografts to predict clinical trial drug response. Nat Med 21, 1318-1325.
Gayet, J., Zhou, X. P., Duval, A., Rolland, S., Hoang, J. M., Cottu, P., and Hamelin, R. (2001). Extensive characterization of genetic alterations in a series of human colorectal cancer cell lines. Oncogene 20, 5025-5032.
Giannakis, M., Hodis, E., Jasmine Mu, X., Yamauchi, M., Rosenbluh, J., Cibulskis, K., Saksena, G., Lawrence, M. S., Qian, Z. R., Nishihara, R., et al. (2014). RNF43 is frequently mutated in colorectal and endometrial cancers. Nat Genet 46, 1264-1266.
Han, T., Schatoff, E. M., Murphy, C., Zafra, M. P., Wilkinson, J. E., Elemento, O., and Dow, L. E. (2017). R-Spondin chromosome rearrangements drive Wnt-dependent tumour initiation and maintenance in the intestine. Nature communications 8, 15945.
Hao, H. X., Xie, Y., Zhang, Y., Charlat, O., Oster, E., Avello, M., Lei, H., Mickanin, C., Liu, D., Ruffner, H., et al. (2012). ZNRF3 promotes Wnt receptor turnover in an R-spondin-sensitive manner. Nature 485, 195-200.
Hinze, L., Pfirrmann, M., Karim, S., Degar, J., McGuckin, C., Vinjamur, D., Sacher, J., Stevenson, K. E., Neuberg, D. S., Orellana, E., et al. (2019). Synthetic Lethality of Wnt Pathway Activation and Asparaginase in Drug-Resistant Acute Leukemias. Cancer Cell 35, 664-676 e667.
Huang, Y. L., Anvarian, Z., Doderlein, G., Acebron, S. P., and Niehrs, C. (2015). Maternal Wnt/STOP signaling promotes cell division during early Xenopus embryogenesis. Proceedings of the National Academy of Sciences of the United States of America 112, 5732-5737.
Jiang, X., Hao, H. X., Growney, J. D., Woolfenden, S., Bottiglio, C., Ng, N., Lu, B., Hsieh, M. H., Bagdasarian, L., Meyer, R., et al. (2013). Inactivating mutations of RNF43 confer Wnt dependency in pancreatic ductal adenocarcinoma. Proceedings of the National Academy of Sciences of the United States of America 110, 12649-12654.
Koepp, D. M., Schaefer, L. K., Ye, X., Keyomarsi, K., Chu, C., Harper, J. W., and Elledge, S. J. (2001). Phosphorylation-dependent ubiquitination of cyclin E by the SCFFbw7 ubiquitin ligase. Science 294, 173-177.
Koo, B. K., Spit, M., Jordens, I., Low, T. Y., Stange, D. E., van de Wetering, M., van Es, J. H., Mohammed, S., Heck, A. J., Maurice, M. M., and Clevers, H. (2012). Tumour suppressor RNF43 is a stem-cell E3 ligase that induces endocytosis of Wnt receptors. Nature 488, 665-669.
Nusse, R., and Clevers, H. (2017). Wnt/beta-Catenin Signaling, Disease, and Emerging Therapeutic Modalities. Cell 169, 985-999.
O'Rourke, K. P., Ackerman, S., Dow, L. E., and Lowe, S. W. (2016). Isolation, Culture, and Maintenance of Mouse Intestinal Stem Cells. Bio-protocol 6.
Ohnuma, T., Holland, J. F., Freeman, A., and Sinks, L. F. (1970). Biochemical and pharmacological studies with asparaginase in man. Cancer Res 30, 2297-2305.
Rizzari, C., Conter, V., Stary, J., Colombini, A., Moericke, A., and Schrappe, M. (2013). Optimizing asparaginase therapy for acute lymphoblastic leukemia. Curr Opin Oncol 25 Suppl 1, S1-9.
Schneider, C. A., Rasband, W. S., and Eliceiri, K. W. (2012). NIH Image to ImageJ: 25 years of image analysis. Nature methods 9, 671-675.
Seshagiri, S., Stawiski, E. W., Durinck, S., Modrusan, Z., Storm, E. E., Conboy, C. B., Chaudhuri, S., Guan, Y., Janakiraman, V., Jaiswal, B. S., et al. (2012). Recurrent R-spondin fusions in colon cancer. Nature 488, 660-664.
Siegel, R. L., Miller, K. D., Fedewa, S. A., Ahnen, D. J., Meester, R. G. S., Barzi, A., and Jemal, A. (2017). Colorectal cancer statistics, 2017. CA Cancer J Clin 67, 177-193.
Siegel, R. L., Miller, K. D., and Jemal, A. (2019). Cancer statistics, 2019. CA Cancer J Clin 69, 7-34.
Siegfried, E., Chou, T. B., and Perrimon, N. (1992). wingless signaling acts through zeste-white 3, the Drosophila homolog of glycogen synthase kinase-3, to regulate engrailed and establish cell fate. Cell 71, 1167-1179.
Stamos, J. L., Chu, M. L., Enos, M. D., Shah, N., and Weis, W. I. (2014). Structural basis of GSK-3 inhibition by N-terminal phosphorylation and by the Wnt receptor LRP6. Elife 3, e01998.
Storm, E. E., Durinck, S., de Sousa e Melo, F., Tremayne, J., Kljavin, N., Tan, C., Ye, X., Chiu, C., Pham, T., Hongo, J. A., et al. (2016). Targeting PTPRK-RSPO3 colon tumours promotes differentiation and loss of stem-cell function. Nature 529, 97-100.
Su, L. K., Kinzler, K. W., Vogelstein, B., Preisinger, A. C., Moser, A. R., Luongo, C., Gould, K. A., and Dove, W. F. (1992). Multiple intestinal neoplasia caused by a mutation in the murine homolog of the APC gene. Science 256, 668-670.
Suraweera, A., Munch, C., Hanssum, A., and Bertolotti, A. (2012). Failure of amino acid homeostasis causes cell death following proteasome inhibition. Mol Cell 48, 242-253. Taelman, V. F., Dobrowolski, R., Plouhinec, J. L., Fuentealba, L. C., Vorwald, P. P., Gumper, I., Sabatini, D. D., and De Robertis, E. M. (2010). Wnt signaling requires sequestration of glycogen synthase kinase 3 inside multivesicular endosomes. Cell 143, 1136-1148.
Tan, D., Ng, M., Subbiah, V., Messersmith, W., Teneggi, V., Diermayr, V., Ethirajulu, K., Yeo, P., Gan, B. H., Lee, L. H., et al. (2018). Phase 1 extension study of ETC-159 an oral PORCN inhibitor administered with bone protective treatment, in patients with advanced solid tumours. Annals of Oncology (ESMO Asia 2018 meeting abstracts) 29 (suppl_9), ix23-ix27.
Thompson, A. A., Dilworth, S., and Hay, R. J. (1985). Isolation and culture of colonic epithelial cells in serum-free medium. Journal of Tissue Culture Methods 9, 117-122. Wagner, F. F., Benajiba, L., Campbell, A. J., Weiwer, M., Sacher, J. R., Gale, J. P., Ross, L., Puissant, A., Alexe, G., Conway, A., et al. (2018). Exploiting an Asp-Glu "switch" in glycogen synthase kinase 3 to design paralog-selective inhibitors for use in acute myeloid leukemia. Sci Transl Med 10.
Wilson, W. L., Weiss, A. J., and Ramirez, G. (1975). Phase I study of L-asparaginase (NSC 109229). Oncology 32, 109-117.
Wu, J., Jiao, Y., Dal Molin, M., Maitra, A., de Wilde, R. F., Wood, L. D., Eshleman, J. R., Goggins, M. G., Wolfgang, C. L., Canto, M. I., et al. (2011). Whole-exome sequencing of neoplastic cysts of the pancreas reveals recurrent mutations in components of ubiquitindependent pathways. Proceedings of the National Academy of Sciences of the United States of America 108, 21188-21193.
Yaeger, R., Chatila, W. K., Lipsyc, M. D., Hechtman, J. F., Cercek, A., Sanchez-Vega, F., Jayakumaran, G., Middha, S., Zehir, A., Donoghue, M. T. A., et al. (2018). Clinical Sequencing Defines the Genomic Landscape of Metastatic Colorectal Cancer. Cancer Cell 33, 125-136 e123.
Zheng, H. F., Tobias, J. H., Duncan, E., Evans, D. M., Eriksson, J., Paternoster, L., Yerges-Armstrong, L. M., Lehtimaki, T., Bergstrom, U., Kahonen, M., et al. (2012). WNT16 influences bone mineral density, cortical bone thickness, bone strength, and osteoporotic fracture risk. PLoS Genet 8, e1002745.

## Claims

1. An asparaginase for use in the treatment of cancer in a subject, wherein:
a) the asparaginase is used in combination with an agent that inhibits GSK3α, or
b) the cancer comprises a mutation that results in inhibition of GSK3α.

2. The asparaginase for use of any preceding claim, wherein the asparaginase is selected from the group consisting of:
L-asparaginase (Elspar), pegaspargase (PEG-asparaginase; Oncaspar), SC-PEG asparaginase (Calaspargase pegol), and Erwinia asparaginase (Erwinaze).

3. The asparaginase for use of any preceding claim, wherein the agent that inhibits GSK3α is selected from the group consisting of:
a small molecule, optionally
BRD0705, BRD4963, BRD1652, BRD3731, CHIR-98014, LY2090314, AZD1080, CHIR-99021 (CT99021) HCl, CHIR-99021 (CT99021), BIO-acetoxime, SB216763, SB415286, Abemaciclib (LY2835210), AT-9283, RGB-286638, PHA-793887, AT-7519, AZD-5438, OTS-167, 9-ING-41, Tideglusib (NP031112), and AR-A014418;
an antibody;
a peptide;
a genome editing system;
an antisense oligonucleotide; and
an RNAi, optionally a microRNA, an siRNA, or a shRNA.

4. The asparaginase for use of any preceding claim, wherein inhibiting GSK3α is inhibiting the expression level and/or activity of GSK3α,
optionally wherein the expression level and/or activity of GSK3α is inhibited by at least 50%, at least 60%, at least 70%, at least 80%, at least 90%, or more as compared to an appropriate control.

5. The asparaginase for use of any preceding claim, wherein the mutation:
results in the activation of the WNT signaling pathway in the cancer cell;
is in a gene selected from the group consisting of: R-spondin1 (RSPO1), R-spondin 2 (RSPO2), R-spondin 3 (RSPO3), R-spondin4 (RSPO4), ring finger protein 43 (RNF43), and glycogen synthase kinase 3 alpha (GSK3A, more commonly referred to as GSK3α); or
alters the expression of a gene selected from the group consisting of: R-spondin1 (RSPO1), R-spondin 2 (RSPO2), R-spondin 3 (RSPO3), R-spondin4 (RSPO4), ring finger protein 43 (RNF43),
and glycogen synthase kinase 3 alpha (GSK3A, more commonly referred to as GSK3α).

6. The asparaginase for use of any preceding claim, wherein the subject:
has, prior to treatment, been diagnosed as having cancer;
has, prior to treatment, received a result from an assay that diagnoses a subject as having cancer;
has, prior to treatment, been identified as having cancer comprising a mutation that results in inhibition of GSK3α,
optionally wherein the mutation is identified in a biological sample, for example a tissue sample or blood sample, obtained from the subject; or
has been identified as having a cancer having a mutation that results in inhibition of GSK3α by an assay of a biological sample,
optionally a tissue sample or a blood sample, from said subject.

7. The asparaginase for use of any one of claims 1-6, wherein the subject has previously been administered an anticancer therapy.

8. The asparaginase for use of any one of claims 1-6, wherein the subject has not previously been administered ar anti-cancer therapy.

9. The asparaginase for use of any preceding claim, wherein the cancer:
is selected from the list consisting of: a carcinoma, a melanoma, a sarcoma, a myeloma, a lymphoma, and a leukemia optionally acute myeloid leukemia (AML), Chronic myeloid leukemia (CML), Acute lymphocytic leukemia (ALL), and Chronic lymphocytic leukemia (CLL);
is a solid tumour;
is colon or pancreatic cancer;
is metastatic;
is resistant to a cancer therapy; or
relapsed following a cancer therapy.

10. The asparaginase for use of any of claims 1-9, wherein the cancer is resistant to an asparaginase

11. The asparaginase for use of any of claims 1-9, wherein the cancer is not resistant to an asparaginase

12. The asparaginase for use of Claim 9, wherein the cancer therapy is chemotherapy, radiation therapy, immunotherapy, surgery, hormone therapy, stem cell therapy, targeted therapy, gene therapy, or precision therapy.

## Patentansprüche

1. Asparaginase zur Verwendung bei der Behandlung von Krebs bei einem Subjekt, wobei:
a) die Asparaginase in Kombination mit einem Mittel verwendet wird, das GSK3α hemmt, oder
b) der Krebs eine Mutation umfasst, die zu einer Hemmung von GSK3α führt.

2. Asparaginase zur Verwendung nach einem der vorhergehenden Ansprüche, wobei die Asparaginase ausgewählt ist aus der Gruppe bestehend aus:
L-Asparaginase (Elspar), Pegaspargase (PEG-Asparaginase; Oncaspar), SC-PEG-Asparaginase (Calaspargase pegol) und Erwinia-Asparaginase (Erwinaze).

3. Asparaginase zur Verwendung nach einem der vorhergehenden Ansprüche, wobei das Mittel, das GSK3α hemmt, ausgewählt ist aus der Gruppe bestehend aus:
einem kleinen Molekül, optional
BRD0705, BRD4963, BRD1652, BRD3731, CHIR-98014, LY2090314, AZD1080, CHIR-99021 (CT99021) HCl, CHIR-99021 (CT99021), BIO-Acetoxim, SB216763, SB415286, Abemaciclib (LY2835210), AT-9283, RGB-286638, PHA-793887, AT-7519, AZD-5438, OTS-167, 9-ING-41, Tideglusib (NP031112) und AR-A014418;
einem Antikörper;
einem Peptid;
einem Genomeditierungssystem;
einem Antisense-Oligonukleotid; und
einer RNAi, optional einer mikroRNA, einer siRNA oder einer shRNA.

4. Asparaginase zur Verwendung nach einem der vorhergehenden Ansprüche, wobei das Hemmen von GSK3α das Hemmen des Expressionsniveaus und/oder der Aktivität von GSK3α ist,
wobei optional das Expressionsniveau und/oder die Aktivität von GSK3α um mindestens 50 %, mindestens 60 %, mindestens 70 %, mindestens 80 %, mindestens 90 % oder mehr im Vergleich zu einer geeigneten Kontrolle gehemmt wird.

5. Asparaginase zur Verwendung nach einem der vorhergehenden Ansprüche, wobei die Mutation:
zur Aktivierung des WNT-Signalwegs in der Krebszelle führt;
in einem Gen liegt, das ausgewählt ist aus der Gruppe bestehend aus: R-Spondin1 (RSPO1), R-Spondin 2 (RSPO2), R-Spondin 3 (RSPO3), R-Spondin 4 (RSPO4), Ring-Finger-Protein 43 (RNF43) und Glykogensynthase-Kinase-3-alpha (GSK3A, allgemeiner als GSK3α bezeichnet); oder
die Expression eines Gens verändert, das ausgewählt ist aus der Gruppe bestehend aus: R-Spondin1 (RSPO1), R-Spondin 2 (RSPO2), R-Spondin 3 (RSPO3), R-Spondin 4 (RSPO4), Ring-Finger-Protein 43 (RNF43)
und Glykogensynthase-Kinase-3-alpha (GSK3A, allgemeiner als GSK3α bezeichnet).

6. Asparaginase zur Verwendung nach einem der vorhergehenden Ansprüche, wobei das Subjekt:
vor der Behandlung als Krebs habend diagnostiziert wurde;
vor der Behandlung ein Ergebnis aus einem Test erhalten hat, der bei einem Subjekt Krebs diagnostiziert;
vor der Behandlung als Krebs aufweisend identifiziert wurde, der eine Mutation umfasst, die zu einer Hemmung von GSK3α führt,
wobei optional die Mutation in einer biologischen Probe, zum Beispiel einer Gewebeprobe oder Blutprobe, identifiziert wird, die von dem Subjekt erhalten wurde; oder
als einen Krebs aufweisend identifiziert wurde, der eine Mutation aufweist, die zu einer Hemmung von GSK3α führt, durch einen Test einer biologischen Probe,
optional einer Gewebeprobe oder einer Blutprobe, von dem Subjekt.

7. Asparaginase zur Verwendung nach einem der Ansprüche 1-6, wobei dem Subjekt zuvor eine Anti-Krebs-Therapie verabreicht wurde.

8. Asparaginase zur Verwendung nach einem der Ansprüche 1-6, wobei dem Subjekt zuvor keine Anti-Krebs-Therapie verabreicht wurde.

9. Asparaginase zur Verwendung nach einem der vorhergehenden Ansprüche, wobei der Krebs:
ausgewählt ist aus der Liste bestehend aus: einem Karzinom, einem Melanom, einem Sarkom, einem Myelom, einem Lymphom und einer Leukämie, optional akuter myeloischer Leukämie (AML), chronischer myeloischer Leukämie (CML), akuter lymphatischer Leukämie (ALL) und chronischer lymphatischer Leukämie (CLL);
ein solider Tumor ist;
Dickdarm- oder Bauchspeicheldrüsenkrebs ist;
metastasierend ist;
gegen eine Krebstherapie resistent ist; oder
nach einer Krebstherapie rezidiviert ist.

10. Asparaginase zur Verwendung nach einem der Ansprüche 1-9, wobei der Krebs gegen eine Asparaginase resistent ist.

11. Asparaginase zur Verwendung nach einem der Ansprüche 1-9, wobei der Krebs gegen eine Asparaginase nicht resistent ist.

12. Asparaginase zur Verwendung nach Anspruch 9, wobei die Krebstherapie Chemotherapie, Strahlentherapie, Immuntherapie, Chirurgie, Hormontherapie, Stammzelltherapie, zielgerichtete Therapie, Gentherapie oder Präzisionstherapie ist.

## Revendications

1. Asparaginase destinée au traitement du cancer chez un sujet, dans laquelle :
a) l'asparaginase est utilisée en association avec un agent qui inhibe la GSK3α, ou
b) le cancer comprend une mutation qui entraîne l'inhibition de la GSK3α.

2. Asparaginase destinée à être utilisée selon une quelconque revendication précédente, dans laquelle l'asparaginase est choisie à partir du groupe constitué de :
L-asparaginase (Elspar), pégaspargase (PEG-asparaginase ; Oncaspar), SC-PEG asparaginase (Calaspargase pegol) et asparaginase d'Erwinia (Erwinaze).

3. Asparaginase destinée à être utilisée selon une quelconque revendication précédente, dans laquelle l'agent qui inhibe la GSK3α est choisi à partir du groupe constitué de :
une petite molécule, éventuellement
BRD0705, BRD4963, BRD1652, BRD3731, CHIR-98014, LY2090314, AZD1080, CHIR-99021 (CT99021) HCl, CHIR-99021 (CT99021), BIO-acétoxime, SB216763, SB415286, Abémaciclib (LY2835210), AT-9283, RGB-286638, PHA-793887, AT-7519, AZD-5438, OTS-167, 9-ING-41, Tideglusib (NP031112), et AR-A014418 ;
un anticorps ;
un peptide ;
un système d'édition de génome ;
un oligonucléotide antisens ; et
un ARNi, éventuellement un microARN, un siARN, ou un shARN.

4. Asparaginase destinée à être utilisée selon une quelconque revendication précédente, dans laquelle l'inhibition de GSK3α consiste à inhiber le niveau d'expression et/ou l'activité de GSK3α,
éventuellement dans laquelle le niveau d'expression et/ou l'activité de GSK3α est inhibé d'au moins 50 %, d'au moins 60 %, d'au moins 70 %, d'au moins 80 %, d'au moins 90 %, ou plus par rapport à une commande appropriée.

5. Asparaginase destinée à être utilisée selon une quelconque revendication précédente, dans laquelle la mutation :
entraîne l'activation de la voie de signalisation WNT dans la cellule cancéreuse ;
se trouve dans un gène choisi à partir du groupe constitué de : R-spondine 1 (RSPO1), R-spondine 2 (RSPO2), R-spondine 3 (RSPO3), R-spondine 4 (RSPO4), protéine à doigt de zinc 43 (RNF43), et glycogène synthase kinase 3 alpha (GSK3A, plus communément appelée GSK3α) ; ou
modifie l'expression d'un gène choisi à partir du groupe constitué de : R-spondine 1 (RSPO1), R-spondine 2 (RSPO2), R-spondine 3 (RSPO3), R-spondine 4 (RSPO4), protéine à doigt de zinc 43 (RNF43),
et glycogène synthase kinase 3 alpha (GSK3A, plus communément appelée GSK3α).

6. Asparaginase destinée à être utilisée selon une quelconque revendication précédente, dans laquelle le sujet :
a reçu, avant le traitement, un diagnostic de cancer ;
a reçu, avant le traitement, un résultat d'un test qui diagnostique un cancer chez un sujet ;
a été identifié, avant le traitement, comme ayant un cancer comprenant une mutation qui entraîne l'inhibition de la GSK3α,
éventuellement dans laquelle la mutation est identifiée dans un échantillon biologique, par exemple un échantillon de tissu ou un échantillon de sang, obtenu à partir du sujet ; ou
a été identifié comme ayant un cancer ayant une mutation qui entraîne l'inhibition de la GSK3α par un test d'un échantillon biologique,
éventuellement un échantillon de tissu ou un échantillon de sang, à partir dudit sujet.

7. Asparaginase destinée à être utilisée selon l'une quelconque des revendications 1 à 6, dans laquelle le sujet a préalablement reçu une thérapie anticancéreuse.

8. Asparaginase destinée à être utilisée selon l'une quelconque des revendications 1 à 6, dans laquelle le sujet n'a pas préalablement reçu de thérapie anticancéreuse.

9. Asparaginase destinée à être utilisée selon une quelconque revendication précédente, dans laquelle le cancer :
est choisi à partir de la liste composée de : un carcinome, un mélanome, un sarcome, un myélome, un lymphome, et une leucémie éventuellement une leucémie myéloïde aiguë (LMA), une leucémie myéloïde chronique (LMC), une leucémie lymphoïde aiguë (LLA), et une leucémie lymphoïde chronique (LLC) ;
est une tumeur solide ;
est un cancer du côlon ou du pancréas ;
est métastatique ;
est résistant à une thérapie anticancéreuse ; ou
récidive après une thérapie anticancéreuse.

10. Asparaginase destinée à être utilisée selon l'une quelconque des revendications 1 à 9, dans laquelle le cancer est résistant à une asparaginase

11. Asparaginase destinée à être utilisée selon l'une quelconque des revendications 1 à 9, dans laquelle le cancer n'est pas résistant à une asparaginase

12. Asparaginase destinée à être utilisée selon la revendication 9, dans laquelle la thérapie anticancéreuse est une chimiothérapie, une radiothérapie, une immunothérapie, une chirurgie, une hormonothérapie, une thérapie par cellules souches, une thérapie ciblée, une thérapie génique, ou une thérapie de précision.
